(19) Europäisches Patentamt
European Patent Office
Office européen des brevets

(11) EP 4 772 510 A2

(12) EUROPEAN PATENT APPLICATION
published in accordance with Art. 153(4) EPC

(43) Date of publication:
**08.07.2026 Bulletin 2026/28**

(21) Application number: **24858808.9**

(22) Date of filing: **31.10.2024**

(51) International Patent Classification (IPC):
*C07D 487/06* (2006.01) *C07D 498/06* (2006.01)
*C07D 513/06* (2006.01) *A61K 31/55* (2006.01)
*A61K 31/553* (2006.01) *A61K 31/554* (2006.01)
*A61P 35/00* (2006.01)

(52) Cooperative Patent Classification (CPC):
**A61K 31/55; A61K 31/553; A61K 31/554; A61P 35/00; C07D 487/06; C07D 498/06; C07D 513/06**

(86) International application number:
**PCT/CN2024/129191**

(87) International publication number:
**WO 2025/045281 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.08.2023 CN 202311116640**

(71) Applicant: **HANGZHOU BIO-SINCERITY PHARMA-TECHCO., LTD.**
**Hangzhou, Zhejiang 311199 (CN)**

(72) Inventors:
- **FENG, Enguang**
  **Hangzhou, Zhejiang 311199 (CN)**
- **ZHANG, Jinzhu**
  **Hangzhou, Zhejiang 311199 (CN)**
- **LIU, Jiacheng**
  **Hangzhou, Zhejiang 311199 (CN)**
- **LEI, Shaowei**
  **Hangzhou, Zhejiang 311199 (CN)**
- **WANG, Tingting**
  **Hangzhou, Zhejiang 311199 (CN)**
- **LI, Yuanyuan**
  **Hangzhou, Zhejiang 311199 (CN)**
- **SHEN, Ximing**
  **Hangzhou, Zhejiang 311199 (CN)**
- **LOU, Jinfang**
  **Hangzhou, Zhejiang 311199 (CN)**

(74) Representative: **karo IP Patentanwälte PartG mbB**
**Steinstraße 16-18**
**40212 Düsseldorf (DE)**

# (54) BENZAZEPINONE COMPOUND AND USE THEREOF

(57) Disclosed in the present invention is a benzazepinone compound. The present invention particularly relates to substance forms of the compound such as a free base, an isomer or a pharmaceutically acceptable salt thereof. Further disclosed in the present invention are a method for preparing the benzazepinone compound and the use of the benzazepinone compound. The compound can be used as an RIPK1 inhibitor for the treatment of diseases, including, but not limited to, tumors, related to the abnormal expression of an RIPK1-mediated signaling pathway, and has a great clinical development value.



Processed by Luminess, 75001 PARIS (FR)

## Description

### TECHNICAL FIELD

The present disclosure belongs to the technical field of small molecule targeted chemicals, and relates to a benzazepinone compound, and in particular to the structural forms of such compound, such as a free base, an isomer, or a pharmaceutically acceptable salt thereof. Further disclosed in the present disclosure are a method for preparing such benzazepinone compound and pharmaceutical use thereof. The compound can be used as a RIPK1 inhibitor for treating diseases related to and mediated by abnormal expression of the RIPK1 signaling pathway, including but not limited to tumors.

### BACKGROUND

Receptor-interacting protein kinase 1 (RIPK1) is a serine/threonine protein kinase. Its C-terminus contains a death domain. By binding to each other or other molecules containing a death domain (such as TRADD, Fas, or TNFRI), it participates in signal transduction stimulated by TNF and FasL, mediates NF-κB activation, and induces apoptosis. Its function is regulated by ubiquitination, zinc finger protein, heat shock protein, etc.

Part of the diseases or conditions regulated by receptor-interacting protein kinase 1, dependent apoptosis, necrosis, or cytokine production include hematologic and solid organ malignancies (GenesDev. 2013, 27, 1640-1649) and bacterial infections and viral infections ((Cell Host&Microbe, 2014, 15, 23-35), including but not limited to tuberculosis and influenza (Cell, 2013, 153, 1-14)). In conclusion, receptor-interacting protein kinase 1, as a key regulatory protein of apoptosis, is involved in not only cell survival-promoting signaling but also programmed cell death signaling in various pathways. Receptor-interacting protein kinase 1 inhibitors have potential therapeutic effects for treating a variety of diseases such as cancer, neurodegenerative diseases, and autoimmune diseases. The receptor-interacting protein kinase 1 inhibitors currently under development worldwide are mainly divided into three categories in terms of compound structure: indoles (e.g., Necrostatin-1s), benzazepines (e.g., GSK-2982772), and pyrazole amides (e.g., GSK-963 and GSK547). These drugs are currently in pre-clinical research or clinical research stages, with no marketed drugs available to date, indicating considerable future market potential. Professor Yuan Junying is an authoritative figure and pioneer in the field of receptor-interacting protein kinase 1 inhibitors. In 2005, she discovered Necrostatin-1s, the first small-molecule receptor-interacting protein kinase 1 inhibitor, which has been extensively utilized in studies investigating the role and mechanisms of receptor-interacting protein kinase 1 in human diseases and animal models. In 2008, its mechanism of action as necrostatins was elucidated, and thus it was identified as a key regulator of necroptosis. Globally, drugs in this class have progressed no further than the pre-clinical development stage.

Patent WO2014125444 discloses a novel benzazepine derivative, which was screened from a gene coding library. In the same year, GSK-2982772, the first receptor-interacting protein kinase 1 inhibitor in the world, was approved for clinical trials. In 2016, GSK-2982772 was advanced to phase II clinical trials, and the indications for phase II clinical trials are psoriasis, rheumatoid arthritis, and ulcerative colitis. In addition to GSK-2982772, GSK-3145095 entered phase II clinical trials on November 16, 2018 for the treatment of pancreatic cancer, and the study of GSK-3145095 for the treatment of pancreatic cancer was terminated on September 12, 2019.

Patent WO2016185423 discloses a pyrazole amide derivative, and the representative compound is GSK-963. However, its poor oral effect limits its development. Subsequently, the compound is used as a lead compound for structural optimization to obtain GSK-547. Due to poor pharmacokinetic data, the molecule has not entered clinical trials.

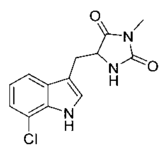
Necrostatin-1s

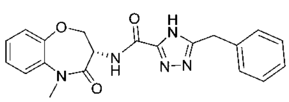
GSK-2982772

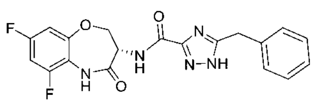
GSK-3145095

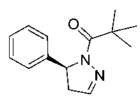
GSK-963

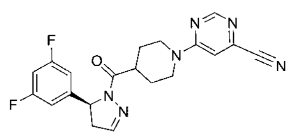
GSK-547

Receptor-interacting protein kinase 1 inhibitors based on the above three main structures exhibit low selectivity, strong toxic and side effects, low potential to be manufactured as a drug, and other defects. Therefore, researchers at home and abroad continue to modify the structure of the compounds on the basis of their structures and hope to find safer and more effective receptor-interacting protein kinase 1 inhibitors to address the lack of drugs acting on this target.

Based on searching, patent documents regarding GSK-2982772 and structural modification and improvement of GSK-2982772 include, for example, the following.

Patent WO2014125444 discloses a compound of structural formula 1, the structural features of which are as follows: the core is a benzazepine ring, the benzazepine ring is connected to a benzene ring or a nitrogen-containing six-membered aromatic heterocycle, the benzazepine ring is connected to ring A through an amide bond, and ring A is connected to ring B through -L-; the representative compound is GSK-2982772.

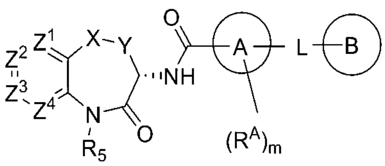
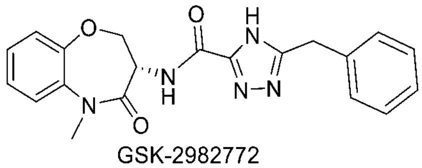

GSK-2982772

Structural formula 1

Patent CN112079839 discloses a compound of structural formula 3, the structural feature of which is as follows: N on the azepine ring, the core, is fused to the benzene ring (or six-membered aromatic heterocycle) on the left to further form a tricyclic structure. The $IC_{50}$ of the representative compound 15 for human monocytic leukemia U937 cells is 2.6 nM, and its oral bioavailability is only 44.8±7.4%. For oral drugs, its bioavailability needs to be improved urgently.

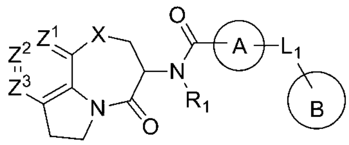

Structural formula 3

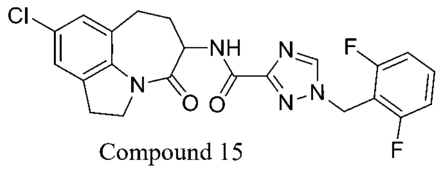

Compound 15

In summary, how to develop a new receptor-interacting protein kinase 1 inhibitor with good biological activity and significant effects on one or more diseases associated with inflammation and tumors, and develop Chinese innovative drugs with independent intellectual property rights on this basis is an urgent technical problem to be solved by those skilled in the art.

## SUMMARY

The technical problem to be solved by the present disclosure is to provide a benzazepinone derivative with a new structure, which can inhibit the activity of receptor-interacting protein kinase 1 and has significant anti-tumor activity and/or anti-inflammatory activity.

In order to solve the above technical problem, the present disclosure provides the following technical solutions. Provided is a benzazepinone compound. The compound is a compound represented by formula I, an isomer, or a pharmaceutically acceptable salt thereof:

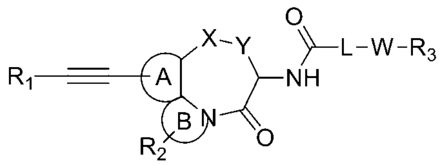

(I)

wherein: ring A is selected from a 6- to 10-membered aromatic ring and a 5- to 10-membered heteroaromatic ring, and ring A is substituted with at least one substituent

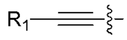;

ring B is selected from 3- to 6-membered cycloalkyl and 3- to 6-membered heterocyclyl, and ring B is substituted with at least one substituent $R_2$;

X and Y are each independently selected from $CR_4$, $OR_4$, $SR_4$, and $NR_4$, and X and Y are not both $NR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_2$ is selected from hydrogen, halogen, $C_{1-6}$ alkyl, and $C_{1-6}$ haloalkyl;
L is selected from a 5- to 6-membered heteroaromatic ring substituted with at least one substituent selected from one or more of the following groups: hydrogen, halogen, $C_{1-6}$ alkyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;
W is selected from one of a linking bond, $-(CH_2)_n-$, -O-, $-(CH_2)_nO(CH_2)_m-$, -CO-, and $-(CH_2)_nCO(CH_2)_m-$;
$R_3$ is selected from a 6- to 10-membered aromatic ring and a 5- to 10-membered heteroaromatic ring substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;
$R_4$ is selected from hydrogen, halogen, $C_{1-6}$ alkyl, $C_{1-6}$ alkoxy, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;
n and m are each independently selected from 0, 1, 2, 3, and 4, and n and m are not both 0;
the 5- to 10-membered heteroaromatic ring, the 5- to 8-membered heteroaryl, and the 3- to 6-membered heterocyclyl contain at least one heteroatom selected from N, O, and S.

Preferably, the compound is a compound represented by formula II, an isomer, or a pharmaceutically acceptable salt thereof:

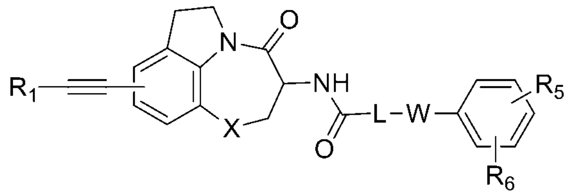
(II)

wherein: X is selected from $CR_4$, $OR_4$, and $SR_4$;
$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;
L is selected from triazole, pyridine, pyrazole, and pyrazolotetrahydropyridone substituted with at least one substituent selected from one or more of the following groups: hydrogen, halogen, $C_{1-6}$ alkyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;
W is selected from one of $-(CH_2)_n-$, -O-, and -CO-;
$R_4$, $R_5$, and $R_6$ are each independently selected from hydrogen, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;
n is selected from 1, 2, 3, and 4;
the 5- to 10-membered heteroaromatic ring, the 5- to 8-membered heteroaryl, and the 3- to 6-membered heterocyclyl contain at least one heteroatom selected from N, O, and S.

More preferably, the compound is a compound represented by formula II-1, an isomer, or a pharmaceutically acceptable salt thereof:

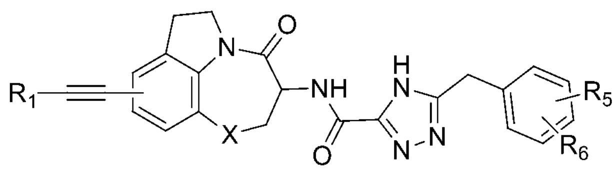
(II-1)

wherein: X is selected from $CR_4$, $OR_4$, and $SR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_4$, $R_5$, and $R_6$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

More preferably, in formula II-1, X is selected from $CH_2$.

More preferably, in formula II-1, $R_5$ and $R_6$ are each selected from hydrogen and halogen.

More preferably, in formula II-1, $R_1$ is selected from the following structures:

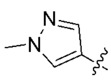, 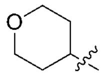, 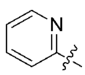, 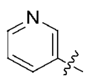, 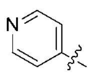, 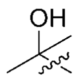, 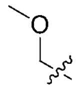, 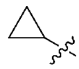, 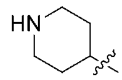, 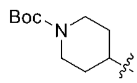,

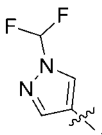, 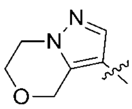, 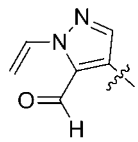, 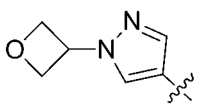, and 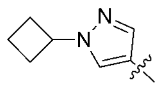.

More preferably, the compound is a compound represented by formula II-2, an isomer, or a pharmaceutically acceptable salt thereof:

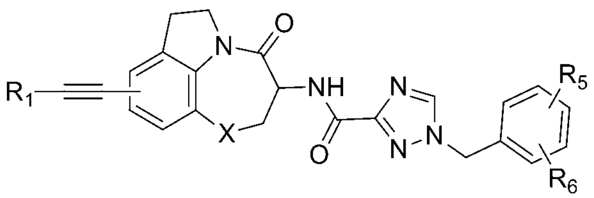

(II-2)

wherein: X is selected from $CR_4$, $OR_4$, and $SR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_4$, $R_5$, and $R_6$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

More preferably, in formula II-2, X is selected from $CH_2$.

More preferably, in formula II-2, $R_5$ and $R_6$ are each selected from hydrogen and halogen.

More preferably, in formula II-2, $R_1$ is selected from the following structures:

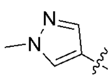, 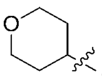, 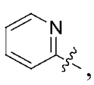, 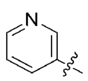, 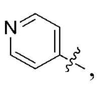, 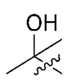, 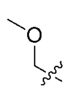, 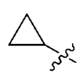, 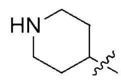, 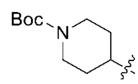, 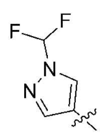,

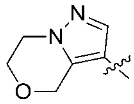, 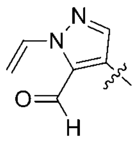, 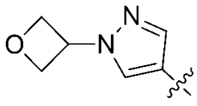, and 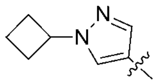.

More preferably, the compound is a compound represented by formula II-3, an isomer, or a pharmaceutically acceptable salt thereof:

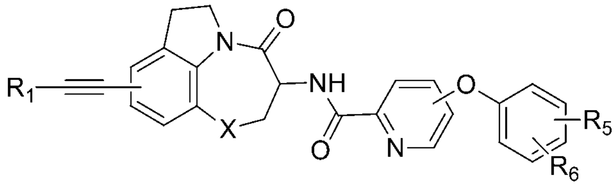

(II-3)

wherein: X is selected from $CR_4$, $OR_4$, and $SR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_4$, $R_5$, and $R_6$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

More preferably, in formula II-3, X is selected from $CH_2$.

More preferably, in formula II-3, $R_5$ and $R_6$ are each selected from hydrogen and halogen.

More preferably, the compound is a compound represented by formula II-4, an isomer, or a pharmaceutically acceptable salt thereof:

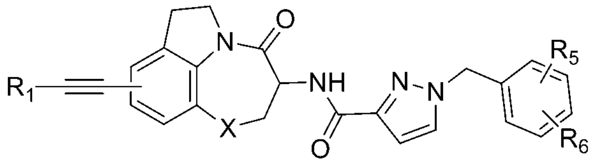

(II-4)

wherein: X is selected from $CR_4$, $OR_4$, and $SR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_4$, $R_5$, and $R_6$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

More preferably, in formula II-4, X is selected from $CH_2$.

More preferably, in formula II-4, $R_5$ and $R_6$ are each selected from hydrogen and halogen.

More preferably, the compound is a compound represented by formula II-5, an isomer, or a pharmaceutically acceptable salt thereof:

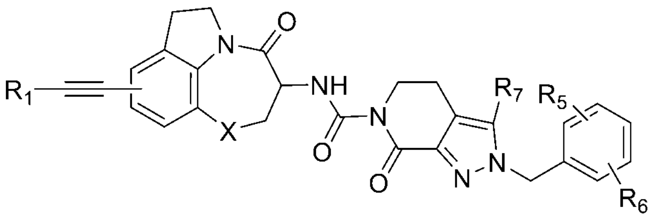

(II-5)

wherein: X is selected from $CR_4$, $OR_4$, and $SR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_4$, $R_5$, $R_6$, and $R_7$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

More preferably, in formula II-5, X is selected from $CH_2$.

More preferably, in formula II-5, $R_5$, $R_6$, and $R_7$ are each selected from hydrogen and halogen.

The present disclosure further provides a benzazepinone compound, including the following compounds numbered I-1 to I-13, II-1 to II-4, III-1 to III-32, IV-1, V-1 to V-2, VI-1, VII-1, VIII-1, IX-1, X-1, XI-1, XII-1, XIII-1 to XIII-2, XIV-1, XV-1, and XVI-1 or pharmaceutically acceptable salts thereof:

Compound I-1: 5-benzyl-*N*-(9-(cyclopropylethynyl)-4-oxo-1,2,3,4,6,7-hexahydroazepino[3,2,1-*hi*]indol-3-yl)-4*H*-1,2,4-triazole-3-carboxamide;

Compound I-2: 5-benzyl-*N*-(4-oxo-9-(pyridin-2-ylethynyl)-1,2,3,4,6,7-hexahydroazepino[3,2,1-*hi*]indol-3-yl)-4*H*-1,2,4-triazole-3-carboxamide;

Compound I-3: 5-benzyl-*N*-(4-oxo-9-(pyridin-3-ylethynyl)-1,2,3,4,6,7-hexahydroazepino[3,2,1-*hi*]indol-3-yl)-4*H*-1,2,4-triazole-3-carboxamide;

Compound I-4: 5-benzyl-*N*-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;

Compound I-5: 5-benzyl-N-(9-((1-methyl-1H-pyrazol-4-yl)ethynyl)-4-oxo-1,2,3,4,6,7-hexahydroazepino[3,2,1-hi]indol-3-yl)-4*H*-1,2,4-triazole-3-carboxamide;

Compound I-6: 5-benzyl-N-(4-oxo-9-((tetrahydro-2H- pyran-4-yl)ethynyl)-1,2,3,4,6,7-hexahydroazepino[3,2,1-hi]indol-3-yl)-4*H*-1,2,4-triazole-3-carboxamide;

Compound I-7: 5-benzyl-N-(9-(3-hydroxy-3-methylbut-1-yn-1-yl)-4-oxo-1,2,3,4,6,7-hexahydroazepino[3,2,1-hi]indol-3-yl)-4*H*-1,2,4-triazole-3-carboxamide;

Compound I-8: 5-(4-fluorobenzyl)-N-(4-oxo-9-(pyridin-4-ylethynyl)-1,2,3,4,6,7-hexahydroazepino[3,2,1-hi]indol-3-yl)-4*H*-1,2,4-triazole-3-carboxamide;

Compound I-9: 5-(4-chlorobenzyl)-N-(4-oxo-9-(pyridin-4-ylethynyl)-1,2,3,4,6,7-hexahydroazepino[3,2,1-hi]indol-3-yl)-4*H*-1,2,4-triazole-3-carboxamide;

Compound I-10: 5-(2-chlorobenzyl)-N-(4-oxo-9-(pyridin-4-ylethynyl)-1,2,3,4,6,7-hexahydroazepino[3,2,1-hi]indol-3-yl)-4*H*-1,2,4-triazole-3-carboxamide;

Compound I-11: 5-(2-fluorobenzyl)-N-(4-oxo-9-(pyridin-4-ylethynyl)-1,2,3,4,6,7-hexahydroazepino[3,2,1-hi]indol-3-yl)-4*H*-1,2,4-triazole-3-carboxamide;

Compound I-12: 5-(2,6-difluorobenzyl)-*N*-(4-oxo-9-(pyridin-4-ylethynyl)-1,2,3,4,6,7-hexahydroazepino(3,2,1-*hi*]indol-3-yl)-4*H*-1,2,4-triazole-3-carboxamide;

Compound I-13: 5-(2,6-dichlorobenzyl)-N-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;

Compound II-1: 5-benzyl-N-(4-oxo-8-(pyridin-2-ylethynyl)-1,2,3,4,6,7-hexahydroazepino[3,2,1-hi]indol-3-yl)-4H-1,2,4-triazole-3-carboxamide;

Compound II-2: 5-benzyl-N-(4-oxo-8-(pyridin-3-ylethynyl)-1,2,3,4,6,7-hexahydroazepino[3,2,1-hi]indol-3-yl)-4H-1,2,4-triazole-3-carboxamide;

Compound II-3: 5-benzyl-*N*-(8-(cyclopropylethynyl)-4-oxo-1,2,3,4,6,7-hexahydroazepino[3,2,1-*hi*]indol-3-yl)-4*H*-1,2,4-triazole-3-carboxamide;

Compound II -4: 5-benzyl-N-(4-oxo-8-(pyridin-4-ylethynyl)-1,2,3,4,6,7-hexahydroazepino[3,2,1-hi]indol-3-yl)-4*H*-1,2,4-triazole-3-carboxamide;

Compound III-1: 1-(2,6-difluorobenzyl)-N-(9-((1-methyl-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-2: 1-(2,6-difluorobenzyl)-N-(4-oxo-9-(pyridin-4-ylethynyl)-1,2,3,4,6,7-hexahydroazepino[3,2,1-hi]indol-3-yl)-1*H*-1,2,4-triazole-3-carboxamide;
Compound III-3: 1-benzyl-N-(4-oxo-9-(pyridin-4-ylethynyl)-1,2,3,4,6,7-hexahydroazepino[3,2,1-hi]indol-3-yl)-1H-1,2,4-triazole-3-carboxamide;
Compound III-4: 1-(2,6-difluorobenzyl)-N-(9-(3-hydroxy-3-methylbut-1-yn-1-yl)-4-oxo-1,2,3,4,6,7-hexahydroazepino[3,2,1-*hi*]indol-3-yl)-1*H*-1,2,4-triazole-3-carboxamide;
Compound III-5: 1-(2,6-difluorobenzyl)-N-(9-(3-methoxyprop-1-yn-1-yl)-4-oxo-1,2,3,4,6,7-hexahydroazepino[3,2,1-hi]indol-3-yl)-1*H*-1,2,4-triazole-3-carboxamide;
Compound III-6: *N*-(9-(cyclopropylethynyl)-4-oxo-1,2,3,4,6,7-hexahydroazepino[3,2,1-*hi*]indol-3-yl)-1-(2,6-difluorobenzyl)-1*H*-1,2,4-triazole-3-carboxamide;
Compound III-7: 1-(2-fluorobenzyl)-N-(9-((1-methyl-1H-pyrazol-4-yl)ethynyl)-4-oxo-1,2,3,4,6,7-hexahydroazepino [3,2,1-*hi*]indol-3-yl)-1*H*-1,2,4-triazole-3-carboxamide;
Compound III-8: 1-(4-fluorobenzyl)-N-(9-((1-methyl-1H-pyrazol-4-yl)ethynyl)-4-oxo-1,2,3,4,6,7-hexahydroazepino [3,2,1-*hi*]indol-3-yl)-1*H*-1,2,4-triazole-3-carboxamide;
Compound III-9: 1-(2,4-difluorobenzyl)-N-(9-((1-methyl-1H-pyrazol-4-yl)ethynyl)-4-oxo-1,2,3,4,6,7-hexahydroazepino[3,2,1-*hi*]indol-3-yl)-1*H*-1 ,2,4-triazole-3-carboxamide;
Compound III-10: 1-(2,5-difluorobenzyl)-N-(9-((1-methyl-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-11: 1-(3,5-difluorobenzyl)-N-(9-((1-methyl-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-12: 1-(3,4-difluorobenzyl)-N-(9-((1-methyl-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-13: N-(9-((1-methyl-1H-pyrazol-4-yl)ethynyl)-4-oxo-1,2,3,4,6,7-hexahydroazepino[3,2,1-hi]indol-3-yl)-1-(2-methylbenzyl)-1*H*-1,2,4-triazole-3-carboxamide;
Compound III-14: N-(9-((1-methyl-1H-pyrazol-4-yl)ethynyl)-4-oxo-1,2,3,4,6,7-hexahydroazepino[3,2,1-hi]indol-3-yl)-1-(4-methylbenzyl)-1*H*-1,2,4-triazole-3-carboxamide;
Compound III-15: 1-(2,6-difluorobenzyl)-*N*-(4-oxo-9-(piperidin-4-ylethynyl)-1,2,3,4,6,7-hexahydroazepino[3,2,1-hi] indol-3-yl)-1*H*-1,2,4-triazole-3-carboxamide;
Compound III-16: tert-butyl 4-((3-(1-(2,6-difluorobenzyl)-1H-1,2,4-triazole-3-carboxamido)-4-oxo-1,2,3,4,6,7-hexahydroazepino[3,2,1-*hi*]indol-9-yl)ethynyl)piperidine-1-carboxylate;
Compound III-17: 1-(2,6-difluorobenzyl)-N-(9-((1-(difluoromethyl)-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-18: 1-(3,5-difluorobenzyl)-N-(9-((1-(difluoromethyl)-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-19: 1-(2,5-difluorobenzyl)-N-(9-((1-(difluoromethyl)-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-20: 1-(2,4-difluorobenzyl)-N-(9-((1-(difluoromethyl)-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-21:*N*(9-((1-(difluoromethyl)-1*H* pyrazol-4-yl)ethynyl)-4-oxo-1,2,3,4,6,7-hexahydroazepino[3,2,1-*hi*] indol-3-yl)-1-(2-fluorobenzyl)-1*H*-1,2,4-triazole-3-carboxamide;
Compound 111-22:*N*-(9-((1-(difluoromethyl)-1*H*-pyrazol-4-yl)ethynyl)-4-oxo-1,2,3,4,6,7-hexahydroazepino [3,2,1-*hi*]indol-3-yl)-1-(2-methylbenzyl)-1*H*-1,2,4-triazole-3-carboxamide;
Compound III-23: 1-(3,4-difluorobenzyl)-N-(9-((1-(difluoromethyl)-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-24:*N* (9-((1-(difluoromethyl)-1*H* pyrazol-4-yl)ethynyl)-4-oxo-1,2,3,4,6,7-hexahydroazepino[3,2,1-*hi*] indol-3-yl)-1-(4-fluorobenzyl)-1*H*-1,2,4-triazole-3-carboxamide;
Compound III-25:*N* (9-((1-(difluoromethyl)-1*H* pyrazol-4-yl)ethynyl)-4-oxo-1,2,3,4,6,7-hexahydroazepino[3,2,1-*hi*] indol-3-yl)-1-(4-methylbenzyl)-1*H*-1,2,4-triazole-3-carboxamide;
Compound III-26: 1-(2,6-difluorobenzyl)-N-(9-ethynyl-4-oxo-1,2,3,4,6,7-hexahydroazepino[3,2,1-hi]indol-3-yl)-1*H*-1,2,4-triazole-3-carboxamide;
Compound III-27: 1-(2,6-difluorobenzyl)-N-(9-((6,7-dihydro-4H-pyrazolo[5,1-c][1,4]oxazin-3-yl)ethynyl)-4-oxo-1,2,3,4,6,7-hexahydroazepino[3,2,1-*hi*]indol-3-yl)-1*H*-1,2,4-triazole-3-carboxamide;
Compound III-28: 1-(2,6-difluorobenzyl)-N-(9-((5-formyl-1-vinyl-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
Compound III-29: 1-(2,6-difluorobenzyl)-N-(9-((1-(oxetan-3-yl)-1H-pyrazol-4-yl)ethynyl)-4-oxo-1,2,3,4,6,7-hexahydroazepino[3,2,1-*hi*]indol-3-yl)-1*H*-1,2,4-triazole-3-carboxamide;

Compound III-30: *N*-(9-((1-cyclobutyl-1*H*-pyrazol-4-yl)ethynyl)-4-oxo-1,2,3,4,6,7-hexahydroazepino[3,2,1-*hi*]indol-3-yl)-1-(2,6-difluorobenzyl)-1*H*-1,2,4-triazole-3-carboxamide;

Compound III-31: N-(9-((1-methyl-1H-pyrazol-4-yl)ethynyl)-4-oxo-1,2,3,4,6,7-hexahydroazepino[3,2,1-hi]indol-3-yl)-1-(pyridin-2-ylmethyl)-1*H*-1,2,4-triazole-3-carboxamide;

Compound III-32: 1-benzyl-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;

Compound IV-1: 1-benzyl-N-(4-oxo-8-(pyridin-4-ylethynyl)-1,2,3,4,6,7-hexahydroazepino[3,2,1-hi]indol-3-yl)-1*H*-1,2,4-triazole-3-carboxamide;

Compound V-1: N-(9-((1-methyl-1H-pyrazol-4-yl)ethynyl)-4-oxo-1,2,3,4,6,7-hexahydroazepino[3,2,1-hi]indol-3-yl)-6-phenoxypicolinamide;

Compound V-2: N-(9-((1-methyl-1H-pyrazol-4-yl)ethynyl)-4-oxo-1,2,3,4,6,7-hexahydroazepino[3,2,1-hi]indol-3-yl)-5-phenoxypicolinamide;

Compound VI-1: 5-[(2,6-difluorophenyl)methyl]-*N*-{9-[(1-methylpyrazol-4-yl)ethynyl]-4-oxo-3,4,6,7-tetrahydro-2*H*-[1,4]oxazepino[2,3,4-*hi*]indol-3-yl}-4*H*-1,2,4-triazole-3-carboxamide;

Compound VII-1: 5-[(2,6-difluorophenyl)methyl]-*N*-{9-[(1-methylpyrazol-4-yl)ethynyl]-4-oxo-3,4,6,7-tetrahydro-2*H*-[1,4]thiazepino[2,3,4-*hi*]indol-3-yl}-4*H*-1,2,4-triazole-3-carboxamide;

Compound VIII-1: N-{7,7-difluoro-9-[(1-methylpyrazol-4-yl)ethynyl]-4-oxo-1,2,4,5,6,7-hexahydroazepino[3,2,1-*hi*]indol-5-yl}-5-[(2,6-difluorophenyl)methyl]-4*H*-1,2,4-triazole-3-carboxamide;

Compound IX-1: 5-benzyl-*N*-[8-(cyclopropylethynyl)-4-oxo-3,4,6,7-tetrahydro-2*H*-[1,4]oxazepino-[2,3,4-*hi*]indol-3-yl]-4*H*-1,2,4-triazole-3-carboxamide;

Compound X-1: 1-[(2,6-difluorophenyl)methyl]-*N*-{9-[(1-methylpyrazol-4-yl)ethynyl]-4-oxo-3,4,6,7-tetrahydro-2*H*-[1,4]oxazepino[2,3,4-*hi*]indol-3-yl}-1,2,4-triazole-3-carboxamide;

Compound XI-1: 1-[(2,6-difluorophenyl)methyl]-*N*-{9-[(1-methylpyrazol-4-yl)ethynyl]-4-oxo-3,4,6,7-tetrahydro-2*H*-[1,4]thiazepino[2,3,4-*hi*]indol-3-yl}-1,2,4-triazole-3-carboxamide;

Compound XII-1: N-{7,7-difluoro-9-[(1-methylpyrazol-4-yl)ethynyl]-4-oxo-1,2,4,5,6,7-hexahydroazepino[3,2,1-*hi*]indol-5-yl}-1-[(2,6-difluorophenyl)methyl]-1,2,4-triazole-3-carboxamide;

Compound XIII-1: 1-(2,6-difluorobenzyl)-N-(9-((1-methyl-1H-pyrazol-4-yl)ethynyl)-4-oxo-1,2,3,4,6,7-hexahydroazepino[3,2,1-*hi*]indol-3-yl)-1*H* pyrazole-3-carboxamide;

Compound XIII-2: 1-[(4-fluorophenyl)methyl]-*N*-{9-[(1-methylpyrazol-4-yl)ethynyl]-4-oxo-1,2,4,5,6,7-hexahydroazepino[3,2,1-*hi*]indol-5-yl}pyrazole-3-carboxamide;

Compound XIV-1: 5-(2-benzyl-3-chloro-7-oxo-4,5,6,7-tetrahydropyrazolo[3,4-c]pyridin-6-yl)-9-[(1-methylpyrazol-4-yl)ethynyl]-1,2,4,5,6,7-hexahydroazepino[3,2,1-*hi*]indole-4-one;

Compound XV-1: 4-[(3-fluorophenyl)methyl]-*N*-{9-[(1-methylpyrazol-4-yl)ethynyl]-4-oxo-1,2,4,5,6,7-hexahydroazepino[3,2,1-*hi*]indol-5-yl}pyrazole-1-carboxamide;

Compound XVI-1: 4-[(3-fluorophenyl)methyl]-*N*-{9-[(1-methylpyrazol-4-yl)ethynyl]-4-oxo-3,4,6,7-tetrahydro-2*H*-[1,4]oxazepino-[2,3,4-hi]indol-3-yl]-4*H*-1,2,4-triazole-3-carboxamide.

The structural formulas of the aforementioned compounds numbered I-1 to I-13, II-1 to II-4, VI-1, VII-1, VIII-1, and IX-1, or pharmaceutically acceptable salts thereof are specifically as follows:

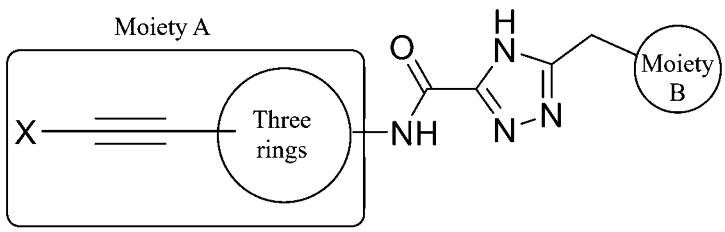

I

| No. | Moiety A | Moiety B |
|---|---|---|
| I-1 | 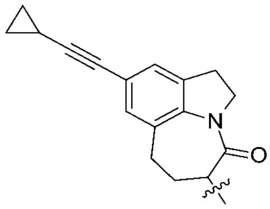 | 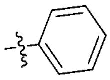 |
| I-2 | 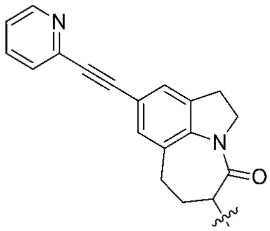 | 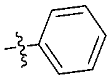 |
| I-3 | 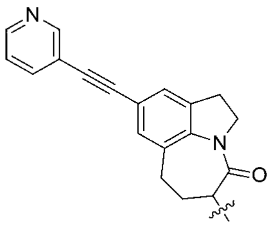 | 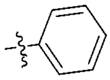 |
| I-4 | 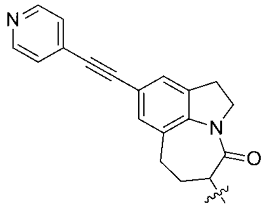 | 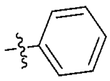 |
| I-5 | 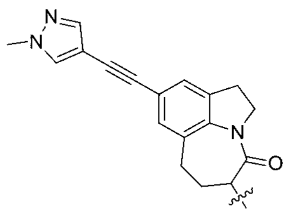 | 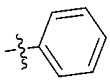 |
| I-6 | 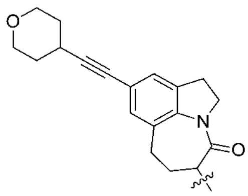 | 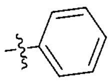 |
| I-7 | 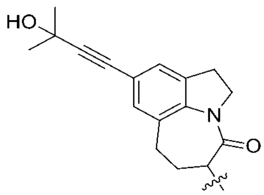 | 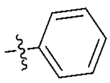 |

(continued)

| No. | Moiety A | Moiety B |
|---|---|---|
| I-8 | 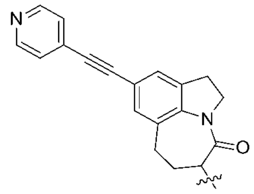 | 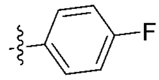 |
| I-9 | 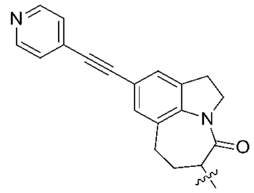 | 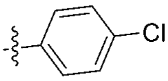 |
| I-10 | 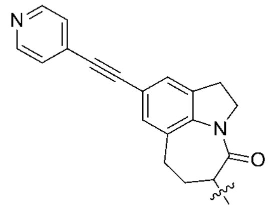 | 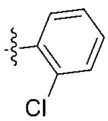 |
| I-11 | 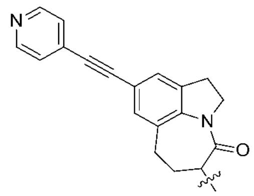 | 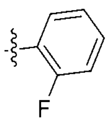 |
| I-12 | 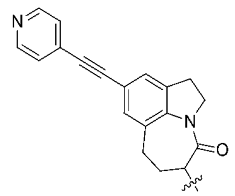 | 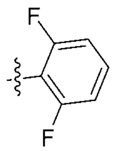 |
| I-13 | 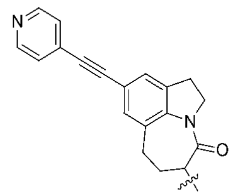 | 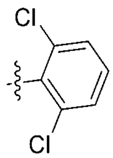 |
| II-1 | 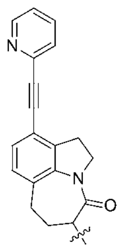 | 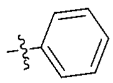 |

(continued)

| No. | Moiety A | Moiety B |
|---|---|---|
| II-2 | 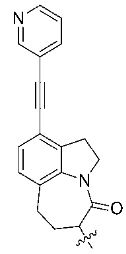 | 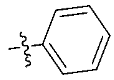 |
| II-3 | 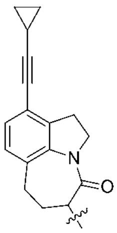 | 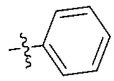 |
| II-4 | 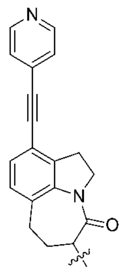 | 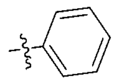 |
| VI-1 | 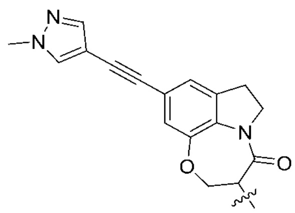 | 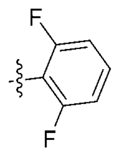 |
| VII-1 | 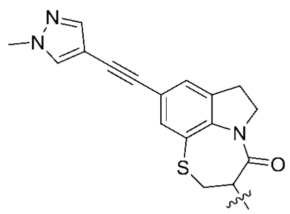 | 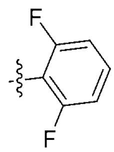 |
| VIII-1 | 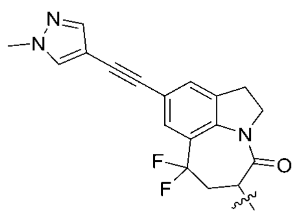 | 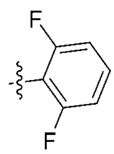 |

(continued)

| No. | Moiety A | Moiety B |
|---|---|---|
| IX-1 | 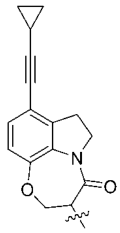 | 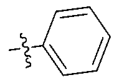 |

The structural formulas of the aforementioned compounds numbered III-1 to III-32, IV-1, X-1, XI-1, and XII-1, or pharmaceutically acceptable salts thereof are specifically as follows:

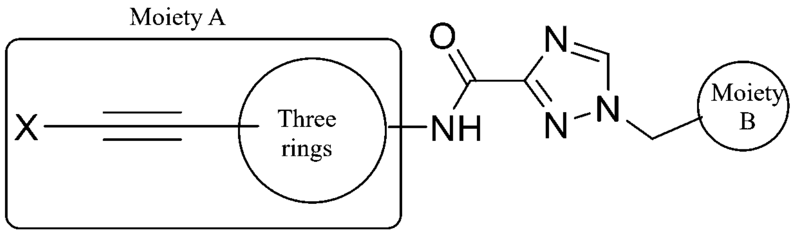

II

| No. | Moiety A | Moiety B |
|---|---|---|
| III-1 | 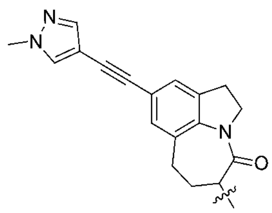 | 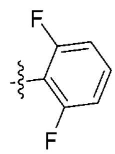 |
| III-2 | 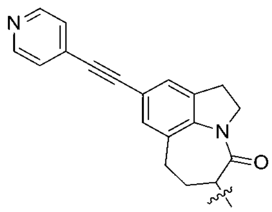 | 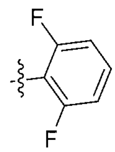 |
| III-3 | 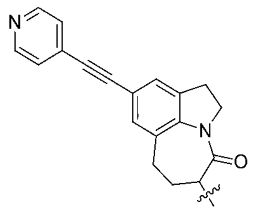 | 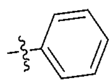 |
| III-4 | 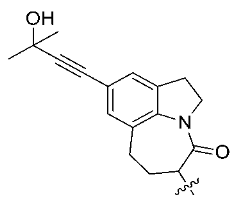 | 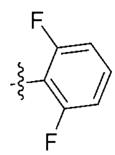 |

(continued)

| No. | Moiety A | Moiety B |
|---|---|---|
| III-5 | 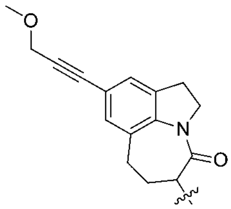 | 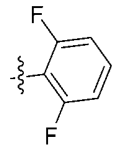 |
| III-6 | 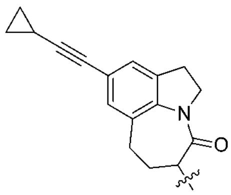 | 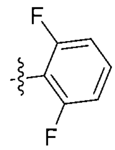 |
| III-7 | 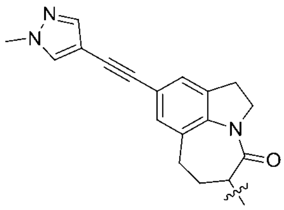 | 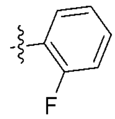 |
| III-8 | 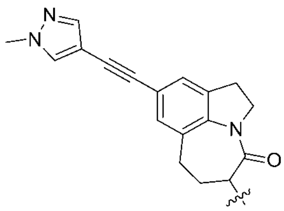 | 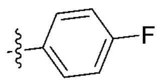 |
| III-9 | 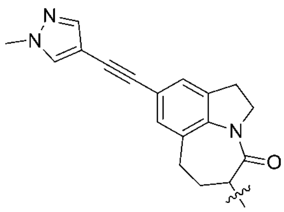 | 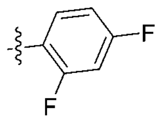 |
| III-10 | 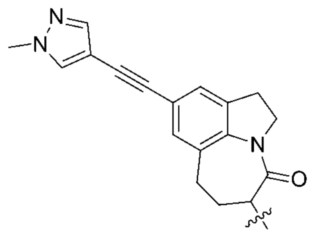 | 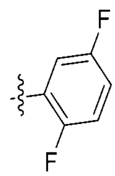 |
| III-11 | 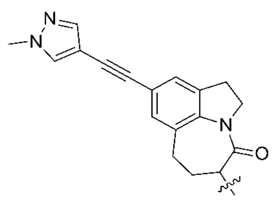 | 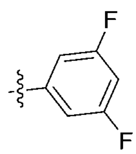 |

(continued)

| No. | Moiety A | Moiety B |
|---|---|---|
| III-12 | 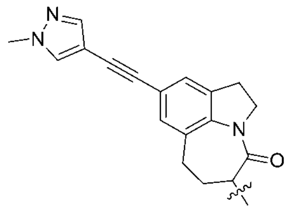 | 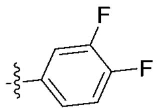 |
| III-13 | 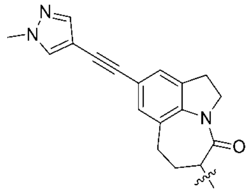 | 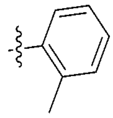 |
| III-14 | 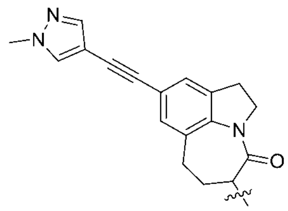 | 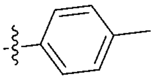 |
| III-15 | 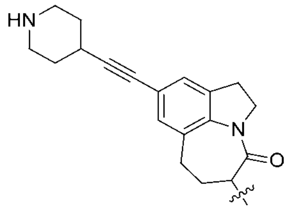 | 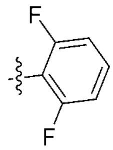 |
| III-16 | 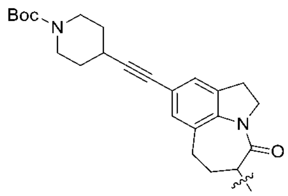 | 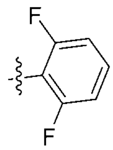 |
| III-17 | 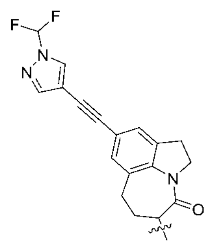 | 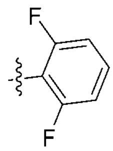 |
| III-18 | 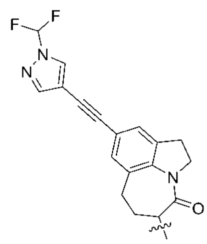 | 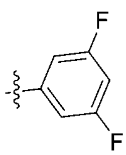 |

(continued)

| No. | Moiety A | Moiety B |
|---|---|---|
| III-19 | 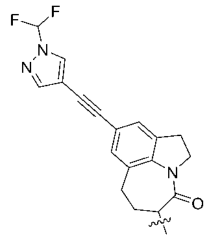 | 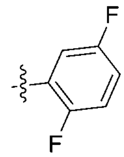 |
| III-20 | 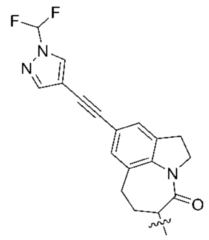 | 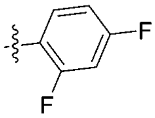 |
| III-21 | 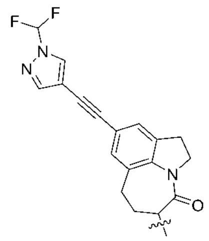 | 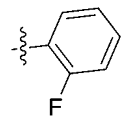 |
| III-22 | 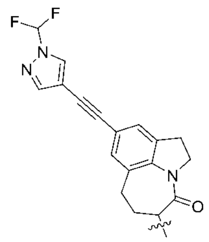 | 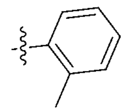 |
| III-23 | 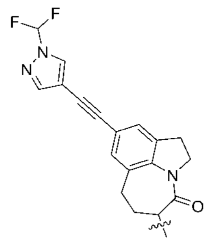 | 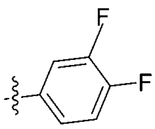 |
| III-24 | 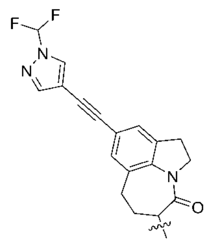 | 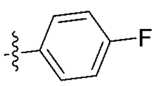 |

(continued)

| No. | Moiety A | Moiety B |
|---|---|---|
| III-25 | 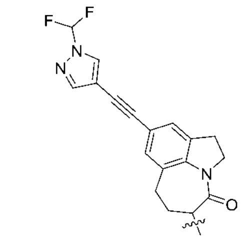 | 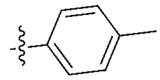 |
| III-26 | 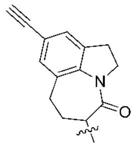 | 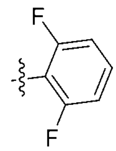 |
| III-27 | 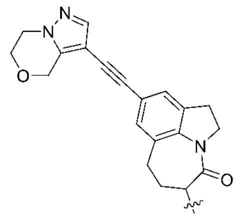 | 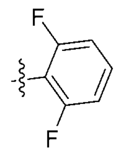 |
| III-28 | 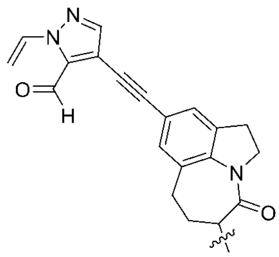 | 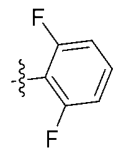 |
| III-29 | 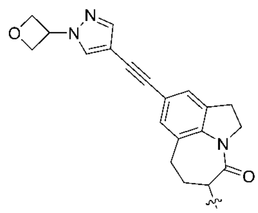 | 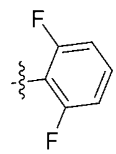 |
| III-30 | 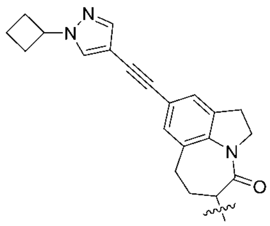 | 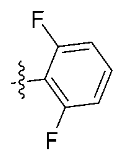 |
| III-31 | 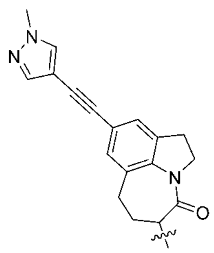 | 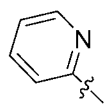 |

(continued)

| No. | Moiety A | Moiety B |
|---|---|---|
| III-32 | 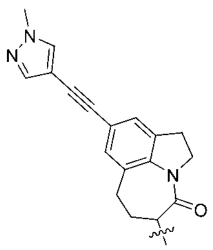 | 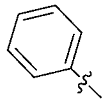 |
| IV-1 | 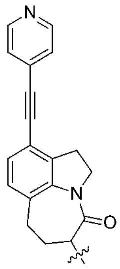 | 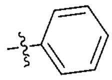 |
| X-1 | 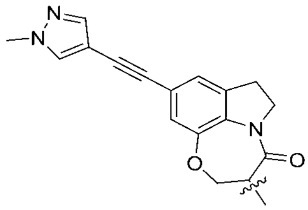 | 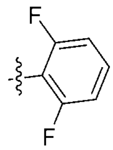 |
| XI-1 | 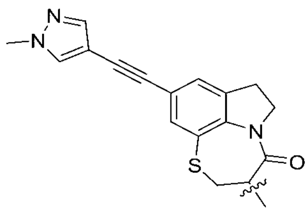 | 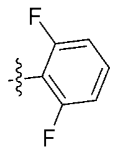 |
| XII-1 | 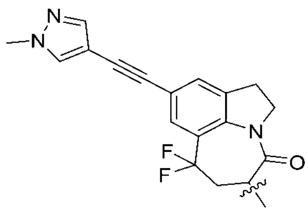 | 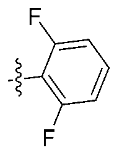 |

The structural formula of the aforementioned compound numbered V-1 or a pharmaceutically acceptable salt thereof is specifically as follows:

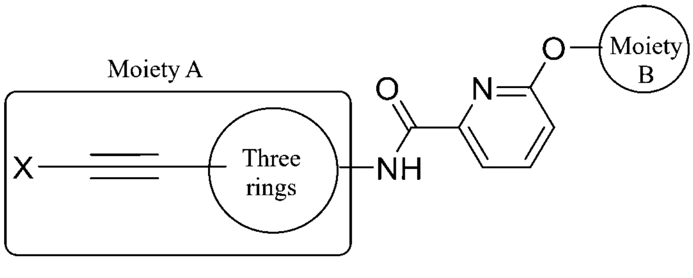

III

| No. | Moiety A | Moiety B |
|---|---|---|
| V-1 | 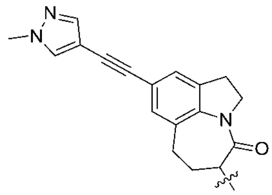 | 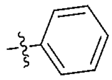 |

The structural formula of the aforementioned compound numbered V-2 or a pharmaceutically acceptable salt thereof is specifically as follows:

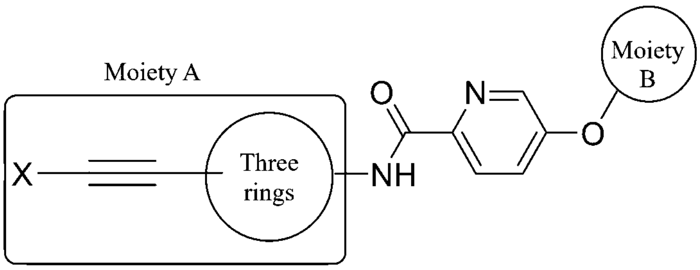

IV

| No. | Moiety A | Moiety B |
|---|---|---|
| V-2 | 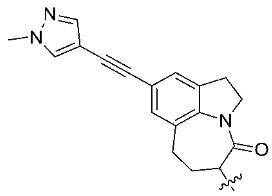 | 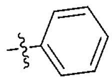 |

The structural formulas of the aforementioned compounds numbered XIII-1 and XIII-2 or pharmaceutically acceptable salts thereof are specifically as follows:

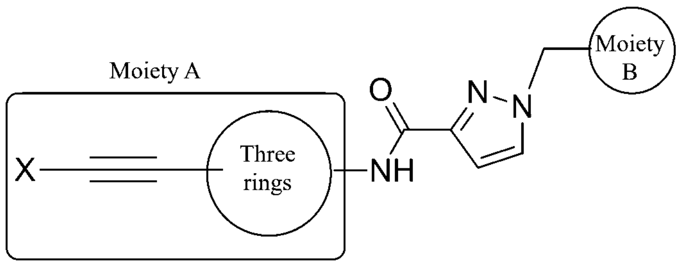

V

| No. | Moiety A | Moiety B |
|---|---|---|
| XIII-1 | 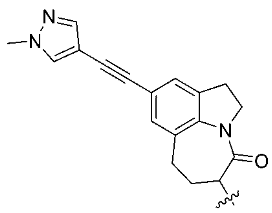 | 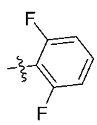 |

(continued)

| No. | Moiety A | Moiety B |
|---|---|---|
| XIII-2 | 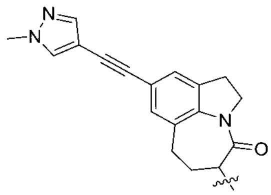 | 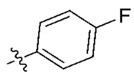 |

The structural formula of the aforementioned compound numbered XIV-1 or a pharmaceutically acceptable salt thereof is specifically as follows:

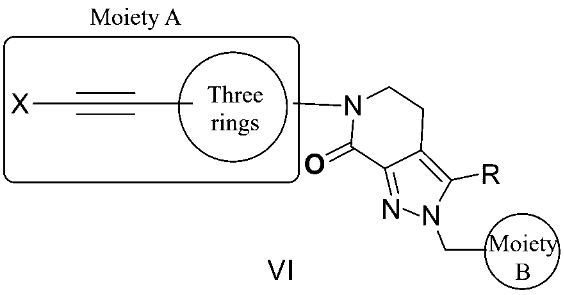

VI

| No. | Moiety A | Moiety B | Moiety R |
|---|---|---|---|
| XIV-1 | 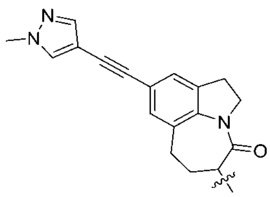 | 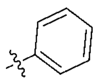 | Cl |

The structural formulas of the aforementioned compounds numbered XV-1 and XVI-1 or pharmaceutically acceptable salts thereof are specifically as follows:

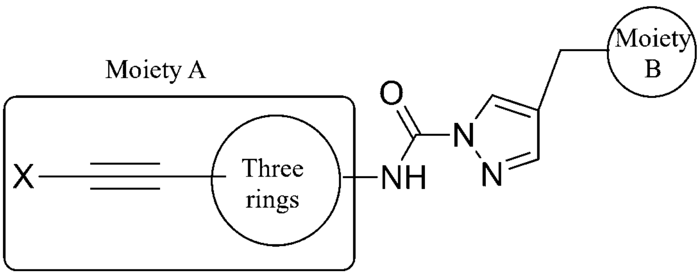

VII

| No. | Moiety A | Moiety B |
|---|---|---|
| XV-1 | 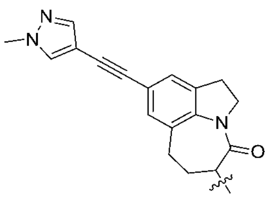 | 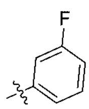 |

(continued)

| No. | Moiety A | Moiety B |
|---|---|---|
| XVI-1 | 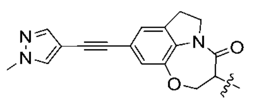 | 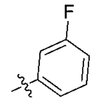 |

The "compounds" of the present disclosure include, but are not limited to, the following forms of a compound: a free base, a stereoisomer, a geometric isomer, a tautomer, an isotope, a pharmaceutically acceptable salt, a solvate, a hydrate, a prodrug (ester or phosphate ester), and the like.

The "compounds" of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be isolated in an optically active pure form or in a racemic form. The optically active pure form may be isolated from a racemic mixture or synthesized using chiral starting materials or chiral reagents.

Compounds having the aforementioned general formula structures; the terms used herein have the following meanings.

The term "pharmaceutically acceptable salt" described herein refers to a salt of the compound of the present disclosure, which is prepared from the compound having particular substituents of the present disclosure and a relatively nontoxic base. When the compound of the present disclosure contains a relatively acidic functional group, a base addition salt can be obtained by contacting the neutral form of such a compound with a sufficient amount of a base in a pure solution or a suitable inert solvent. Pharmaceutically acceptable base addition salts include, but are not limited to, sodium, potassium, calcium, magnesium salts, ammonium, or organic amines, for example, alkali metal salts, alkaline earth metal salts, other metal salts, inorganic base salts, organic base salts, inorganic acid salts, lower alkanesulfonic acid salts, arylsulfonic acid salts, organic acid salts, and amino acid salts.

In addition to the salt forms, the compounds provided by the present disclosure also have prodrug forms. Prodrugs of the compounds described herein may readily undergo chemical changes under physiological conditions and thus be converted to the compounds of the present disclosure. In addition, prodrugs can be converted to the compounds of the present disclosure in an *in vivo* environment by chemical or biochemical methods.

The term "$C_{1-6}$ alkyl", unless a different number of atoms is specified, refers to linear or branched alkane containing 1-6 carbon atoms. Examples of "alkyl" used herein include, but are not limited to, methyl, ethyl, n-propyl, n-butyl, n-pentyl, isobutyl, isopropyl, and tert-butyl. "Alkyl" also includes substituted alkyl. The alkyl may be optionally substituted one or more times with halogen or hydroxy.

The term "$C_{1-6}$ fluoroalkyl" refers to an alkyl group containing a fluorine atom, wherein alkyl is as defined above. Examples of "$C_{1-6}$ fluoroalkyl" used herein include, but are not limited to, trifluoromethyl, difluoromethyl, and 2.2.2-trifluoroethyl. "$C_{1-6}$ fluoroalkyl" also includes $C_{1-6}$ substituted fluoroalkyl. The $C_{1-6}$ substituted fluoroalkyl may be optionally substituted one or more times with halogen.

The term "$C_{1-6}$ alkoxy" refers to an -O-alkyl group, wherein alkyl is as defined above. Examples of "alkoxy" used herein include, but are not limited to, methoxy, ethoxy, n-propoxy, isopropoxy, n-butoxy, and tert-butoxy. "Alkoxy" also includes substituted alkoxy. Alkoxy may be optionally substituted one or more times with halogen.

The term "$C_{1-6}$ fluoroalkoxy" refers to an -O-alkyl group containing a fluorine atom, wherein alkyl is as defined above. Examples of "$C_{1-6}$ fluoroalkoxy" used herein include, but are not limited to, trifluoromethoxy and difluoromethoxy.

The term "$C_{1-6}$ monoalkylamino" refers to an amino group substituted with a single $C_{1-6}$ alkyl group, wherein alkyl is as defined above. Examples of "$C_{1-6}$ monoalkylamino" used herein include, but are not limited to, methylamino, ethylamino, propylamino, cyclopropylamino, and isopropylamino.

The term "$C_{1-6}$ dialkylamino" refers to an amino group substituted with two $C_{1-6}$ alkyl groups, wherein alkyl is as defined above. Examples of "$C_{1-6}$ dialkylamido" used herein include, but are not limited to, dimethylamino, diethylamino, aziridinyl, azetidinyl, azolidinyl, and azinanyl.

The term"halogen" used herein refers to fluorine, chlorine, bromine, or iodine.

The present disclosure relates to benzazepinone compounds (including those described above), as well as pharmaceutically acceptable salts, pharmaceutical compositions, stereoisomers, and anti-inflammatory and anti-tumor uses thereof.

The present disclosure also provides a pharmaceutical composition comprising at least one active ingredient and at least one pharmaceutically acceptable carrier. The active ingredient may optionally be selected from any one or more of the compound of the general formula I and preferred compounds thereof, and pharmaceutically acceptable salts, prodrugs (esters or phosphate esters), stereoisomers, deuterides, and solvates thereof.

The "pharmaceutically acceptable carrier" refers to conventional pharmaceutical carriers in the pharmaceutical

field, including conventional diluents, excipients (such as water), fillers (such as starch), binders (such as cellulose derivatives and gelatin), wetting agents (such as glycerol), disintegrants (such as agar and calcium carbonate), absorption enhancers (such as quaternary ammonium compounds), surfactants (such as cetyl alcohol), adsorption carriers (such as saccharides (sucrose and lactose), kaolin, and bentonite), and lubricants (such as talc); flavoring agents, sweeteners, and the like may also be added if necessary.

The following routes 1-5 are general reaction routes of the aforementioned compounds of the present disclosure:

**Route 1: Preparation of series I target compounds**

Synthesis scheme I:

Synthesis scheme II:

Synthesis scheme III:

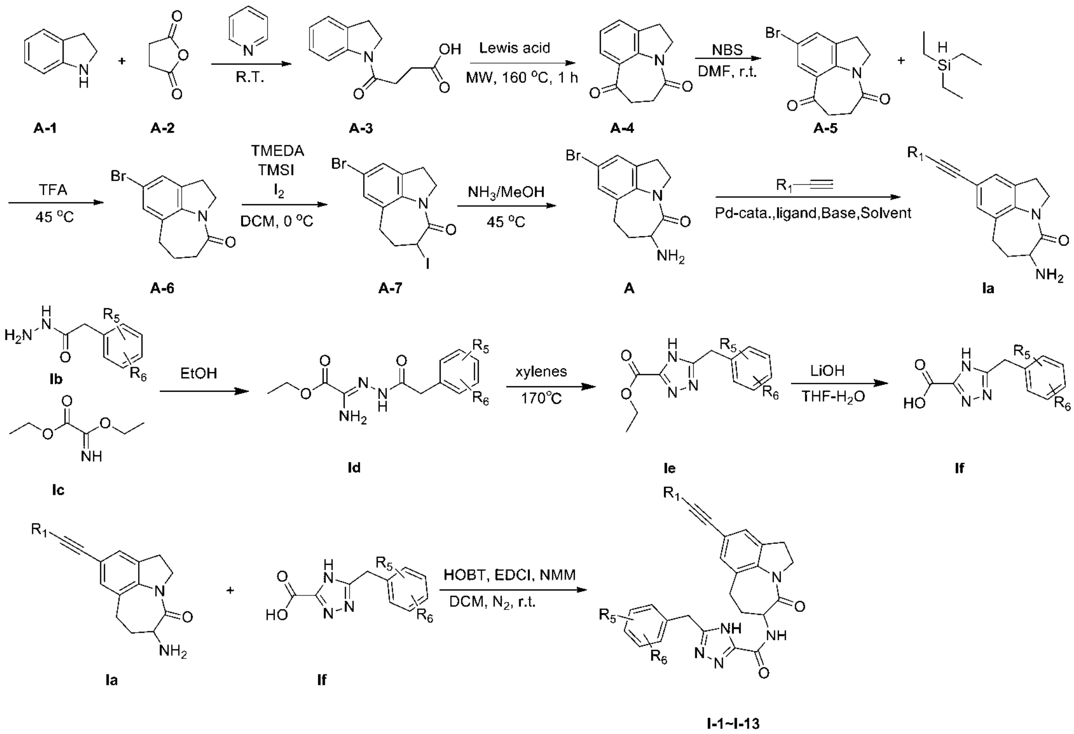

A-1 A-2 A-3 A-4 A-5
A-6 A-7 A Ia
Ib Ic Id Ie If
Ia If I-1~I-13

wherein $R_1$, $R_5$, and $R_6$ are as defined above.

The specific reaction process is as follows:

Starting materials A-1 and A-2 react under basic conditions to obtain intermediate A-3; intermediate A-3, under the catalysis of a Lewis acid catalyst (including but not limited to aluminum trichloride, ferric chloride, boron trifluoride, niobium pentachloride, HFIP, oxalyl chloride, trifluoromethanesulfonate, and the like) or an acid (polyphosphoric acid, methanesulfonic acid, trifluoroacetic acid, trifluoroacetic anhydride, or trifluoromethanesulfonic acid), undergoes a ring-closing reaction in a sealed tube or a microwave reactor to obtain a tricyclic compound A-4; intermediate **A-4** is brominated using NBS (N-bromosuccinimide) to introduce a bromine atom into the benzene ring at a position para to the nitrogen atom, thus obtaining intermediate **A-5;** under acidic conditions, the ketone carbonyl group in intermediate **A-5** is reduced using triethylsilane to obtain intermediate **A-6;** an iodine atom is introduced into intermediate **A-6** at a position ortho to amide in the presence of a Lewis acid TMSI (trimethylsilyl iodide) to obtain intermediate **A-7;** the iodo compound **A-7** undergoes a substitution reaction in a solution of ammonia in methanol to obtain intermediate **A;** intermediate **A** is coupled with substituted alkyne under the catalysis of a transition palladium metal catalyst to obtain an alkyne-substituted tricyclic compound **(Ia);** substituted phenylacetylhydrazine **(Ib)** undergoes a substitution reaction with intermediate **Ic** to obtain intermediate **Id;** then, intermediate **Id** undergoes a ring-closing reaction at a high temperature to obtain intermediate **Ie**; intermediate **Ie** is hydrolyzed under basic conditions to obtain substituted triazolecarboxylic acid **(If);** intermediates **Ia** and **If** undergo an amide condensation reaction to obtain the final target products **I-1** to **I-13**.

Transition metal palladium catalysts include, but are not limited to, bis(triphenylphosphine)palladium(II) dichloride, allylpalladium(II) chloride dimer, [1,1'-bis(diphenylphosphino)ferrocene]palladium(II) dichloride ((dppf)$PdCl_2$), palladium(II) acetate, and palladium($\pi$-cinnamyl) chloride dimer [(cinnamyl)PdCl]$_2$.

Generally, the coupling reaction is effected in the presence of a metal catalyst (including, but not limited to, combined action of a metal palladium catalyst and a metal copper catalyst (e.g., cuprous iodide)), a phosphine ligand (including, but not limited to, a BINAP ligand, a DPPF ligand, a Xantphos ligand, a DPBP ligand, a X-phos ligand, and a cBRIDP ligand), and a base (including, but not limited to, triethylamine, pyridine, diisopropylethylamine, potassium carbonate, cesium carbonate, potassium tert-butoxide, and potassium phosphate) and in a suitable solvent (including, but

not limited to, N,Ndimethylformamide, dimethylacetamide, acetonitrile, dimethoxyethane, dimethyl sulfoxide, dioxane, tetrahydrofuran, ethylene glycol dimethyl ether, toluene, and 2 wt% TPGs-750-w/$H_2O$) at an elevated temperature or under reflux conditions.

In the amide condensation reaction in Synthesis scheme III, the amide condensation agent includes, but is not limited to, a combination of HOBT and EDCI, HATU, $T_3P$, and the like.

## Route 2: Preparation of series II target compounds

Synthesis scheme I:

Synthesis scheme II:

Synthesis scheme III:

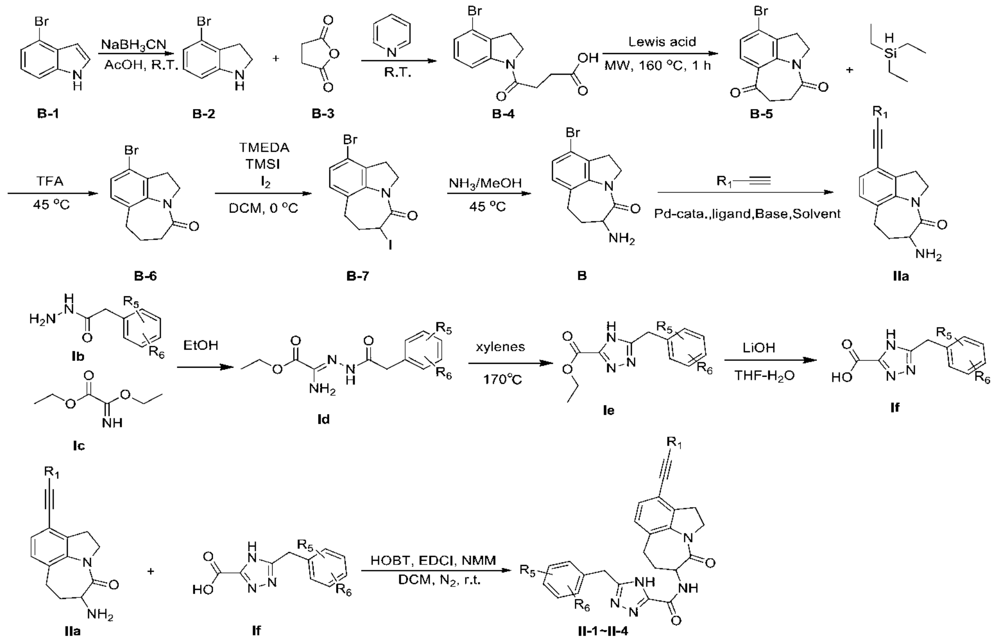


wherein $R_1$, $R_5$, and $R_6$ are as defined above.

The specific reaction process is as follows:

Starting material **B-1** is reduced by sodium cyanoborohydride under acidic conditions to obtain intermediate **B-2**; then, intermediate **B-2** reacts with intermediate **B-3** under basic conditions to obtain amide intermediate **B-4;** intermediate **B-4,** under the catalysis of a Lewis acid catalyst (including but not limited to aluminum trichloride, ferric chloride, boron trifluoride, niobium pentachloride, HFIP, oxalyl chloride, trifluoromethanesulfonate, and the like) or an acid (polyphosphoric acid, methanesulfonic acid, trifluoroacetic acid, trifluoroacetic anhydride, or trifluoromethanesulfonic acid), undergoes a ring-closing reaction in a sealed tube or a microwave reactor to obtain a tricyclic compound **B-5;** under acidic conditions, the ketone carbonyl group in intermediate **B-5** is reduced using triethylsilane to obtain intermediate **B-6;** an iodine atom is introduced into intermediate **B-6** at a position ortho to amide in the presence of a Lewis acid TMSI (trimethylsilyl iodide) to obtain intermediate **B-7;** the iodo compound **B-7** undergoes a substitution reaction in a solution of ammonia in methanol to obtain intermediate **B;** intermediate **B** is coupled with substituted alkyne under the catalysis of a transition metal palladium catalyst to obtain an alkyne-substituted tricyclic compound **IIa;** substituted phenylacetylhydrazine **(Ib)** undergoes a substitution reaction with intermediate **Ic** to obtain intermediate **Id;** then, intermediate **Id** undergoes a ring-closing reaction at a high temperature to obtain substituted triazole carboxylic acid ester **(Ie);** intermediate **Ie** is hydrolyzed under basic conditions to obtain substituted triazole carboxylic acid **(If);** intermediate **IIa** and intermediate **If** undergo an amide condensation reaction to obtain the final target products **II-1 to II-4.**

Transition metal palladium catalysts include, but are not limited to, bis(triphenylphosphine)palladium(II) dichloride, allylpalladium(II) chloride dimer, [1,1'-bis(diphenylphosphino)ferrocene]palladium(II) dichloride ((dppf)$PdCl_2$), palladium(II) acetate, and palladium($\pi$-cinnamyl) chloride dimer [(cinnamyl)$PdCl]_2$.

Generally, the coupling reaction is effected in the presence of a metal catalyst (including, but not limited to, combined action of a metal palladium catalyst and a metal copper catalyst (e.g., cuprous iodide)), a phosphine ligand (including, but not limited to, a BINAP ligand, a DPPF ligand, a Xantphos ligand, a DPBP ligand, a X-phos ligand, and a cBRIDP ligand), and a base (including, but not limited to, triethylamine, pyridine, diisopropylethylamine, potassium carbonate, cesium carbonate, potassium tert-butoxide, and potassium phosphate) and in a suitable solvent (including, but not limited to, N,Ndimethylformamide, dimethylacetamide, acetonitrile, dimethoxyethane, dimethyl sulfoxide, dioxane, tetrahydrofuran, ethylene glycol dimethyl ether, toluene, and 2 wt% TPGs-750-w/$H_2O$) at an elevated temperature or under reflux conditions.

In the amide condensation reaction in Synthesis scheme III, the amide condensation agent includes, but is not

limited to, a combination of HOBT and EDCI, HATU, $T_3P$, and the like.

## Route 3: Preparation of series III target compounds

Synthesis scheme I:

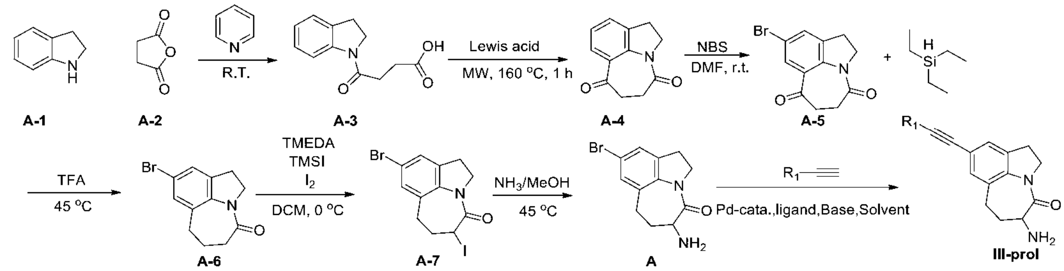


A-1 A-2 A-3 A-4 A-5 A-6 A-7 A III-prol

Synthesis scheme II:

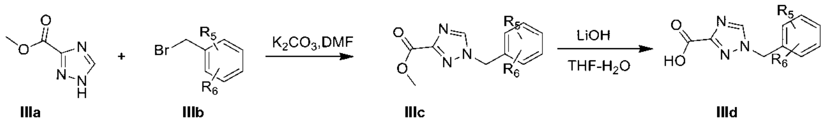


IIIa IIIb IIIc IIId

Synthesis scheme III:

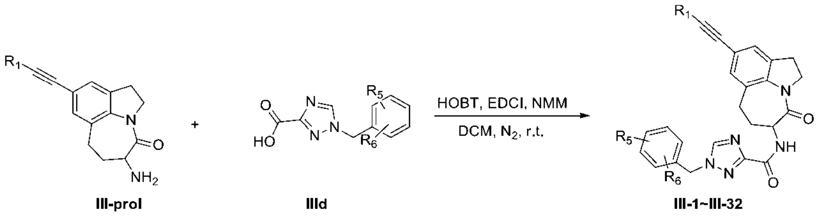


III-prol IIId III-1~III-32

wherein $R_1$, $R_5$, and $R_6$ are as defined above.

The specific reaction process is as follows:

Starting materials **A-1** and **A-2** undergo a substitution reaction under basic conditions to obtain intermediate **A-3;** intermediate **A-3,** under the catalysis of a Lewis acid catalyst (including but not limited to aluminum trichloride, ferric chloride, boron trifluoride, niobium pentachloride, HFIP, oxalyl chloride, trifluoromethanesulfonate, and the like) or an acid (polyphosphoric acid, methanesulfonic acid, trifluoroacetic acid, trifluoroacetic anhydride, or trifluoromethanesulfonic acid), undergoes a ring-closing reaction in a sealed tube or a microwave reactor to obtain a tricyclic compound **A-4;** intermediate **A-4** is brominated using NBS (N-bromosuccinimide) to introduce a bromine atom into the benzene ring at a position para to the nitrogen atom, thus obtaining intermediate **A-5;** under acidic conditions, the ketone carbonyl group in intermediate **A-5** is reduced using triethylsilane to obtain intermediate **A-6;** an iodine atom is introduced into intermediate **A-6** at a position ortho to amide in the presence of a Lewis acid TMSI (trimethylsilyl iodide) to obtain intermediate **A-7;** the iodo compound **A-7** undergoes a substitution reaction in a solution of ammonia in methanol to obtain intermediate **A;** intermediate **A** is coupled with substituted alkyne under the catalysis of a transition metal catalyst palladium to obtain an alkyne-substituted tricyclic compound **(III-prol);** substituted benzyl bromide **(IIIb)** undergoes a substitution reaction with triazole carboxylic acid methyl ester **(IIIa)** to obtain intermediate **IIIc;** then, intermediate **IIIc** is hydrolyzed under basic conditions to obtain substituted triazole carboxylic acid **(IIId);** intermediates **III-prol** and **IIId** undergo an amide condensation reaction to obtain the final products **III-1 to III-16.**

Transition metal palladium catalysts include, but are not limited to, bis(triphenylphosphine)palladium(II) dichloride, allylpalladium(II) chloride dimer, [1,1'-bis(diphenylphosphino)ferrocene]palladium(II) dichloride ((dppf)$PdCl_2$), palladium(II) acetate, and palladium($\pi$-cinnamyl) chloride dimer [(cinnamyl)$PdCl]_2$.

Generally, the coupling reaction is effected in the presence of a metal catalyst (including, but not limited to, combined action of a metal palladium catalyst and a metal copper catalyst (e.g., cuprous iodide)), a phosphine ligand (including, but not limited to, a BINAP ligand, a DPPF ligand, a Xantphos ligand, a DPBP ligand, a X-phos ligand, and a cBRIDP ligand), and a base (including, but not limited to, triethylamine, pyridine, diisopropylethylamine, potassium carbonate, cesium carbonate, potassium tert-butoxide, and potassium phosphate) and in a suitable solvent (including, but not limited to, N,Ndimethylformamide, dimethylacetamide, acetonitrile, dimethoxyethane, dimethyl sulfoxide, dioxane, tetrahydrofuran, ethylene glycol dimethyl ether, toluene, and 2 wt% TPGs-750-w/$H_2O$) at an elevated temperature or under reflux conditions.

In the amide condensation reaction in Synthesis scheme III, the amide condensation agent includes, but is not limited to, a combination of HOBT and EDCI, HATU, $T_3P$, and the like.

## Route 4: Preparation of series IV target compounds

**Synthesis scheme I:**

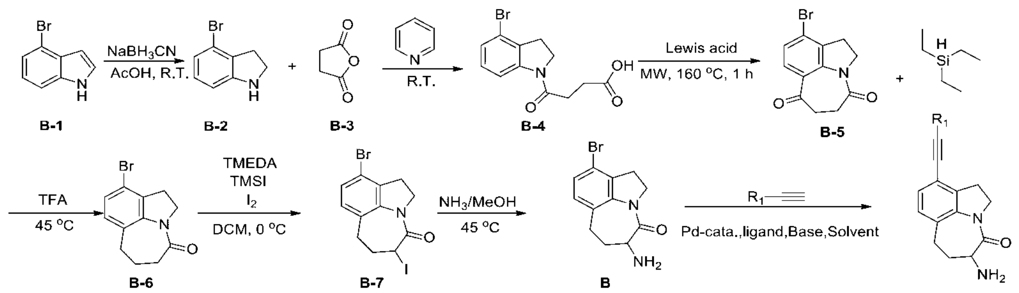

B-1 B-2 B-3 B-4 B-5 B-6 B-7 B IV-prol

**Synthesis scheme II:**

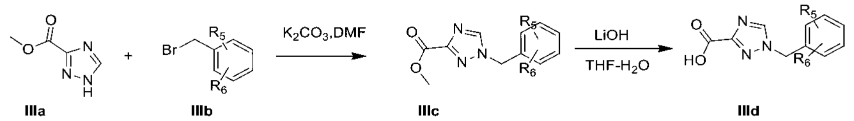

IIIa IIIb IIIc IIId

**Synthesis scheme III:**

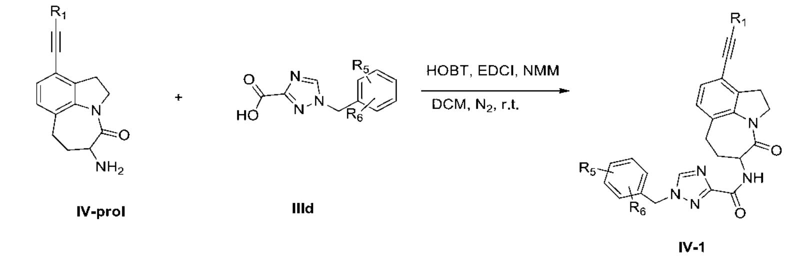

IV-prol IIId IV-1

wherein $R_1$, $R_5$, and $R_6$ are as defined above.

The specific reaction process is as follows:

Starting material **B-1** is reduced by sodium cyanoborohydride under acidic conditions to obtain intermediate **B-2;** then, intermediate **B-2** reacts with intermediate **B-3** under basic conditions to obtain amide intermediate **B-4;** intermediate **B-4,** under the catalysis of a Lewis acid catalyst (including but not limited to aluminum trichloride, ferric chloride, boron trifluoride, niobium pentachloride, HFIP, oxalyl chloride, trifluoromethanesulfonate, and the like) or an acid (polyphosphoric acid, methanesulfonic acid, trifluoroacetic acid, trifluoroacetic anhydride, or trifluoromethanesulfonic acid), undergoes a ring-closing reaction in a sealed tube or a microwave reactor to obtain a tricyclic compound **B-5;** under acidic conditions, the ketone carbonyl group in intermediate **B-5** is reduced using triethylsilane to obtain intermediate **B-6;** an iodine atom is introduced into intermediate **B-6** at a position ortho to amide in the presence of a Lewis acid TMSI (trimethylsilyl iodide) to obtain intermediate **B-7;** the iodo compound **B-7** undergoes a substitution reaction in a solution of ammonia in methanol to obtain intermediate **B;** intermediate **B** is coupled with substituted alkyne under the catalysis of a transition metal catalyst palladium to obtain an alkyne-substituted tricyclic compound **IV-prol;** substituted benzyl bromide **(IIIb)** undergoes a substitution reaction with triazole carboxylic acid methyl ester **(IIIa)** to obtain intermediate **IIIc;** then, intermediate **IIIc** is hydrolyzed under basic conditions to obtain substituted triazole carboxylic acid **(IIId);** intermediates **IV-prol** and **IIId** undergo an amide condensation reaction to obtain the final target product IV-1.

Transition metal palladium catalysts include, but are not limited to, bis(triphenylphosphine)palladium(II) dichloride, allylpalladium(II) chloride dimer, [1,1'-bis(diphenylphosphino)ferrocene]palladium(II) dichloride ((dppf)$PdCl_2$), palladium(II) acetate, and palladium($\pi$-cinnamyl) chloride dimer [(cinnamyl)PdCl]$_2$.

Generally, the coupling reaction is effected in the presence of a metal catalyst (including, but not limited to, combined action of a metal palladium catalyst and a metal copper catalyst (e.g., cuprous iodide)), a phosphine ligand (including, but not limited to, a BINAP ligand, a DPPF ligand, a Xantphos ligand, a DPBP ligand, a X-phos ligand, and a cBRIDP ligand), and a base (including, but not limited to, triethylamine, pyridine, diisopropylethylamine, potassium carbonate, cesium carbonate, potassium tert-butoxide, and potassium phosphate) and in a suitable solvent (including, but not limited to, N,Ndimethylformamide, dimethylacetamide, acetonitrile, dimethoxyethane, dimethyl sulfoxide, dioxane, tetrahydrofuran, ethylene glycol dimethyl ether, toluene, and 2 wt% TPGs-750-w/$H_2O$) at an elevated temperature or under reflux conditions.

In the amide condensation reaction in Synthesis scheme III, the amide condensation agent includes, but is not limited to, a combination of HOBT and EDCI, HATU, $T_3P$, and the like.

## Route 5: Preparation of series V target compounds

Synthesis scheme I:

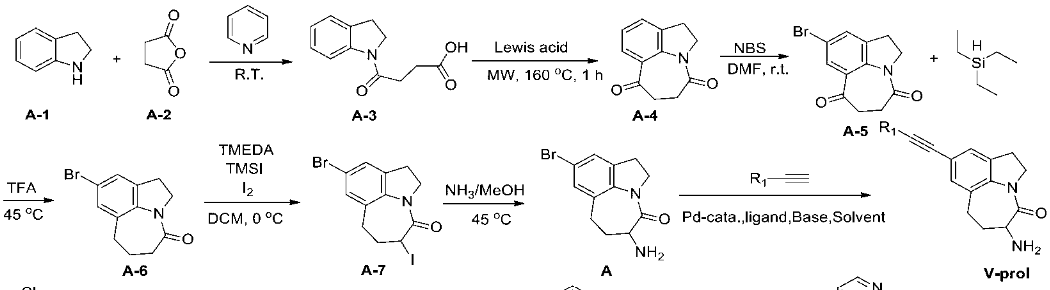


Synthesis scheme II:

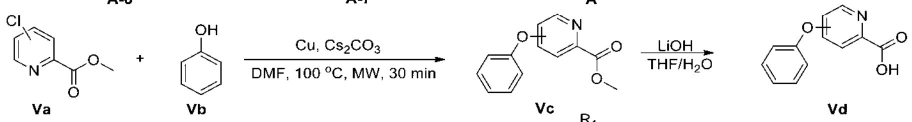


Synthesis scheme III:

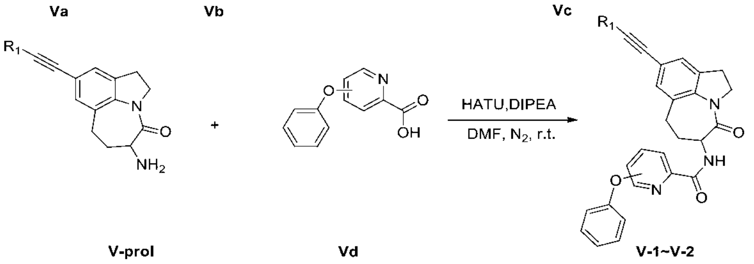


wherein, $R_1$ is as defined above;

The specific reaction process is as follows:

Starting materials A-1 and A-2 undergo a substitution reaction under basic conditions to obtain intermediate A-3; intermediate A-3, under the catalysis of a Lewis acid catalyst (including but not limited to aluminum trichloride, ferric chloride, boron trifluoride, niobium pentachloride, HFIP, oxalyl chloride, trifluoromethanesulfonate, and the like) or an acid (polyphosphoric acid, methanesulfonic acid, trifluoroacetic acid, trifluoroacetic anhydride, or trifluoromethanesulfonic acid), undergoes a ring-closing reaction in a sealed tube or a microwave reactor to obtain a tricyclic compound **A-4;** intermediate **A-4** is brominated using NBS (N-bromosuccinimide) to introduce a bromine atom into the benzene ring at a position para to the nitrogen atom, thus obtaining intermediate **A-5;** under acidic conditions, the ketone carbonyl group in intermediate **A-5** is reduced using triethylsilane to obtain intermediate **A-6;** an iodine atom is introduced into intermediate **A-6** at a position ortho to amide in the presence of a Lewis acid TMSI (trimethylsilyl iodide) to obtain intermediate **A-7;** the iodo compound **A-7** undergoes a substitution reaction in a solution of ammonia in methanol to obtain intermediate **A;** intermediate **A** is coupled with substituted alkyne under the catalysis of a transition metal catalyst palladium to obtain an alkyne-substituted tricyclic compound **(V-prol);** intermediates **Va** with **Vb** undergo a substitution reaction to obtain intermediate **Vc;** then, intermediate **Vc** is hydrolyzed under basic conditions to obtain intermediate-substituted pyridine carboxylic acid **Vd;** intermediates **V-prol** and **Vd** undergo an amide condensation reaction to obtain the final products **V-1 to V-2.**

Transition metal palladium catalysts include, but are not limited to, bis(triphenylphosphine)palladium(II) dichloride, allylpalladium(II) chloride dimer, [1,1'-bis(diphenylphosphino)ferrocene]palladium(II) dichloride ((dppf)$PdCl_2$), palladium(II) acetate, and palladium($\pi$-cinnamyl) chloride dimer [(cinnamyl)PdCl]$_2$.

Generally, the coupling reaction is effected in the presence of a metal catalyst (including, but not limited to, combined action of a metal palladium catalyst and a metal copper catalyst (e.g., cuprous iodide)), a phosphine ligand (including, but not limited to, a BINAP ligand, a DPPF ligand, a Xantphos ligand, a DPBP ligand, a X-phos ligand, and a cBRIDP ligand), and a base (including, but not limited to, triethylamine, pyridine, diisopropylethylamine, potassium carbonate, cesium carbonate, potassium tert-butoxide, and potassium phosphate) and in a suitable solvent (including, but not limited to, N,Ndimethylformamide, dimethylacetamide, acetonitrile, dimethoxyethane, dimethyl sulfoxide, dioxane, tetrahydrofuran, ethylene glycol dimethyl ether, toluene, and 2 wt% TPGs-750-w/$H_2O$) at an elevated temperature or under reflux conditions.

In the amide condensation reaction in Synthesis scheme III, the amide condensation agent includes, but is not limited to, a combination of HOBT and EDCI, HATU, $T_3P$, and the like.

Compared with the prior art, the present disclosure has the following beneficial effects:

(1) The *in vitro* activity test shows that most of the molecules in this patent show excellent anti-tumor activity;

(2) The compounds of the present application not only exhibit effective activity against systemic inflammatory responses but also exhibit superior activity compared to the positive control compound GSK2982772;

(3) Compared with the compound of Example 13 in CN201910518248X, some of the target compounds of the present disclosure significantly improve the exposure and bioavailability and demonstrate good pharmacokinetic characteristics.

In summary, the compounds of the present application have significant anti-tumor activity and/or anti-inflammatory activity and good pharmacokinetic activity, and demonstrate great clinical application prospects.

**DETAILED DESCRIPTION**

The following are specific examples of the present disclosure to further describe the technical solutions of the present disclosure, but the protection scope of the present disclosure is not limited to these examples. Any modification or equivalent replacement without departing from the concept of the present disclosure falls within the protection scope of the present disclosure.

In addition, all operations involving easily oxidizable or hydrolyzable materials are performed under a nitrogen atmosphere. Unless otherwise stated, the starting materials used in the present disclosure are commercially available and can be used without further purification.

The starting materials and common intermediates for each reaction involved in the examples of the present disclosure can be commercially available or self-made, wherein the preparation process of the starting materials and common intermediates that need to be self-made is described in detail below.

Explanation of abbreviations of solvents or materials used:
NBS: N-bromosuccinimide; TMEDA: tetramethylethylenediamine; TMSI: trimethylsilyl iodide; DMF: *N,N*-dimethylformamide; $I_2$: elemental iodine; DCM: dichloromethane; $NH_3$/MeOH: a solution of ammonia in methanol; EtOH: ethanol; Xylenes: xylol; LiOH: lithium hydroxide; THF-$H_2O$: a solution of tetrahydrofuran in water; $NaBH_3CN$: sodium cyanoborohydride; AcOH: acetic acid; TFA: trifluoroacetic acid; HOBT: 1-hydroxybenzotriazole; EDCI: 1-(3-dimethylaminopropyl)-3-ethylcarbodiimide hydrochloride; NMM: N-methylmorpholine; $K_2CO_3$: potassium carbonate; Cu: copper; $Cs_2CO_3$: cesium carbonate; HATU: O-(7-azabenzotriazol-1-yl)-*N,N,N',N'*-tetramethyluronium hexafluorophosphate; DIPEA: N,N-diisopropylethylamine; $T_3P$: 1-propylphosphonic anhydride; THF: tetrahydrofuran; EA: ethyl acetate; BINAP ligand: S-(-)-2,2'-bis(diphenylphosphino)-1,1'-binaphthyl ligand; DPPF ligand: 1,1'-bis(diphenylphosphino)ferrocene ligand; Xantphos ligand: 4,5-bisdiphenylphosphino-9,9-dimethylxanthene ligand; DPBP ligand: 2,2'-bis(diphenylphosphino) benzophenone ligand; X-phos ligand: 2-dicyclohexylphosphino-2',4',6'-triisopropylbiphenyl ligand; cBRIDP ligand: dicyclohexyl(2,2-diphenyl-1-methylcyclopropyl)phosphine ligand; HFIP: hexafluoroisopropanol.

**I. Preparation of common intermediate** A

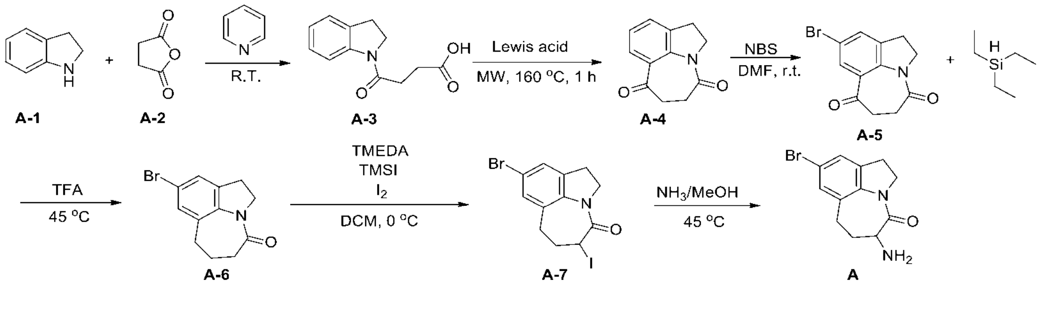


Step 1: Synthesis of compound A-3

In a 250 mL eggplant-shaped flask, the starting material A-1 (5.96 g, 50 mmol) was dissolved in 60 mL of anhydrous pyridine, and the mixture was stirred in an ice bath. The starting material A-2 (5.00 g, 50 mmol) was slowly added in portions. After nitrogen purging, the ice bath was removed, and the system was warmed to room temperature and allowed to react overnight. After thin layer chromatography showed that the reaction of the starting materials was substantially completed, 6 M hydrochloric acid was added to adjust the system to be acidic, and the system was extracted with EA three times. The organic phase was washed with brine, dried over anhydrous sodium sulfate, and concentrated. The crude product was purified by column chromatography to give compound A-3 in the form of a pink solid (10.5 g, yield: 95.8%).

MS(m/z): 220.1 $[M+H]^+$.

Step 2: Synthesis of compound A-4

In a 50 mL microwave reaction flask, intermediate **A-3** (1.75 g, 8.0 mmol) was dissolved in 15 mL of trifluoromethanesulfonic acid solution, and $AlCl_3$ (catalytic amount) was added in portions. The system was purged with nitrogen, placed in a microwave reactor, and allowed to react at 160 °C for 1 h. After thin layer chromatography showed that the reaction of the starting materials was substantially completed, a sodium hydroxide solution was added to adjust the system to be neutral to slightly alkaline, and the system was extracted with DCM. The organic phase was washed with brine, dried over anhydrous sodium sulfate, and concentrated. The crude product was purified by column chromatography to give compound **A-4** in the form of a gray solid (2.40 g, yield: 34.5%).
MS(m/z): 202.1 $[M+H]^+$.

**Step 3: Synthesis of compound A-5**

In a 250 mL eggplant-shaped flask, intermediate **A-4** (3.6 g, 18.0 mmol) was dissolved in 60 mL of anhydrous DMF, and then NBS (6.37 g, 36 mmol) was added in portions to the reaction system. The system was purged with nitrogen and allowed to react overnight at room temperature. After thin layer chromatography showed that the reaction of the starting materials was substantially completed, water was added to quench the reaction, and the system was extracted with ethyl acetate. The organic phase was washed with brine, dried over anhydrous sodium sulfate, and concentrated. The crude product was purified by column chromatography to give compound **A-5** in the form of a yellow solid, which was directly used in the next step without further purification.
MS (m/z): 280.1 $[M+H]^+$.

**Step 4: Synthesis of compound A-6**

In a 250 mL eggplant-shaped flask, **A-5** (5.0 g, 18 mmol) was dissolved in 60 mL of anhydrous trifluoroacetic acid in an ice bath, and triethylsilane (10.4 g, 90 mmol) was slowly added in portions under ice-bath cooling. After nitrogen purging, the ice bath was removed, and the system was slowly warmed to room temperature and finally transferred to a 45 °C oil bath for reaction overnight. After thin layer chromatography showed that the reaction of the starting materials was substantially completed, saturated sodium bicarbonate was added to the system to adjust the pH to > 7, and the system was extracted with ethyl acetate. The organic phase was washed with brine, dried over anhydrous sodium sulfate, and concentrated. The crude product was purified by column chromatography to give compound **A-6** in the form of a greyish-green solid (2.43 g); the total yield of steps 3 and 4 was 51.05%.
MS(m/z): 266.1 $[M+H]^+$.

**Step 5: Synthesis of compound A-7**

In a 250 mL eggplant-shaped flask, intermediate **A-6** (0.81 g, 3.04 mmol) was dissolved in 30 mL of dichloromethane, and the solution was stirred in an ice bath. TMEDA (1.06 g, 9.12 mmol) and TMSI (1.83 g, 9.12 mmol) were slowly added in portions. After nitrogen purging, the system was stirred in an ice bath for 2 h. $I_2$ (1.08 g, 4.26 mmol) was added, and the system was purged with nitrogen and stirred for 2 h in an ice bath. After thin layer chromatography showed that the reaction of the starting materials was substantially completed, saturated sodium bicarbonate was added to quench the reaction, and the system was extracted with DCM. The organic phases were combined, washed with saturated brine, dried over anhydrous sodium sulfate, filtered, and concentrated to dryness by rotary evaporation to give compound **A-7** in the form of a gray solid, which was directly used in the next step without further purification.
MS(m/z): 391.9 $[M+H]^+$.

**Step 6: Synthesis of compound A**

In a 100 mL eggplant-shaped flask, intermediate **A-7** in the previous step was dissolved in a $NH_3$/MeOH (45 mL) solution. The solution was purged with nitrogen three times in vacuo, placed in a 45 °C oil bath, and allowed to react overnight in a sealed tube. After thin layer chromatography showed that the reaction of the starting materials was substantially completed, the system was concentrated to dryness by rotary evaporation. The crude product was purified by column chromatography to give compound **A** in the form of a brown solid (0.4 g); the total yield of steps 5 and 6 was 46.87%.
MS(m/z): 281.1 $[M+H]^+$.
$^1H$ NMR (400 MHz, DMSO-$d_6$) $\delta$ 8.40 (br, 2H), 7.37 (s, 1H), 7.28 (s, 1H), 4.28-4.21 (m, 1H), 4.18-4.12 (m, 1H), 3.95-3.85 (m, 1H), 3.38-3.34 (m, 2H), 3.24-3.21 (m, 2H), 2.14-2.06(m, 1H), 2.07-1.94 (m, 1H).

## II. Preparation of common intermediate B

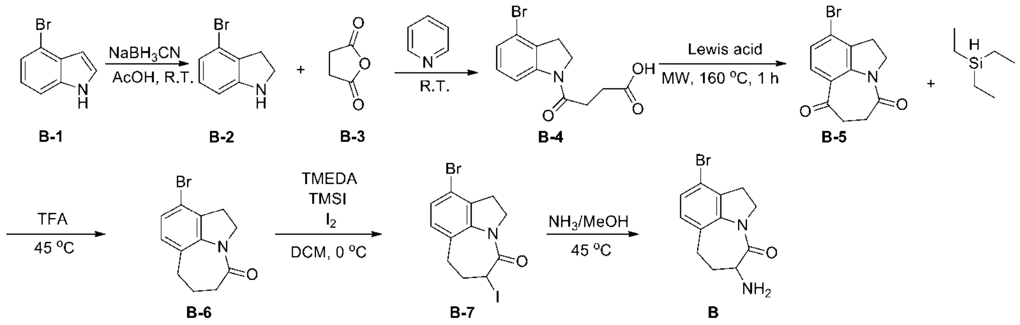


### Step 1: Synthesis of compound B-2

In a 100 mL eggplant-shaped flask, the starting material **B-1** (1.95 g, 10 mmol) was dissolved in 40 mL of acetic acid. After nitrogen purging, $NaBH_3CN$ (1.89 g, 30 mmol) was added in portions in an ice bath, and the system was warmed to room temperature and allowed to react for 4 h. After thin layer chromatography showed that the reaction of the starting materials was substantially completed, an aqueous sodium hydroxide solution was added dropwise to the system to adjust the pH to > 7, and the system was extracted with EA three times. The organic phases were combined, washed with brine, dried over anhydrous sodium sulfate, and filtered, and the filtrate was concentrated under reduced pressure to give a crude product in the form of a brown liquid. The organic layer was dried and concentrated. The crude product was purified by silica gel column chromatography to give compound **B-2** in the form of a pale yellow liquid (1.87 g, yield: 94.8%).

MS (m/z): 198.2 $[M+H]^+$.

### Step 2: Synthesis of compound B-4

In a 100 mL eggplant-shaped flask, intermediate **B-2** (1.87 g, 9.5 mmol) was dissolved in 20 mL of anhydrous pyridine, and the system was stirred in an ice bath. After nitrogen purging, compound **B-3** (0.95 g, 9.5 mmol) was added in portions, the ice bath was removed, and the system was allowed to react overnight at room temperature. After thin layer chromatography showed that the reaction of the starting materials was substantially completed, 6 M hydrochloric acid was added to adjust the system to be acidic, and the system was extracted with EA three times. The organic phases were combined, washed with brine, dried over anhydrous sodium sulfate, and filtered, and the filtrate was concentrated under reduced pressure to give a crude product in the form of a brown liquid. The organic layer was dried and concentrated. The crude product was purified by silica gel column chromatography to give compound **B-4** in the form of a pink solid (2.79 g, yield: 99.7%).

MS(m/z): 298.3 $[M+H]^+$.

### Step 3: Synthesis of compound B-5

In a 50 mL microwave reaction flask, intermediate **B-4** (2.79 g, 9.4 mmol) was dissolved in 45 mL of trifluoromethanesulfonic acid solution, and then $AlCl_3$ (catalytic amount) was added to the reaction system. The reaction system was purged with nitrogen, placed in a microwave reactor, and allowed to react at 160 °C for 1 h. After thin layer chromatography showed that the reaction of the starting materials was substantially completed, a 1 M sodium hydroxide solution was added to adjust the system to be alkaline, and the system was extracted with DCM. The organic phases were combined, washed with brine, dried over anhydrous sodium sulfate, and concentrated to give a crude product, compound **B-5** in the form of a gray solid (1.56 g), which was directly used in the next step without purification.

MS(m/z): 280.1 $[M+H]^+$.

### Step 4: Synthesis of compound B-6

In a 100 mL eggplant-shaped flask, the purified intermediate **B-5** (1.56 g, 5.6 mmol) was dissolved in 50 mL of trifluoroacetic acid. The solution was cooled in an ice bath, and triethylsilane (3.24 g, 27.9 mmol) was slowly added. After nitrogen purging, the system was transferred to 45 °C environment and allowed to react overnight. After thin layer chromatography showed that the reaction of the starting materials was substantially completed, saturated sodium

bicarbonate was added to the system to adjust the pH to > 7, and the system was extracted with EA. The organic phase was washed with brine, dried over anhydrous sodium sulfate, and concentrated. The crude product was purified by silica gel column chromatography to give compound **B-6** in the form of a pale yellow liquid (1.35 g, yield: 91.2%).

MS (m/z): 266.0 [M+H]+.

**Step 5: Synthesis of compound B-7**

In a 100 mL eggplant-shaped flask, intermediate **B-6** (1.35 g, 5.1 mmol) was dissolved in 50 mL of dichloromethane, and the solution was stirred in an ice bath. TMEDA (1.52 g, 13.1 mmol) and TMSI (2.62 g, 13.1 mmol) were added in portions. After nitrogen purging, the system was stirred in an ice bath for 2 h. $I_2$ (1.81 g, 7.1 mmol) was added, and the system was purged with nitrogen and stirred for 2 h in an ice bath. After thin layer chromatography showed that the reaction of the starting materials was substantially completed, water was added dropwise to quench the reaction, and the system was extracted with DCM. The organic phase was washed with saturated brine, dried over anhydrous sodium sulfate, filtered, and concentrated to dryness by rotary evaporation to give **B-7** in the form of a solid substance, which was directly used in the next step without further purification.

MS(m/z): 391.9 [M+H]+.

**Step 6: Synthesis of compound B**

In a 250 mL eggplant-shaped flask, intermediate **B-7** was dissolved in a $NH_3$/MeOH (100 mL) solution. After nitrogen purging, the system was allowed to react overnight at 45 °C in a sealed tube. After thin layer chromatography showed that the reaction of the starting materials was substantially completed, the reaction solution was concentrated to dryness by rotary evaporation. The crude product was purified by silica gel column chromatography to give compound **B** in the form of a brown solid powder (1.12 g, yield: 78.5%).

MS(m/z): 281.0 [M+H]+.

**Example 1: Preparation of Target Compound** 5-benzyl-*N*-(9-(cyclopropylethynyl)-4-oxo-1,2,3,4,6,7-hexahydroazepino[3,2,1-hi]indol-3-yl)-4H-1,2,4-triazole-3-carboxamide **(I-1)**

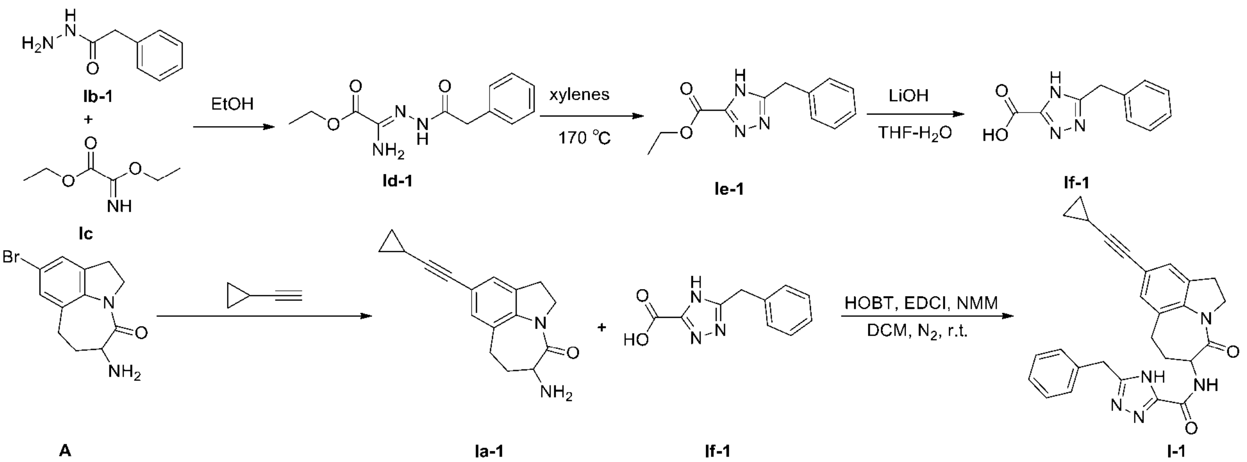


Ib-1 + Ic; Id-1; Ie-1; If-1; A; Ia-1; If-1; I-1

**Step 1: Synthesis of compound Id-1**

In a 100 mL eggplant-shaped reaction flask, the starting material **Ib-1** (1.8 g, 12 mmol) was dissolved in a mixed solvent of 7.5 mL of ethanol and 22.5 mL of diethyl ether. The system was cooled in an ice bath, and the starting material **Ic** (1.8 g, 12.4 mmol) was slowly added dropwise. After the dropwise addition was completed, the system was purged with nitrogen, and the ice bath was removed. The system was slowly warmed to room temperature and allowed to react for 2 h. After thin layer chromatography showed that the reaction of the starting materials was substantially completed, the system was filtered, washed with diethyl ether, and dried under reduced pressure to give compound **Id-1** in the form of a pale yellow solid (1.8 g, yield: 60.2%), which was directly used in the next step without purification.

MS(m/z): 250.1 [M+H]+.

**Step 2: Synthesis of compound Ie-1**

In a 100 mL eggplant-shaped reaction flask, intermediate **Id-1** (1.8 g, 7.2 mmol) was dissolved in 20 mL of anhydrous xylene. A reflux condenser and a water separator were erected, and the system was allowed to react at 170 °C for 24 h. After the reaction was completed, the system was cooled to room temperature, and a large amount of solid was precipitated. 40 mL of diethyl ether was added, and the system was slurried in an ice bath. The system was filtered under reduced pressure, and the filter residue was rinsed with diethyl ether to give compound Ie-1 in the form of a pale yellow solid (1.0 g, yield: 59.8%), which was directly used in the next step without purification. MS (m/z): 232.1 $[M+H]^+$.

**Step 3: Synthesis of compound If-1**

In a 100 mL eggplant-shaped reaction flask, intermediate Ie-1 (1.0 g, 4.3 mmol) was dissolved in 20 mL of anhydrous THF solution. Then, lithium hydroxide (455 mg, 10.8 mmol) was dissolved in 4 mL of water, and the solution was slowly added dropwise to the reaction system. The reaction system was allowed to react overnight at room temperature. After thin layer chromatography showed that the reaction of the starting materials was substantially completed, THF was removed under reduced pressure, 6 mL of water was added, and then concentrated hydrochloric acid was added dropwise to adjust the pH to 1-2. A white solid was gradually precipitated, followed by slurring and filtration under reduced pressure. The filter residue was washed with water to give compound If-1 in the form of a light white solid (873 mg, yield: 99.4%), which was directly used in the next step without purification.

MS(m/z): 204.1 $[M+H]^+$.

$^1$H NMR (400 MHz, DMSO-$d_6$) $\delta$ 14.35 (s, 1H), 7.49-7.01 (m, 5H), 4.07 (s, 2H).

**Step 4: Preparation of compound Ia-1**

Intermediate A was obtained with reference to the "method for preparing common intermediate A".

Intermediate A (0.5 mmol, 1 eq.), ethynylcyclopropane (1.2 eq.), and triethylamine (2 mL) were dissolved in 5 mL of DMF. After nitrogen purging, the system was stirred for 10 min. Under a nitrogen atmosphere, cuprous iodide (0.25 eq.) and bis(triphenylphosphine)palladium(II) dichloride (0.25 eq.) were added. After nitrogen purging again, the system was warmed up and allowed to react overnight. After thin layer chromatography showed that the reaction of the starting materials was substantially completed, the system was filtered through celite and washed with methanol, and the filtrate was dried and concentrated. The crude product was directly used in the next step without purification.

MS(m/z): 267.1 $[M+H]^+$.

The cuprous iodide and bis(triphenylphosphine)palladium(II) dichloride described above act as metal co-catalysts, and based on the common knowledge of those skilled in the art, generally, the coupling reaction is effected in the presence of a metal catalyst (including, but not limited to, combined action of a metal palladium catalyst and a metal copper catalyst (e.g., cuprous iodide), such as cuprous iodide and bis(triphenylphosphine)palladium(II) dichloride used in this example), a phosphine ligand (including, but not limited to, a BINAP ligand, a DPPF ligand, a Xantphos ligand, a DPBP ligand, a X-phos ligand, and a cBRIDP ligand), and a base (including, but not limited to, triethylamine, pyridine, diisopropylethylamine, potassium carbonate, cesium carbonate, potassium tert-butoxide, and potassium phosphate, such as triethylamine used in this example) and in a suitable solvent (including, but not limited to, N,N-dimethylformamide, dimethylacetamide, acetonitrile, dimethoxyethane, dimethyl sulfoxide, dioxane, tetrahydrofuran, ethylene glycol dimethyl ether, toluene, and 2 wt% TPGs-750-w/$H_2O$, such as *N,N*-dimethylformamide use in this example) at an elevated temperature or under reflux conditions. The reaction is a classical metal-catalyzed coupling reaction, and those skilled in the art can adaptively modify one or more of the transition metal palladium catalyst, the phosphine ligand, the base, and the solvent of the present disclosure based on the characteristics of the reaction substrates.

Transition metal palladium catalysts include, but are not limited to, bis(triphenylphosphine)palladium(II) dichloride, allylpalladium(II) chloride dimer, [1,1'-bis(diphenylphosphino)ferrocene]palladium(II) dichloride ((dppf)$PdCl_2$), palladium(II) acetate, and palladium($\pi$-cinnamyl) chloride dimer $[(cinnamyl)PdCl]_2$.

**Step 5: Preparation** of target **compound I-1**

In a 50 mL eggplant-shaped flask, the purified intermediate Ia-1 (29 mg, 0.11 mmol), HOBT (16 mg, 0.12 mmol), EDCI (22 mg, 0.12 mmol), and NMM (32 mg, 0.31 mmol) were sequentially dissolved in DCM (5 mL), and the system was purged with nitrogen three times. After stirring at room temperature for 10 min, If-1 (32 mg, 0.16 mmol) dissolved in DCM (4 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL $\times$ 3). The organic phases were combined, washed with a saturated sodium chloride solution, dried over anhydrous sodium sulfate, filtered, and

concentrated under reduced pressure. The residue was purified by preparative liquid chromatography to give the target compound **I-1** in the form of a white solid (10 mg, yield: 20.8%).

MS(m/z): 452.1 $[M+H]^+$.

$^1$H NMR (400 MHz, Chloroform-d) δ 8.44 (d, *J*= 6.0 Hz, 1H), 7.31-7.26 (m, 4H), 7.26-7.24 (m, 2H), 7.09 (d, *J*= 7.2 Hz, 2H), 4.56 (t, *J* = 8.4 Hz, 1H), 4.49-4.43 (m, 1H), 4.19 (s, 2H), 3.88 (q, *J= 10.2* Hz, 1H), 3.26 (d, *J*= 14.4 Hz, 1H), 3.12-3.06 (m, 1H), 3.04-2.94 (m, 2H), 2.42 (d, J= 13.8 Hz, 1H), 2.03 (d*, J* = 13.8 Hz, 1H), 1.46 - 1.41 (m, 1H), 0.88-0.86 (m, 2H), 0.80-0.79 (m, 2H).

Example 2: Preparation of Target **Compound** 5-benzyl-N-(4-oxo-9-(pyridin-2-ylethynyl)-1,2,3,4,6,7-hexahydroazepino [3,2,1-hi]indol-3-yl)-4H-1,2,4-triazole-3-carboxamide **(I-2)**

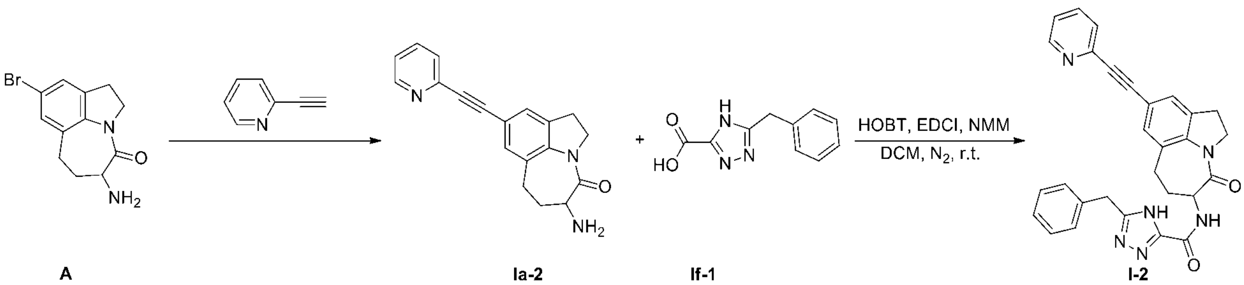


A Ia-2 If-1 I-2

Step 1: **Preparation** of **compound Ia-2**

Intermediate A was obtained with reference to the "method for preparing common intermediate A".

Compound **Ia-2** (84 mg, yield: 36.8%) was obtained with reference to the "method for preparing Ia-1" in Example 1, wherein the intermediate "ethynylcyclopropane" was replaced with "2-ethynylpyridine" while the remaining procedures followed the "method for preparing Ia-1" in Example 1.

MS (m/z): 304.1 $[M+H]^+$.

Step 2: **Preparation** of target **compound I-2**

Intermediate **If-1** was obtained with reference to the "method for preparing compound If-1" in Example 1.

In a 50 mL eggplant-shaped flask, intermediate Ia-2 (84 mg, 0.28 mmol), HOBT (42 mg, 0.31 mmol), EDCI (59 mg, 0.31 mmol), and NMM (85 mg, 0.83 mmol) were dissolved in DCM (5 mL), and the system was thoroughly purged with nitrogen. After stirring at room temperature for 10 min, If-1 (85 mg, 0.42 mmol) dissolved in DCM (5 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-2** in the form of a white solid (10 mg, yield: 7.35%).

MS(m/z): 489.1 $[M+H]^+$.

$^1$H NMR (400 MHz, Chloroform-d) δ 8.57 (d, *J* = 4.8 Hz, 1H), 8.37 (d, *J* = 6.0 Hz, 1H), 7.71 (t, *J* = 7.8 Hz, 1H), 7.49 (d, *J= 7.8* Hz, 1H), 7.26- 7.18 (m, 9H), 4.54 (dd, *J* = 10.2, 6.0 Hz, 1H), 4.46-4.40 (m, 1H), 4.13 (s, 2H), 3.86 *(q, J= 10.2* Hz, 1H), 3.29-3.23 (m, 1H), 3.11-2.94 (m, 3H), 2.39 (dt, *J* = 13.8, 4.8 Hz, 1H), 2.04-1.98 (m, 1H).

Example 3: Preparation of Target Compound 5-benzyl-N-(4-oxo-9-(pyridin-3-ylethynyl)-1,2,3,4,6,7-hexahydroazepino [3,2,1-hi]indol-3-yl)-4H-1,2,4-triazole-3-carboxamide (I-3)

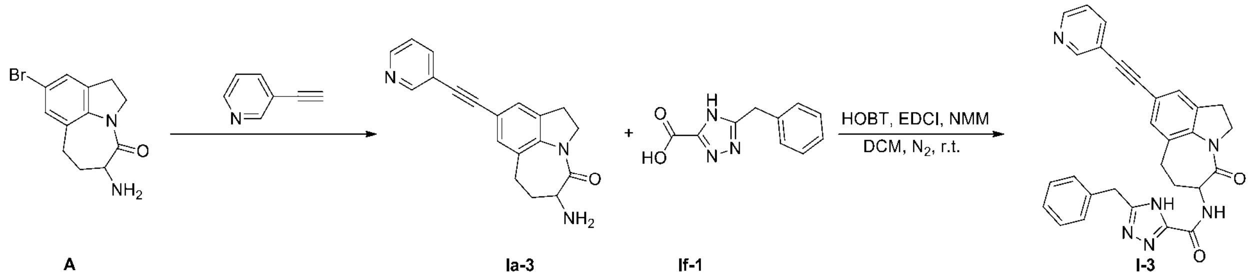


A Ia-3 If-1 I-3

Step **1: Preparation of compound Ia-3**

Intermediate A was obtained with reference to the "method for preparing common intermediate A".

Compound **Ia-3** (52 mg, yield: 30.7%) was obtained with reference to the "method for preparing **Ia-1"** in Example 1, wherein the intermediate "ethynylcyclopropane" was replaced with "3-ethynylpyridine" while the remaining procedures followed the "method for preparing **Ia-1"** in Example 1.

MS (m/z): 304.1 $[M+H]^+$.

Step 2: **Preparation of target compound I-3**

Intermediate **If-1** was obtained with reference to the "method for preparing compound **If-1"** in Example 1.

In a 50 mL eggplant-shaped flask, intermediate **Ia-3** (52 mg, 0.17 mmol), HOBT (26 mg, 0.19 mmol), EDCI (36 mg, 0.19 mmol), and NMM (53 mg, 0.52 mmol) were dissolved in anhydrous DCM (3 mL), and the system was thoroughly purged with nitrogen. After stirring at room temperature for 10 min, **If-1** (53 mg, 0.26 mmol) dissolved in DCM (3 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-3** in the form of a white solid (5 mg, yield: 5.88%). MS(m/z): 489.1 $[M+H]^+$.

$^1H$ NMR (400 MHz, Chloroform-d) δ 8.75 (s, 1H), 8.55 (dd, *J* = 6.0, 1.6 Hz, 1H), 8.45 (d, *J*= 6.0 Hz, 1H), 7.80 (dt, *J*= 8.0, 2.0 Hz, 1H), 7.33-7.31 (m, 4H), 7.30-7.27 (m, 5H), 4.66-4.59 (m, 1H), 4.51 (ddd, *J*= 13.2, 10.4, 3.2 Hz, 1H), 4.20 (s, 2H), 3.94 (dt, *J*= 12.0, 10.0 Hz, 1H), 3.36 (ddd, *J* = 18.0, 12.4, 5.6 Hz, 1H), 3.23-3.09 (m, 2H), 3.09-3.02 (m, 1H), 2.48 *(d, J*= 14.0 Hz, 1H), 2.15-2.03 (m, 1H).

Example **4:** Preparation of Target **Compound** 5-benzyl-N-(4-oxo-9-(pyridin-4-ylethynyl)-1,2,3,4,6,7-hexahydroazepino [3,2,1-hi]indol-3-yl)-4H-1,2,4-triazole-3-carboxamide **(I-4)**

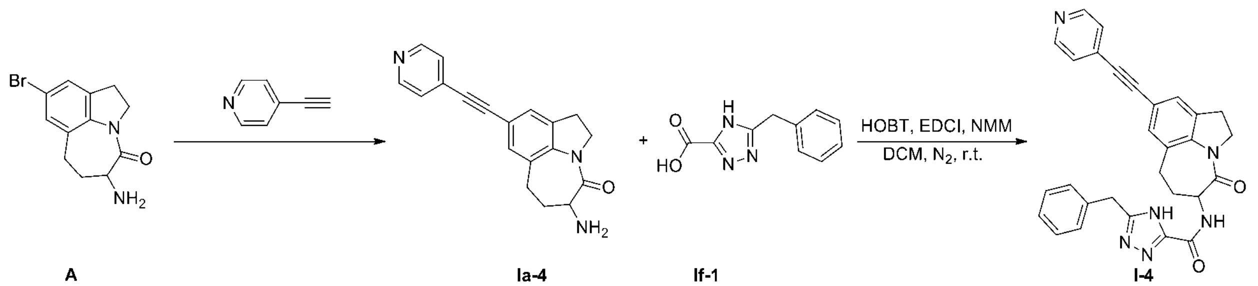


A Ia-4 If-1 I-4

**Step** 1: **Preparation of compound Ia-4**

Intermediate A was obtained with reference to the "method for preparing common intermediate A".

Compound Ia-4 (89 mg, yield: 12.3%) was obtained with reference to the "method for preparing Ia-1" in Example 1, wherein the intermediate "ethynylcyclopropane" was replaced with "4-ethynylpyridine" while the remaining procedures followed the "method for preparing Ia-1" in Example 1.

MS(m/z): 304.1 $[M+H]^+$.

Step 2: **Preparation** of target **compound I-4**

Intermediate **If-1** was obtained with reference to the "method for preparing compound If-1" in Example 1.

In a 50 mL eggplant-shaped flask, intermediate Ia-4 (89 mg, 0.3 mmol), HOBT (45 mg, 0.33 mmol), EDCI (63 mg, 0.33 mmol), and NMM (91 mg, 0.9 mmol) were dissolved in anhydrous DCM (3 mL), and the system was thoroughly purged with nitrogen. After stirring at room temperature for 10 min, If-1 (40 mg, 0.20 mmol) dissolved in DCM (3 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give I-4 in the form of a white solid (20 mg, yield: 13.7%).

MS(m/z): 489.1 $[M+H]^+$.

$^1$H NMR (400 MHz, Chloroform-d) δ 8.65-8.53 (m, 2H), 8.46 (d, *J* = 6.0 Hz, 1H), 7.38 (d, *J* = 5.2Hz, 2H), 7.34-7.27 (m, 6H), 7.24-7.23 (m, 1H), 4.62 (dd, *J* = 10.4, 6.0 Hz, 1H), 4.51 (ddd, *J*= 13.2, 10.4, 3.2 Hz, 1H), 4.21 (s, 2H), 3.97-3.90 (m, 1H), 3.34 (dt, *J* = 12.4, 6.0 Hz, 1H), 3.23-2.98 (m, 4H), 2.51-2.44 (m, 1H), 2.14-2.05 (m, 1H).

Example 5: Preparation of Target **Compound** 5-benzyl-*N*-(9-((1-methyl-1*H*-pyrazol-4-yl)ethynyl)-4-oxo-1,2,3,4,6,7-hexahydroazepino[3,2,1-hi]indol-3-yl)-4H-1,2,4-triazole-3-carboxamide (I-5)

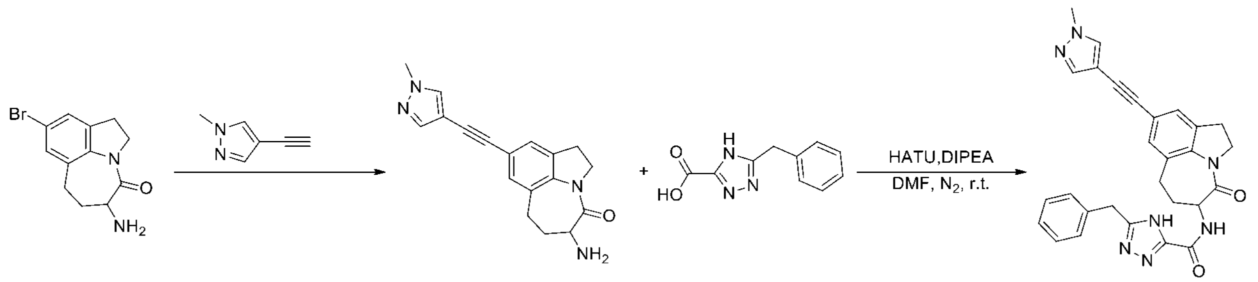


A Ia-5 If-1 I-5

**Step** 1: **Preparation of compound Ia-5**

Intermediate A was obtained with reference to the "method for preparing common intermediate A".

Compound Ia-5 (75 mg, yield: 29.4%) was obtained with reference to the "method for preparing Ia-1" in Example 1, wherein the intermediate "ethynylcyclopropane" was replaced with "*N*-methyl-4-alkynylpyrazole" while the remaining procedures followed the "method for preparing **Ia-1"** in Example 1.

MS (m/z): 307.1 $[M+H]^+$.

**Step 2: Preparation of target compound I-5**

Intermediate **If-1** was obtained with reference to the "method for preparing compound **If-1"** in Example 1.

In a 50 mL eggplant-shaped flask, intermediate **Ia-5** (75 mg, 0.25 mmol), **If-1** (102 mg, 0.50 mmol), HATU (190 mg, 0.5 mmol), and DIPEA (161 mg, 1.25 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-5** in the form of a white solid (15 mg, yield: 12.3%).

MS(m/z): 492.2 $[M+H]^+$.

$^1$H NMR (400 MHz, Chloroform-d) δ 8.46 (d, *J*= 6.0 Hz, 1H), 7.63 (s, 1H), 7.54 (s, 1H), 7.35-7.26 (m, 5H), 7.25-7.21 (m, 1H), 7.19 (d, *J* = 2.8 Hz, 2H), 4.64-4.56 (m, 1H), 4.48 (dt, *J*= 11.2, 10.0, 3.2 Hz, 1H), 4.20 (s, 2H), 3.95-3.87 (m, 4H), 3.37-3.25 (m, 1H), 3.19-2.93 (m, 3H), 2.44 (d, *J*= 13.6 Hz, 1H), 2.12-2.00 (m, 1H).

**Example 6:** Preparation of **Target Compound** 5-benzyl-N-(4-oxo-9-((tetrahydro-2H-pyran-4-yl)ethynyl)-1,2,3,4,6,7-hexahydroazepino[3,2,1-hi]indol-3-yl)-4H-1,2,4-triazole-3-carboxamide **(I-6)**

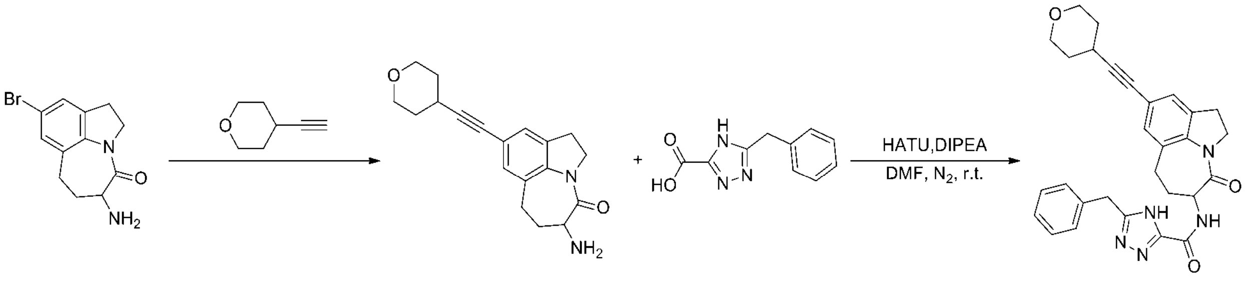


A Ia-6 If-1 I-6

**Step 1: Preparation of compound Ia-6**

Intermediate **A** was obtained with reference to the "method for preparing common intermediate A".

Compound **Ia-6** (118 mg, yield: 100%) was obtained with reference to the "method for preparing **Ia-1"** in Example 1, wherein the intermediate "ethynylcyclopropane" was replaced with "4-ethynylpyran" while the remaining procedures followed the "method for preparing **Ia-1"** in Example 1.

MS(m/z): 311.1 $[M+H]^+$.

**Step 2: Preparation of target compound I-6**

Intermediate **If-1** was obtained with reference to the "method for preparing compound **If-1"** in Example 1.

In a 50 mL eggplant-shaped flask, intermediate **Ia-6** (118 mg, 0.38 mmol), **If-1** (154 mg, 0.76 mmol), HATU (288 mg, 0.76 mmol), and DIPEA (147 mg, 1.14 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-6** in the form of a white solid (16 mg, yield: 4.0%).

MS(m/z): 496.2 $[M+H]^+$.

$^1H$ NMR (400 MHz, DMSO-$d_6$) δ 8.62 (d, *J*= 6.8 Hz, 1H), 7.38-7.20 (m, 5H), 7.13 (d, *J*= 13.2 Hz, 2H), 4.51 (ddd, *J*= 9.6, 6.8, 2.0 Hz, 1H), 4.23 (ddd, *J*= 11.6, 10.4, 4.4 Hz, 1H), 4.12 (s, 2H), 3.87-3.75 (m, 3H), 3.44 (ddd, *J*= 11.6, 9.2, 2.8 Hz, 3H), 3.10-2.95 (m, 4H), 2.87 (tt, *J* = 8.8, 4.0 Hz, 1H), 2.19 (ddd, *J* = 11.6, 6.0, 3.2 Hz, 1H), 2.05 (ddt, *J*= 14.0, 10.0, 5.6 Hz, 1H), 1.82 (dq, *J*= 12.4, 4.0 Hz, 2H), 1.62-1.53 (m, 2H).

Example 7: Preparation of Target Compound 5-benzyl-N-(9-(3-hydroxy-3-methylbut-1-yn-1-yl)-4-oxo-1,2,3,4,6,7-hexahydroazepino[3,2,1-hi]indol-3-yl)-4H-1,2,4-triazole-3-carboxamide (I-7)

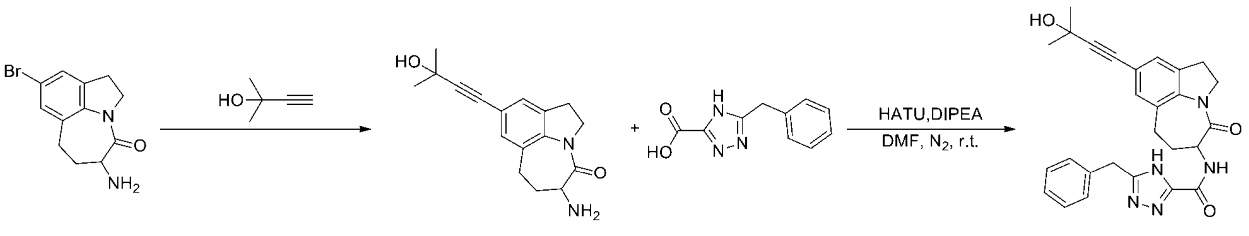


A Ia-7 If-1 I-7

**Step** 1: Preparation of compound Ia-7

Intermediate A was obtained with reference to the "method for preparing common intermediate A".

Compound Ia-7 (98 mg, yield: 34.5%) was obtained with reference to the "method for preparing Ia-1" in Example 1, wherein the intermediate "ethynylcyclopropane" was replaced with "2-methyl-3-butyn-2-ol" while the remaining procedures followed the "method for preparing Ia-1" in Example 1.

MS (m/z): 311.1 $[M+H]^+$.

Step 2: **Preparation** of target **compound I-7**

Intermediate **If-1** was obtained with reference to the "method for preparing compound If-1" in Example 1.

In a 50 mL eggplant-shaped flask, intermediate Ia-7 (48 mg, 0.17 mmol), If-1 (52 mg, 0.25 mmol), HATU (129 mg, 0.34 mmol), and DIPEA (66 mg, 0.51 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL $\times$ 3). The organic phases were combined, washed with a saturated sodium chloride solution, dried over anhydrous sodium sulfate, filtered, and concentrated under reduced pressure. The residue was purified by preparative liquid chromatography to give the target compound **I-7** in the form of a white solid (5 mg, yield: 6.3%).

MS(m/z): 470.3 $[M+H]^+$.

$^1$H NMR (400 MHz, DMSO-$d_6$) $\delta$ 14.42 (s, 1H), 8.63 (s, 1H), 7.35-7.23 (m, 5H), 7.12 (d, *J*= 13.2 Hz, 2H), 5.42 (s, 1H), 4.51 *(t, J*= 8.4 Hz, 1H), 4.30-4.19 (m, 1H), 4.12 (s, 2H), 3.85 (q, *J*= 10.4 Hz, 1H), 3.10-2.98 (m, 4H), 2.19 (d, *J= 13.6* Hz, 1H), 2.06 (d, *J* = 12.4 Hz, 1H), 1.45 (s, 6H).

Example 8: Preparation of Target **Compound** 5-(4-fluorobenzyl)-N-(4-oxo-9-(pyridin-4-ylethynyl)-1,2,3,4,6,7-hexahydroazepino[3,2,1-hi]indol-3-yl)-4H-1,2,4-triazole-3-carboxamide (I-8)

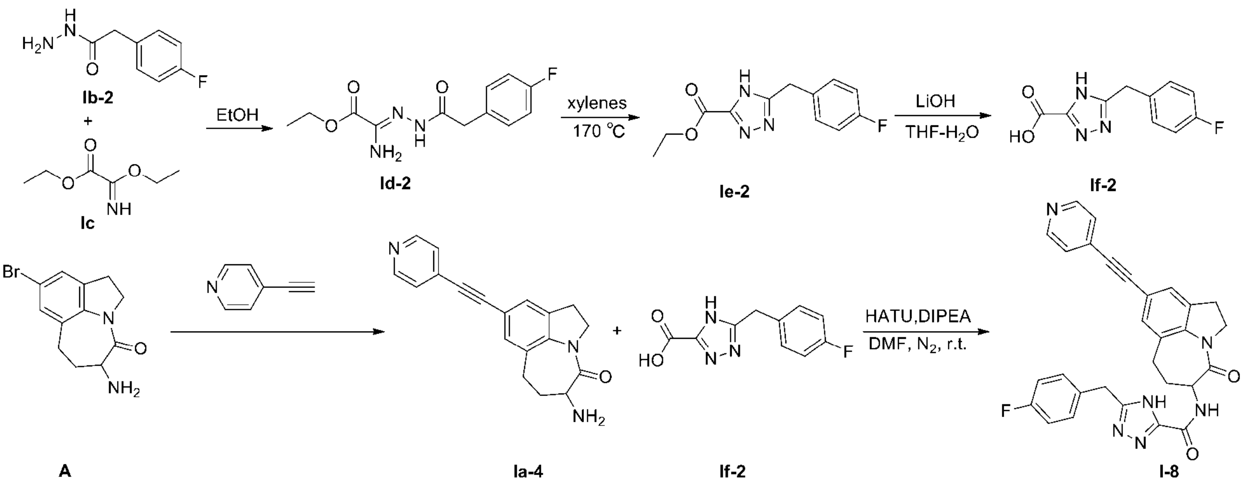

Ib-2 + Ic, Id-2, Ie-2, If-2, A, Ia-4, If-2, I-8

**Step** 1: **Preparation of compound If-2**

Compound **If-2** (475 mg, three-step total yield: 43.1%) was obtained with reference to the "method for preparing compound If-1" in Example 1, wherein only the starting material "2-phenylacetylhydrazine" was replaced with "2-(4-fluorophenyl)-acetylhydrazine" while the remaining procedures were the same.

MS(m/z): 222.1 $[M+H]^+$.

$^1$H NMR (400 MHz, DMSO-$d_6$) $\delta$ 13.76 (s, 1H), 8.32 (s, 1H), 7.56-6.78 (m, 4H), 4.11-4.03(m, 2H).

Step 2: **Preparation of target compound I-8**

Intermediate **Ia-4** was obtained with reference to the "method for preparing compound Ia-4" in Example 4.

In a 50 mL eggplant-shaped flask, intermediate Ia-4 (95 mg, 0.31 mmol), If-2 (139 mg, 0.63 mmol), HATU (238

mg, 0.63 mmol), and DIPEA (121 mg, 0.94 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-8** in the form of a white solid (21 mg, yield: 13.3%).

MS(m/z): 507.2 $[M+H]^+$.

$^1$H NMR (600 MHz, DMSO-$d_6$) δ 14.41 (s, 1H), 8.85-8.44 (m, 3H), 7.56-7.43 (m, 2H), 7.42-7.24 (m, 4H), 7.21-7.09 (m, 2H), 4.58-4.55 (m, 1H), 4.29-4.21 (m, 1H), 4.12 (s, 2H), 3.92-3.87 (m, 1H), 3.14-3.02 (m, 4H), 2.23-2.19 (m, 1H), 2.13-2.06 (m, 1H).

**Example** 9: Preparation of **Target Compound** 5-(4-chlorobenzyl)-N-(4-oxo-9-(pyridin-4-ylethynyl)-1,2,3,4,6,7-hexahydroazepino[3,2,1-hi]indol-3-yl)-4H-1,2,4-triazole-3-carboxamide (I-9)

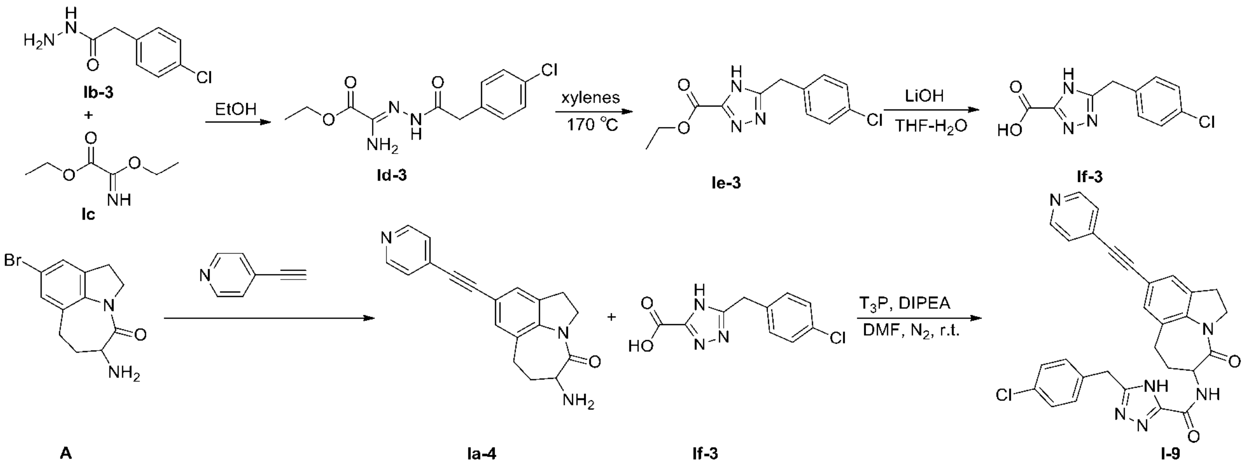


Ib-3 + Ic → Id-3 → Ie-3 → If-3

A → Ia-4 + If-3 → I-9

**Step** 1: **Preparation of compound If-3**

Compound **If-3** (1.07 g, three-step total yield: 90.3%) was obtained with reference to the "method for preparing compound If-1" in Example 1, wherein only the starting material "2-phenylacetylhydrazine" was replaced with "2-(4-chlorophenyl)-acetylhydrazine" while the remaining procedures were the same.

MS (m/z): 238.0 $[M+H]^+$.

$^1$H NMR (400 MHz, DMSO-$d_6$) δ 7.43-7.24 (m, 4H), 4.05 (s, 2H), 3.47 (s, 2H).

Step 2: **Preparation of target compound I-9**

Intermediate **Ia-4** was obtained with reference to the "method for preparing compound Ia-4" in Example 4.

In a 50 mL eggplant-shaped flask, intermediate Ia-4 (91 mg, 0.3 mmol), If-3 (85 mg, 0.36 mmol), $T_3P$ (50% wt in DMF, 286 mg, 0.45 mmol), and DIPEA (193 mg, 1.5 mmol) were dissolved in anhydrous DMF (5 mL), and the system was thoroughly purged with nitrogen and allowed to react at room temperature for 3 h. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-9** in the form of a white solid (20 mg, yield: 12.8%).

MS(m/z): 523.2 $[M+H]^+$.

$^1$H NMR (400 MHz, DMSO-$d_6$) δ 14.41 (s, 1H), 8.80-8.53 (m, 3H), 7.61-7.46 (m, 2H), 7.45-7.10 (m, 6H), 4.59-4.54 (m, 1H), 4.29-4.22 (m, 1H), 4.13 (s, 2H), 3.93-3.86 (m, 1H), 3.18-2.98 (m, 4H), 2.25-2.16 (m, 1H), 2.15-2.04 (m, 1H).

**Example 10:** Preparation of **Target** Compound5-(2-chlorobenzyl)-N-(4-oxo-9-(pyridin-4-ylethynyl)-1,2,3,4,6,7-hexahydroazepino[3,2,1-hi]indol-3-yl)-4H-1,2,4-triazole-3-carboxamide (I-10)

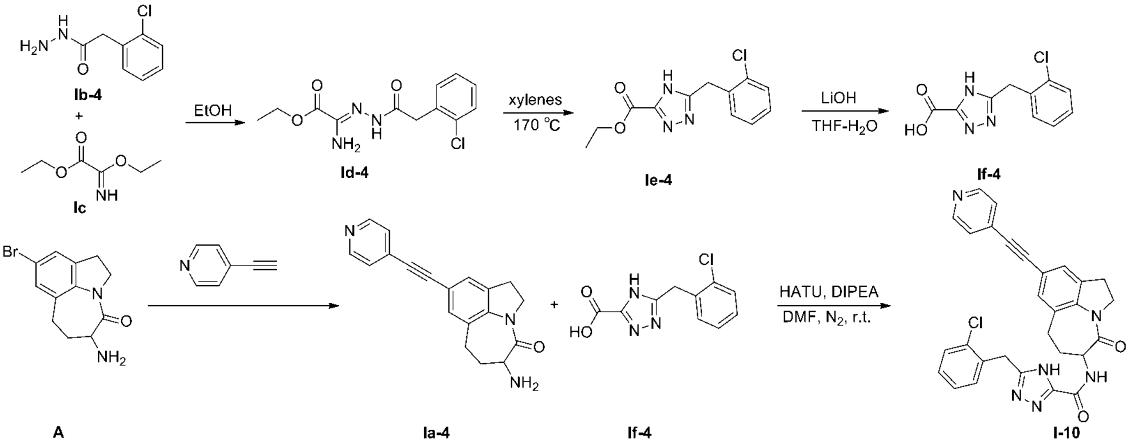


Ib-4 + Ic → Id-4 → Ie-4 → If-4

A → Ia-4; Ia-4 + If-4 → I-10

Step **1: Preparation of compound If-4**

Compound **If-4** (335 mg, three-step total yield: 56.6%) was obtained with reference to the "method for preparing compound **If-1"** in Example 1, wherein only the starting material "2-phenylacetylhydrazine" was replaced with "2-(2-chlorophenyl)-acetylhydrazine" while the remaining procedures were the same.

$^1$H NMR (400 MHz, DMSO-$d_6$) δ 14.21 (s, 1H), 8.21 (s, 1H), 7.50-7.25 (m, 4H), 4.19 (s, 2H).

MS (m/z): 238.0 [M+H]$^+$.

Step 2: **Preparation** of target **compound I-10**

Intermediate **Ia-4** was obtained with reference to the "method for preparing compound **Ia-4"** in Example 4.

In a 50 mL eggplant-shaped flask, intermediate **Ia-4** (50 mg, 0.17 mmol), **If-4** (85 mg, 0.25 mmol), HATU (126 mg, 0.33 mmol), and DIPEA (106 mg, 0.83 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-10** in the form of a white solid (7 mg, yield: 8.1%).

MS(m/z): 523.3 [M+H]$^+$.

$^1$H NMR (400 MHz, DMSO-$d_6$) δ 14.51 (br, 1H), 8.62 (d, $J$ = 5.2 Hz, 3H), 7.63-7.18 (m, 8H), 4.56 (t, $J$ = 8.4 Hz, 1H), 4.24 (s, 3H), 3.90 (q, $J$= 10.8 Hz, 1H), 3.18-2.95 (m, 4H), 2.21 (d, $J$ = 13.6 Hz, 1H), 2.09 (d, $J$= 11.6 Hz, 1H).

**Example 11:** Preparation of **Target Compound** 5-(2-fluorobenzyl)-N-(4-oxo-9-(pyridin-4-ylethynyl)-1,2,3,4,6,7-hexahydroazepino[3,2,1-hi]indol-3-yl)-4H-1,2,4-triazole-3-carboxamide **(I-11)**

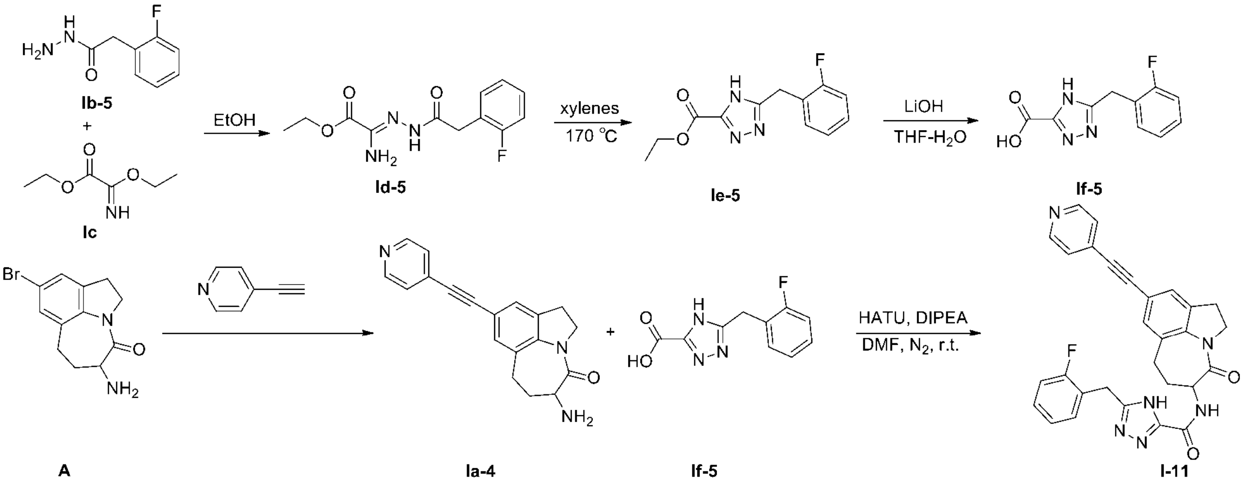


Ib-5 + Ic → Id-5 → Ie-5 → If-5

A → Ia-4 + If-5 → I-11

**Step** 1: **Preparation of compound If-5**

Compound **If-5** (230 mg, three-step total yield: 40.5%) was obtained with reference to the "method for preparing compound If-1" in Example 1, wherein only the starting material "2-phenylacetylhydrazine" was replaced with "2-(2-fluorophenyl)-acetylhydrazine" while the remaining procedures were the same.

MS (m/z): 222.1 $[M+H]^+$.

$^1$H NMR (600 MHz, DMSO-$d_6$) δ 13.45 (s, 1H), 7.38-7.12 (m, 5H), 4.10 (s, 2H).

Step 2: **Preparation** of target **compound I-11**

Intermediate **Ia-4** was obtained with reference to the "method for preparing compound Ia-4" in Example 4.

In a 50 mL eggplant-shaped flask, intermediate Ia-4 (50 mg, 0.17 mmol), If-5 (55 mg, 0.25 mmol), HATU (126 mg, 0.33 mmol), and DIPEA (64 mg, 0.50 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-11** in the form of a white solid (6 mg, yield: 7.2%).

MS(m/z): 507.3 $[M+H]^+$.

$^1$H NMR (400 MHz, DMSO-$d_6$) δ 14.30 (br, 1H), 8.62 (d, *J* = 5.6 Hz, 3H), 7.68-6.97 (m, 8H), 4.57 (t, *J* = 8.8 Hz, 1H), 4.28-4.15 (m, 3H), 3.93-3.86 (m, 1H), 3.19-2.99 (m, 4H), 2.26-2.16 (m, 1H), 2.15-2.03 (m, 1H).

**Example 12:** Preparation of **Target Compound** 5-(2,6-difluorobenzyl)-N-(4-oxo-9-(pyridin-4-ylethynyl)-1,2,3,4,6,7-hexahydroazepino[3,2,1-hi]indol-3-yl)-4H-1,2,4-triazole-3-carboxamide (I-12)

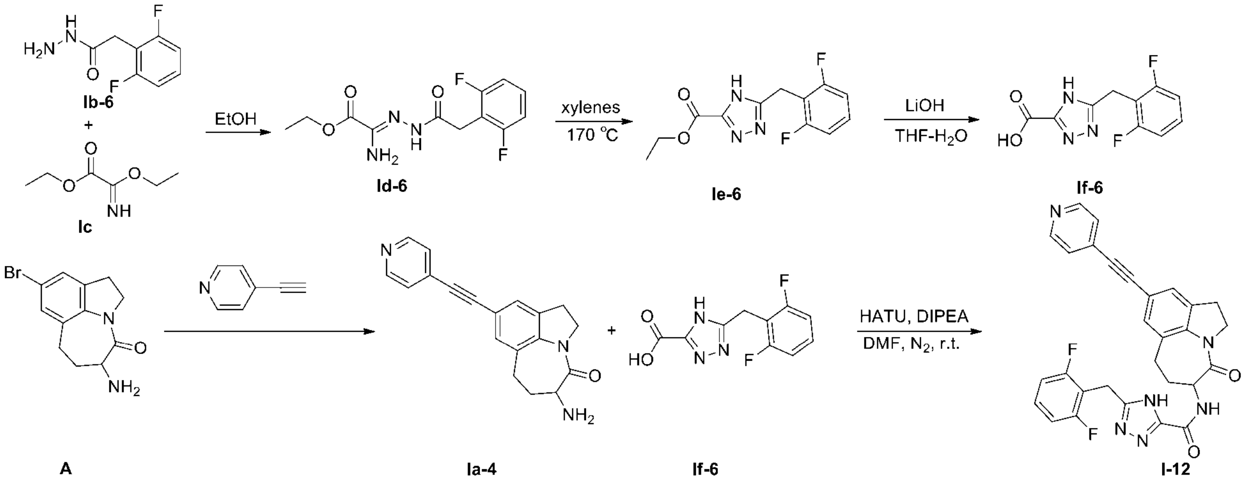


Ib-6 + Ic → Id-6 → Ie-6 → If-6

A → Ia-4 + If-6 → I-12

**Step** 1: **Preparation of compound If-6**

Compound **If-6** (270 mg, three-step total yield: 16.8%) was obtained with reference to the "method for preparing compound If-1" in Example 1, wherein only the starting material "2-phenylacetylhydrazine" was replaced with "2-(2,6-difluorophenyl)-acetylhydrazine" while the remaining procedures were the same.

MS (m/z): 240.0 $[M+H]^+$.

$^1$H NMR (600 MHz, DMSO-$d_6$) δ 13.79 (s, 1H), 7.87-6.94 (m, 4H), 4.04 (s, 2H).

Step 2: **Preparation** of target **compound** I-12

Intermediate **Ia-4** was obtained with reference to the "method for preparing compound Ia-4" in Example 4.

In a 50 mL eggplant-shaped flask, intermediate Ia-4 (120 mg, 0.40 mmol), If-6 (142 mg, 0.60 mmol), HATU (304 mg, 0.80 mmol), and DIPEA (155 mg, 1.20 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-12** in the form of a white solid (32 mg, yield: 15.2%).

MS(m/z): 525.2 $[M+H]^+$.

$^1$H NMR (400 MHz, DMSO-$d_6$) δ 14.31 (s, 1H), 8.67-8.61 (m, 3H), 7.49 (d, *J* = 5.2 Hz, 2H), 7.45-7.35 (m, 3H), 7.13 (t, *J* = 7.6 Hz, 2H), 4.61-4.52 (m, 1H), 4.28-4.21 (m, 1H), 4.14 (s, 2H), 3.89 (q, *J* = 10.2 Hz, 1H), 3.13-3.03 (m, 4H), 2.21-2.17 (m, 1H), 2.13-2.07 (m, 1H).

Example 13: Preparation of **Target Compound** 5-(2,6-dichlorobenzyl)-N-(4-oxo-9-(pyridin-4-ylethynyl)-1,2,3,4,6,7-hexahydroazepino[3,2,1-hi]indol-3-yl)-4H-1,2,4-triazole-3-carboxamide (I-13)

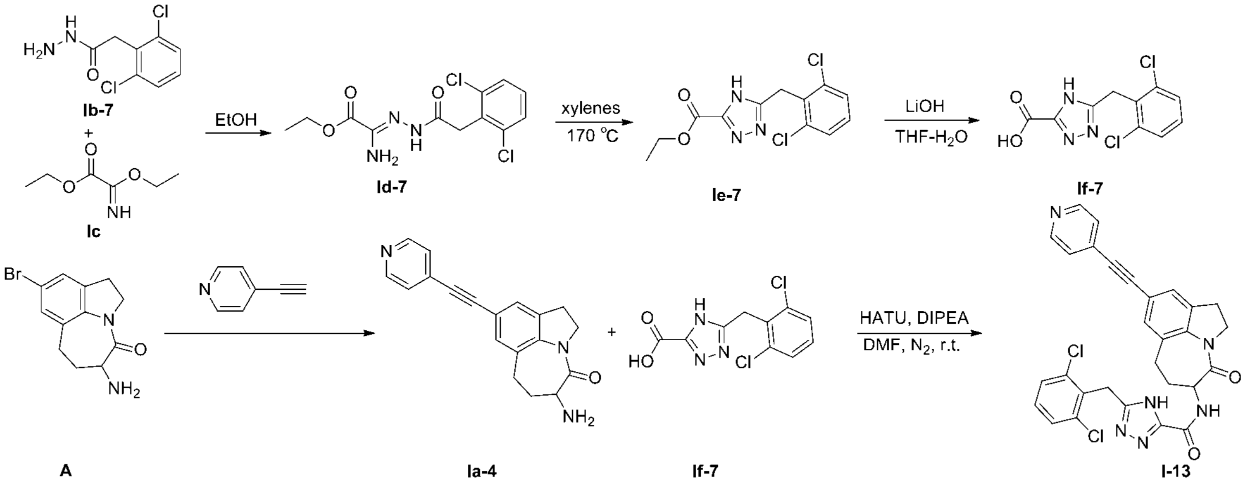


Ib-7 Ic Id-7 Ie-7 If-7 A Ia-4 If-7 I-13

Step 1: **Preparation of compound If-7**

Compound **If-7** (310 mg, three-step total yield: 36.9%) was obtained with reference to the "method for preparing compound **If-1"** in Example 1, wherein only the starting material "2-phenylacetylhydrazine" was replaced with "2-(2,6-dichlorophenyl)-acetylhydrazine" while the remaining procedures were the same.

MS (m/z): 238.0 $[M+H]^+$.

**Step 2: Preparation of target compound I-13**

Intermediate **Ia-4** was obtained with reference to the "method for preparing compound **Ia-4"** in Example 4.

In a 50 mL eggplant-shaped flask, intermediate **Ia-4** (170 mg, 0.56 mmol), **If-7** (182 mg, 0.607 mmol), HATU (426 mg, 1.12 mmol), and DIPEA (218 mg, 1.68 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **I-13** in the form of a white solid (20 mg, yield: 6.4%).

MS(m/z): 557.3 $[M+H]^+$.

$^1$H NMR (400 MHz, DMSO-$d_6$) δ 14.61 (s, 1H), 8.65-8.60 (m, 2H), 7.57-7.47 (m, 4H), 7.38 (t, *J* = 9.2 Hz, 3H), 5.35 (s, 1H), 4.56 (s, 1H), 4.40 (s, 2H), 4.25 (td, *J* = 10.8, 10.4, 4.4 Hz, 1H), 3.90 (q, *J*= 10.4 Hz, 1H), 3.23 (d, *J* = 7.2 Hz, 1H), 2.87 (d, *J*= 7.2 Hz, 1H), 2.20 (d, *J*= 12.8 Hz, 1H), 2.11 (s, 1H), 2.00 (q, *J*= 7.2 Hz, 1H), 1.77 (s, 1H).

Example **14:** Preparation of Target **Compound** 5-benzyl-N-(4-oxo-8-(pyridin-2-ylethynyl)-1,2,3,4,6,7-hexahydroazepino[3,2,1-hi]indol-3-yl)-4H-1,2,4-triazole-3-carboxamide **(II-1)**

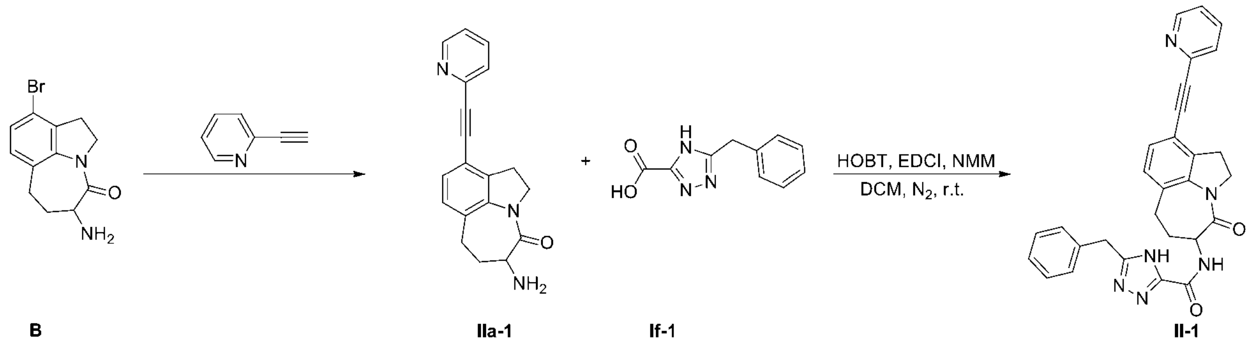


B IIa-1 If-1 II-1

**Step** 1: Preparation of compound IIa-1

Intermediate B was obtained with reference to the "method for preparing common intermediate B".
Compound **IIa-1** (100 mg, yield: 55.2%) was obtained with reference to the "method for preparing Ia-2" in Example 2, wherein the intermediate "A" was replaced with intermediate "B" while the remaining procedures followed the "method for preparing Ia-2" in Example 2.
MS(m/z): 304.1 $[M+H]^+$.

Step 2: **Preparation** of target **compound II-1**

Intermediate **If-1** was obtained with reference to the "method for preparing compound If-1" in Example 1.
In a 50 mL eggplant-shaped flask, intermediate IIa-1 (94 mg, 0.31 mmol), HOBT (46 mg, 0.34 mmol), EDCI (65 mg, 0.34 mmol), and NMM (94 mg, 0.93 mmol) were dissolved in DCM (3 mL), and the system was thoroughly purged with nitrogen. After stirring at room temperature for 10 min, If-1 (91 mg, 0.45 mmol) dissolved in DCM (3 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **II-1** in the form of a white solid (20 mg, yield: 13.2%).
MS(m/z): 489.2 $[M+H]^+$.
$^1$H NMR (600 MHz, Chloroform-*d*) δ 8.55 (d, *J* = 4.8 Hz, 1H), 8.41 (d, *J* = 7.2 Hz, 1H), 7.64 (t, *J* = 7.8 Hz, 1H), 7.46 (d, *J*= *7.8* Hz, 1H), 7.33-7.20 (m, 6H), 7.18-7.12 (m, 2H), 6.98 (d, *J* = 7.8 Hz, 1H), 4.52 (t, *J*= 8.4 Hz, 1H), 4.47-4.39 (m, 1H), 4.14 (s, 2H), 3.86 (q, *J* = 10.8 Hz, 1H), 3.3-3.24 (m, 1H), 3.23-3.14 (m, 2H), 3.02 (d, *J* = 16.8 Hz, 1H), 2.37 (d, *J*= 13.2 Hz, 1H), 2.04-1.97 (m, 1H).

**Example 15:** Preparation of **Target Compound** 5-benzyl-*N*-(4-oxo-8-(pyridin-3-ylethynyl)-1,2,3,4,6,7-hexahydroazepino[3,2,1-*hi*]indol-3-yl)-4*H*-1,2,4-triazole-3-carboxamide **(II-2)**

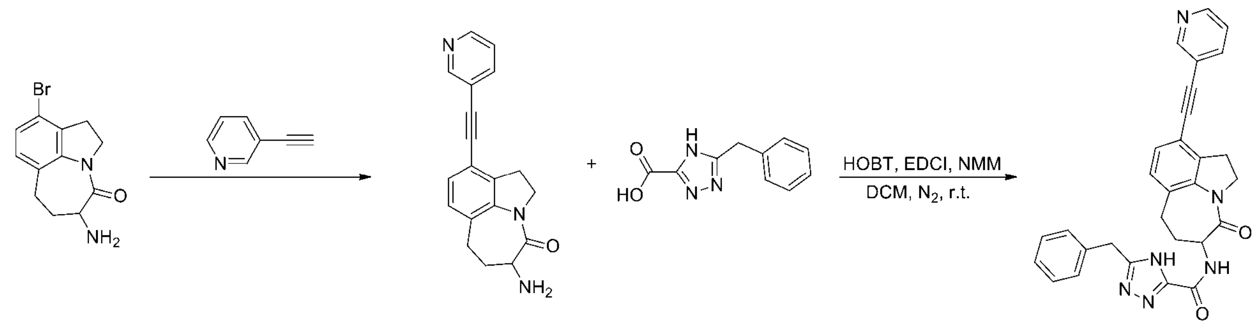


B IIa-2 If-1 II-2

Step 1: **Preparation** of **compound IIa-2**

Intermediate B was obtained with reference to the "method for preparing common intermediate **B".**
Compound **IIa-2** (110 mg, yield: 61.3%) was obtained with reference to the "method for preparing Ia-3" in Example 3, wherein the intermediate **"A"** was replaced with intermediate **"B"** while the remaining procedures followed the "method for preparing **Ia-3"** in Example 3.
MS(m/z): 304.1 $[M+H]^+$.

**Step 2: Preparation of target compound II-2**

Intermediate **If-1** (106 mg) was obtained with reference to the "method for preparing compound If-1" in Example 1.
In a 50 mL eggplant-shaped flask, intermediate **IIa-2** (105 mg, 0.35 mmol), HOBT (52 mg, 0.38 mmol), EDCI (73 mg, 0.38 mmol), and NMM (105 mg, 1.04 mmol) were dissolved in DCM (3 mL), and the system was thoroughly purged with nitrogen. After stirring at room temperature for 10 min, If-1 (106 mg, 0.52 mmol) dissolved in DCM (3 mL) was added under

a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **II-2** in the form of a white solid (21 mg, yield: 12.4%).

MS(m/z): 489.2 $[M+H]^+$.

$^1$H NMR (400 MHz, Chloroform-*d*) δ 8.75 (d, *J* = 2.0 Hz, 1H), 8.56 (dd, *J* = 4.8, 1.6 Hz, 1H), 8.49 (d, *J* = 6.0 Hz, 1H), 7.81 (dt, *J* = 8.0, 2.0 Hz, 1H), 7.35-7.26 (m, 6H), 7.25-7.21 (m, 1H), 7.17 (d, *J* = 8.0 Hz, 1H), 7.06 (d, *J* = 8.0 Hz, 1H), 4.61 (dd, *J*= 10.4, 6.0 Hz, 1H), 4.57-4.51 (m, 1H), 4.22 (s, 2H), 3.96 (q, *J*= 10.4 Hz, 1H), 3.36 (ddd, *J*= 18.0, 12.4, 5.6 Hz, 1H), 3.28-3.19 (m, 2H), 3.11 *(dt, J*= 18.0, 4.0 Hz, 1H), 2.47 *(d, J*= 13.6 Hz, 1H), 2.13-2.06 (m, 1H).

**Example 16:** Preparation of **Target Compound** 5-benzyl-*N*-(8-(cyclopropylethynyl)-4-oxo-1,2,3,4,6,7-hexahydroazepino[3,2,1-*hi*]indol-3-yl)-4*H*-1,2,4-triazole-3-carboxamide **(II-3)**

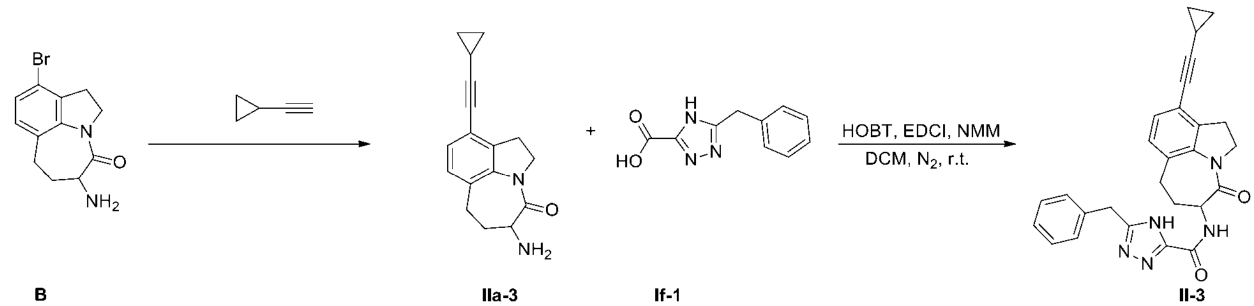


**B** **IIa-3** **If-1** **II-3**

Step **1: Preparation of compound IIa-3**

Intermediate B was obtained with reference to the "method for preparing common intermediate B".

Compound **IIa-3** (110 mg, yield: 68.2%) was obtained with reference to the "method for preparing **Ia-1"** in Example 1, wherein the intermediate "A" was replaced with intermediate "B" while the remaining procedures followed the "method for preparing **Ia-1"** in Example 1.

MS(m/z): 267.1 $[M+H]^+$.

**Step 2: Preparation of target compound II-3**

Intermediate **If-1** was obtained with reference to the "method for preparing compound **If-1"** in Example 1.

In a 50 mL eggplant-shaped flask, intermediate **IIa-3** (103 mg, 0.39 mmol), HOBT (57 mg, 0.43 mmol), EDCI (81 mg, 0.43 mmol), and NMM (117 mg, 1.16 mmol) were dissolved in anhydrous DCM (4 mL), and the system was thoroughly purged with nitrogen. After stirring at room temperature for 10 min, **If-1** (117 mg, 0.58 mmol) dissolved in DCM (4 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **II-3** in the form of a white solid (10 mg, yield: 5.7%).

MS(m/z): 452.2 $[M+H]^+$.

$^1$H NMR (600 MHz, Chloroform-d) δ 8.46 (d, *J*= 6.0 Hz, 1H), 7.31 (d, *J* = 4.2 Hz, 4H), 7.27-7.23 (m, 2H), 7.00 (d, *J*= 7.8 Hz, 1H), 6.95 (d, *J*= 7.8 Hz, 1H), 4.57 (dd, *J*= 10.2, 6.0 Hz, 1H), 4.58-4.55 (m, 1H), 4.19 (s, 2H), 3.88 (q, *J* = 10.8 Hz, 1H), 3.35-3.29 (m, 1H), 3.12-3.04 (m, 3H), 2.47-2.41 (m, 1H), 2.07 (dt, *J* = 12.0, 10.2, 4.2 Hz, 1H),

1.47 (dt, *J* = 8.4, 3.6 Hz, 1H), 0.91-0.89 (m, 2H), 0.80-0.79 (m, 2H).

Example **17:** Preparation of Target **Compound** 5-benzyl-N-(4-oxo-8-(pyridin-4-ylethynyl)-1,2,3,4,6,7-hexahydroazepino[3,2,1-hi]indol-3-yl)-4H-1,2,4-triazole-3-carboxamide **(II-4)**

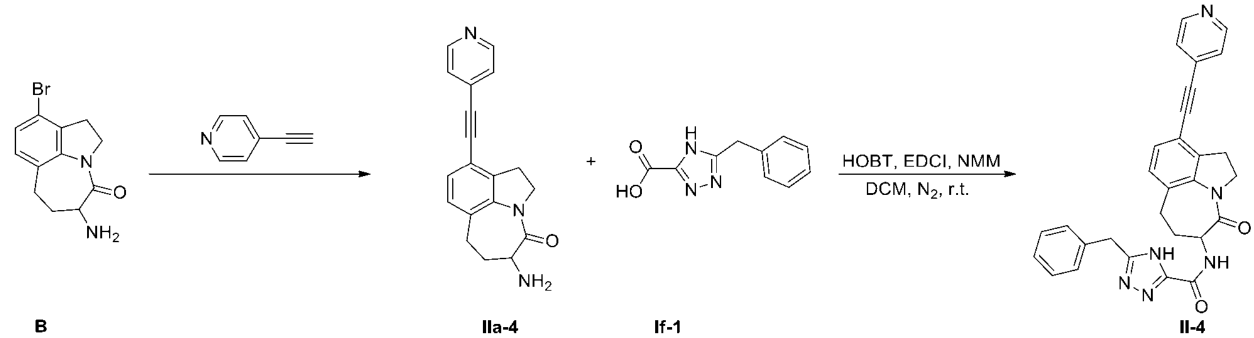


B IIa-4 If-1 II-4

Step **1: Preparation of compound IIa-4**

Intermediate B was obtained with reference to the "method for preparing common intermediate B".

Compound **IIa-4** (45 mg, yield: 27.6%) was obtained with reference to the "method for preparing **Ia-4"** in Example 4, wherein the intermediate "A" was replaced with intermediate "B" while the remaining procedures followed the "method for preparing **Ia-4"** in Example 4.

MS(m/z): 304.1 $[M+H]^+$.

**Step 2: Preparation of target compound II-4**

Intermediate **If-1** was obtained with reference to the "method for preparing intermediate **If-1"** in Example 1.

In a 50 mL eggplant-shaped flask, intermediate **IIa-4** (40 mg, 0.13 mmol), HOBT (20 mg, 0.15 mmol), EDCI (28 mg, 0.15 mmol), and NMM (40 mg, 0.40 mmol) were dissolved in DCM (2 mL), and the system was thoroughly purged with nitrogen. After stirring at room temperature for 10 min, **If-1** (40 mg, 0.20 mmol) dissolved in DCM (2 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL $\times$ 3). The organic phases were combined, washed with a saturated sodium chloride solution, dried over anhydrous sodium sulfate, filtered, and concentrated under reduced pressure. The residue was purified by preparative liquid chromatography to give the target compound **II-4** in the form of a white solid (6 mg, yield: 9.3%).

MS(m/z): 489.2 $[M+H]^+$.

$^1$H NMR (400 MHz, Chloroform-*d*) $\delta$ 11.85 (s, 1H), 8.56-8.38 (m, 2H), 7.80-6.79 (m, 9H), 5.34 (s, 1H), 4.60-4.47 (m, 1H), 4.13- 3.91 (m, 2H), 3.32-2.97 (m, 2H), 2.40-2.02 (m, 2H), 1.80-1.58 (m, 2H), 1.22-1.20 (m, 2H).

Example **18:** Preparation of Target **Compound** 1-(2,6-difluorobenzyl)-N-(9-((1-methyl-1H-pyrazol-4-yl)ethynyl)-4-oxo-1,2,3,4,6,7-hexahydroazepino[3,2,1-hi]indol-3-yl)-1H-1,2,4-triazole-3-carboxamide **(III-1)**

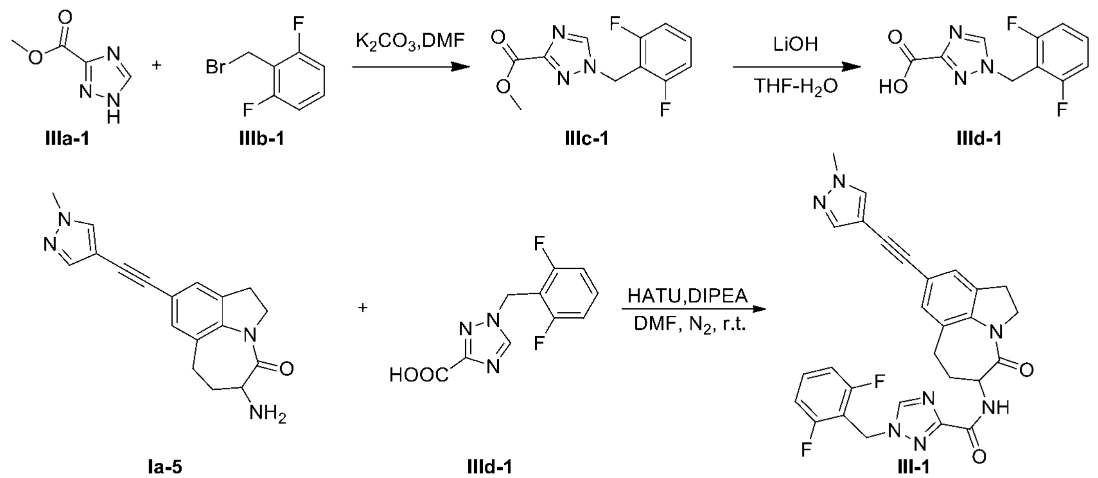


IIIa-1 IIIb-1 IIIc-1 IIId-1

Ia-5 IIId-1 III-1

Step 1: Synthesis of **compound IIIc-1**

In a 100 mL eggplant-shaped reaction flask, the starting material **IIIa-1** (1.9 g, 15 mmol) and potassium carbonate (4.14 g, 30 mmol) were dissolved in 15 mL anhydrous DMF solvent. The system was cooled in an ice bath, and **IIIb-1** (3.73 g, 18 mmol) was slowly added dropwise. After the dropwise addition was completed, the system was purged with nitrogen, and the ice bath was removed. The system was slowly warmed to room temperature and allowed to react overnight. After thin layer chromatography showed that the reaction of the starting materials was substantially completed, water was added to quench the reaction, and the system was extracted with ethyl acetate (50 mL × 3). The organic phases were combined, washed with saturated brine, dried over anhydrous sodium sulfate, and concentrated under reduced pressure to give compound **IIIc-1** in the form of a white solid (1.42 g, yield: 37.4%), which was directly used in the next step without purification.
MS(m/z): 254.1 $[M+H]^+$.

Step 2: Synthesis of **compound IIId-1**

In a 100 mL eggplant-shaped reaction flask, intermediate **IIIc-1** (1.42 g, 5.6 mmol) was dissolved in 32 mL of anhydrous THF solution. Then, lithium hydroxide (270 mg, 11.2 mmol) was dissolved in 8 mL of water, and the solution was slowly added dropwise to the reaction system in an ice bath. After the dropwise addition was completed, the ice bath was removed, and the system was warmed to room temperature and allowed to react overnight. After thin layer chromatography showed that the reaction of the starting materials was substantially completed, THF was removed under reduced pressure, 8 mL of water was added, and then concentrated hydrochloric acid was added dropwise to adjust the pH to 1-2. A white solid was gradually precipitated, followed by slurring and filtration under reduced pressure. The filter residue was washed with water to give **IIId-1** in the form of a light white solid (1.05 g, yield: 78.5%), which was directly used in the next step without purification.
MS (m/z): 240.1 $[M+H]^+$.
$^1$H NMR (600 MHz, DMSO-$d_6$) δ 13.33 (s, 1H), 8.81 (s, 1H), 7.54-7.49 (m, 1H), 7.23-7.13 (m, 2H), 5.56 (s, 2H).

Step 3: **Preparation** of target **compound III-1**

Intermediate **Ia-5** was obtained with reference to the "method for preparing compound Ia-5" in Example 5.
In a 50 mL eggplant-shaped flask, intermediate Ia-5 (110 mg, 0.36 mmol), **IIId-1** (172 mg, 0.72 mmol), HATU (273 mg, 0.72 mmol), and DIPEA (233 mg, 1.80 mmol) were dissolved in anhydrous DMF (8 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-1** in the form of a white solid (80 mg, yield: 42.24%).
MS(m/z): 527.2 $[M+H]^+$.
$^1$H NMR (400 MHz, Chloroform-d) δ 8.39 (d, J= 6.0 Hz, 1H), 8.14 (s, 1H), 7.63 (s, 1H), 7.54 (s, 1H), 7.38 *(tt, J*= 8.4, 6.4 Hz, 1H), 7.19 (d, *J*= 3.6 Hz, 2H), 6.97 (t, *J*= 8.0 Hz, 2H), 5.49 (s, 2H), 4.62 (dd, *J*= 10.2, 6.0 Hz, 1H), 4.50 (ddd, *J*= 12.0, 10.2, 3.2 Hz, 1H), 3.91 (s, 4H), 3.38 (ddd, *J* = 18.0, 12.8, 5.6 Hz, 1H), 3.20-2.96 (m, 3H), 2.54-2.45 (m, 1H), 2.08-1.99 (m, 1H).

Example 19: Preparation of Target Compound 1-(2,6-difluorobenzyl)-N-(4-oxo-9-(pyridin-4-ylethynyl)-1,2,3,4,6,7-hexahydroazepino[3,2,1-hi]indol-3-yl)-1H-1,2,4-triazole-3-carboxamide **(III-2)**

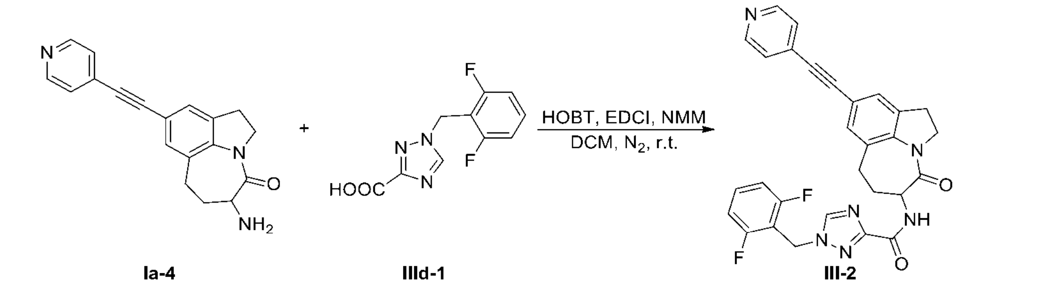


Ia-4 IIId-1 III-2

Intermediate **Ia-4** was obtained with reference to the "method for preparing compound **Ia-4"** in Example 4; intermediate **IIId-1** was obtained with reference to the "preparation of compound **IIId-1"** in Example 18.

In a 50 mL eggplant-shaped flask, intermediate **Ia-4** (210 mg, 0.7 mmol), HOBT (103 mg, 0.76 mmol), EDCI (146 mg, 0.76 mmol), and NMM (210 mg, 2.1 mmol) were dissolved in anhydrous DCM (5 mL), and the system was thoroughly purged with nitrogen. After stirring at room temperature for 10 min, **IIId-1** (248 mg, 1.04 mmol) dissolved in DCM (5 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-2** in the form of a white solid (30 mg, yield: 17.8%).

MS(m/z): 525.2 $[M+H]^+$.

$^1$H NMR (400 MHz, Chloroform-d) δ 8.60 (d, *J* = 5.2 Hz, 2H), 8.38 (d, *J*= 6.0 Hz, 1H), 8.15 (s, 1H), 7.43-7.34 (m, 3H), 7.29 (s, 2H), 6.98 (t, *J* = 8.0 Hz, 2H), 5.50 (s, 2H), 4.68-4.60 (m, 1H), 4.52 (ddd, *J*= 12.0, 10.2, 3.2 Hz, 1H), 3.94 (dt, *J*= 12.0, 10.2 Hz, 1H), 3.41 (ddd, *J*= 18.0, 12.4, 5.6 Hz, 1H), 3.23-2.99 (m, 3H), 2.55-2.49 (m, 1H), 2.11-2.00 (m, 1H).

Example **20:** Preparation of Target **Compound** 1-benzyl-N-(4-oxo-9-(pyridin-4-ylethynyl)-1,2,3,4,6,7-hexahydroazepino[3,2,1-*hi*]indol-3-yl)-1*H*-1,2,4-triazole-3-carboxamide; **(III-3)**

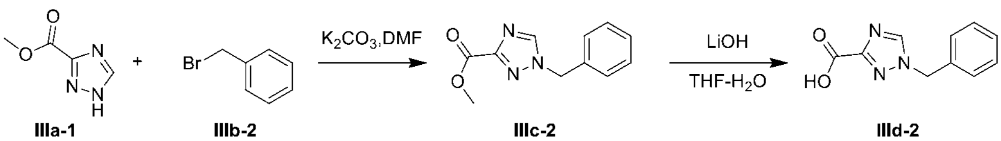


IIIa-1 IIIb-2 IIIc-2 IIId-2

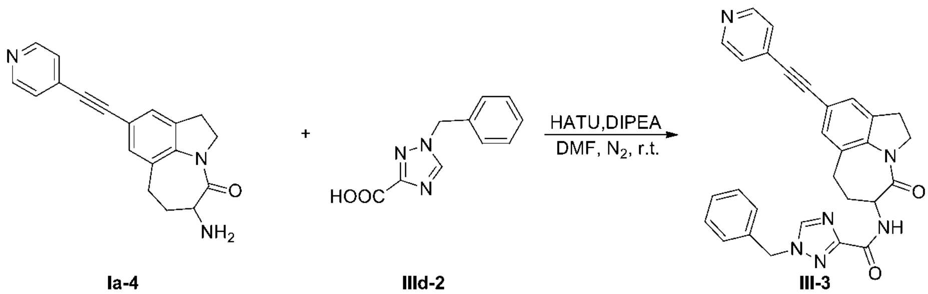


Ia-4 IIId-2 III-3

Step 1: Preparation of compound IIId-2

Compound IIId-2 (811 mg, two-step total yield: 40.0%) was obtained with reference to the "method for preparing compound IIId-1" in Example 18, wherein only the starting material "2-(bromomethyl)-1,3-difluorobenzene" was replaced with "benzyl bromide" while the remaining procedures were the same.

MS(m/z): 204.1 $[M+H]^+$.

$^1$H NMR (600 MHz, DMSO-$d_6$) δ 13.32 (s, 1H), 8.79 (s, 1H), 7.46-7.20 (m, 5H), 5.48 (s, 2H).

Step 2: Preparation of target compound III-3

Intermediate Ia-4 was obtained with reference to the "method for preparing compound Ia-4" in Example 4.

In a 50 mL eggplant-shaped flask, intermediate Ia-4 (95 mg, 0.31 mmol), IIId-2 (127 mg, 0.63 mmol), HATU (238

mg, 0.63 mmol), and DIPEA (121 mg, 0.94 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound III-3 in the form of a white solid (21 mg, yield: 13.7%).

MS(m/z): 489.2 $[M+H]^+$.

$^1H$ NMR (600 MHz, DMSO-$d_6$) δ 8.84 (s, 1H), 8.68-8.56 (m, 3H), 7.56-7.46 (m, 2H), 7.44-7.25 (m, 7H), 5.50 (s, 2H), 4.57-4.53 (m, 1H), 4.30-4.23 (m, 1H), 3.93-3.86 (m, 1H), 3.17-3.00 (m, 4H), 2.23-2.20 (m, 1H), 2.11-2.04 (m, 1H).

Example 21: Preparation of Target Compound 1-(2,6-difluorobenzyl)-N-(9-(3-hydroxy-3-methylbut-1-yn-1-yl)-4-oxo-1,2,3,4,6,7-hexahydroazepino[3,2,1-hi]indol-3-yl)-1H-1,2,4-triazole-3-carboxamide **(III-4)**

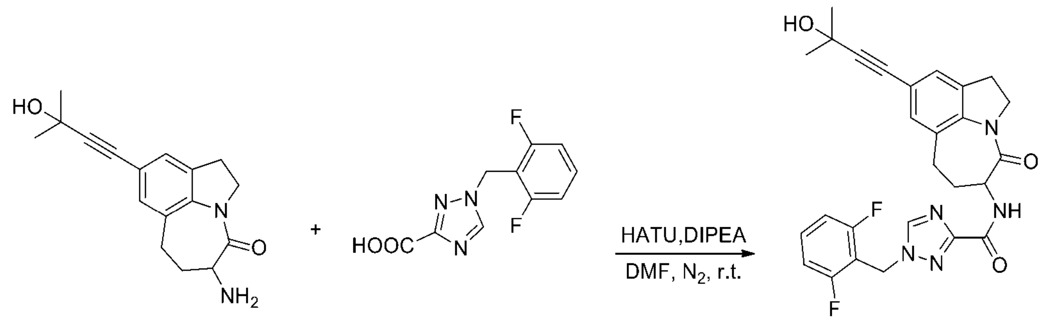


**Ia-7** **IIId-1** **III-4**

Intermediate Ia-7 was obtained with reference to the "method for preparing compound Ia-7" in Example 7; intermediate **IIId-1** was obtained with reference to the "preparation of compound IIId-1" in Example 18.

In a 50 mL eggplant-shaped flask, intermediate Ia-7 (50 mg, 0.18 mmol), **IIId-1** (63 mg, 0.26 mmol), HATU (134 mg, 0.35 mmol), and DIPEA (68 mg, 0.53 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-4** in the form of a white solid (10 mg, yield: 11.4%).

MS(m/z): 506.3 $[M+H]^+$.

$^1H$ NMR (400 MHz, DMSO-$d_6$) δ 8.85 (s, 1H), 8.58 (d, *J* = 6.8 Hz, 1H), 7.52 (p, *J* = 7.6 Hz, 1H), 7.27-7.06 (m, 4H), 5.58 (s, 2H), 5.42 (s, 1H), 4.48 (t, *J*= 8.4 Hz, 1H), 4.24 (td, *J*= 11.2, 4.4 Hz, 1H), 3.85 (q, *J*= 10.4 Hz, 1H), 3.15-2.93 (m, 4H), 2.21-2.16 (m, 1H), 2.08-1.98 (m, 1H), 1.45 (s, 6H).

**Example 22:** Preparation of **Target Compound** 1-(2,6-difluorobenzyl)-N-(9-(3-methoxyprop-1-yn-1-yl)-4-oxo-1,2,3,4,6,7-hexahydroazepino[3,2,1-hi]indol-3-yl)-1H-1,2,4-triazole-3-carboxamide **(III-5)**

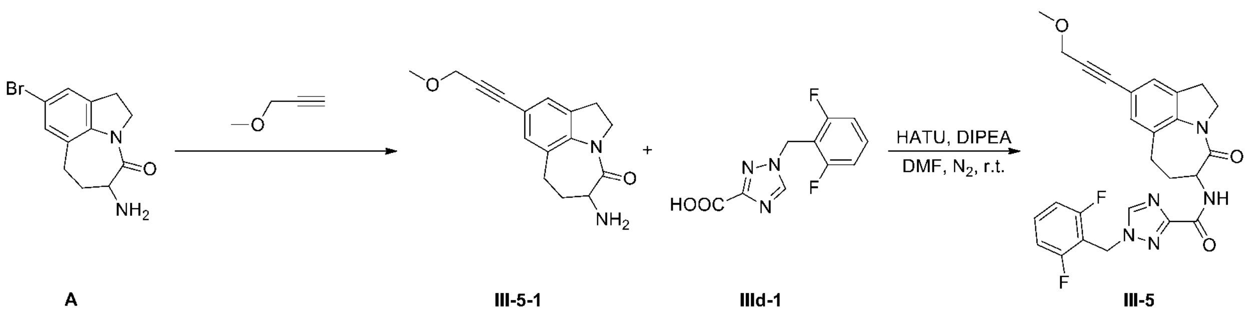


**A** **III-5-1** **IIId-1** **III-5**

Step 1: **Preparation** of **compound III-5-1**

Intermediate A was obtained with reference to the "method for preparing common intermediate A".

Compound **III-5-1** (160 mg, yield: 59.3%) was obtained with reference to the "method for preparing Ia-1" in Example 1, wherein the intermediate "ethynylcyclopropane" was replaced with "3-methoxy-1-propyne" while the remaining procedures followed the "method for preparing Ia-1" in Example 1.

MS(m/z): 271.2 $[M+H]^+$.

Step 2: **Preparation** of target **compound III-5**

Intermediate IIId-1 was obtained with reference to the "preparation of compound **IIId-1"** in Example 18.

In a 50 mL eggplant-shaped flask, intermediate **III-5-1** (51 mg, 0.20 mmol), **IIId-1** (48 mg, 0.20 mmol), HATU (152 mg, 0.40 mmol), and DIPEA (78 mg, 0.60 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-5** in the form of a white solid (5 mg, yield: 5.1%).

MS(m/z): 492.3 $[M+H]^+$.

$^1$H NMR (400 MHz, DMSO-$d_6$) δ 8.86 (s, 1H), 8.59 (d, *J*= 6.8 Hz, 1H), 7.56-7.48 (m, 1H), 7.29-7.12 (m, 4H), 5.58 (s, 2H), 4.50 (ddd, *J= 9.6,* 6.8, 2.0 Hz, 1H), 4.31 (s, 2H), 4.27-4.19 (m, 1H), 3.85 *(q, J*= 10.4 Hz, 1H), 3.32 (s, 3H), 3.13-2.97 (m, 4H), 2.21 (d, *J*= 3.6 Hz, 1H), 2.05 (ddd, *J*= 14.4, 10.8, 4.4 Hz, 1H).

Example 23: Preparation of Target Compound *N*-(9-(cyclopropylethynyl)-4-oxo-1,2,3,4,6,7-hexahydroazepino[3,2,1-hi]indol-3-yl)-1-(2,6-difluorobenzyl)-1H-1,2,4-triazole-3-carboxamide **(III-6)**

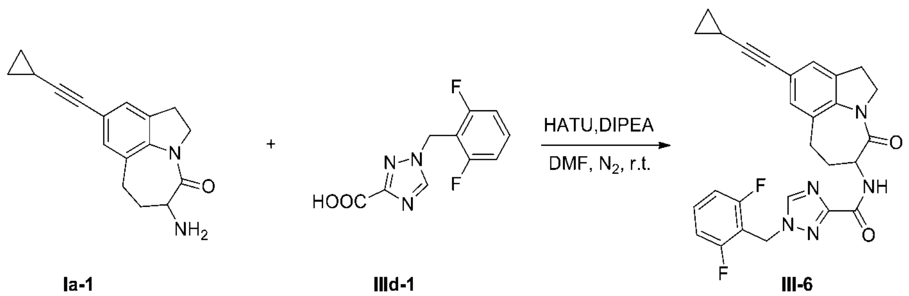


**Ia-1** **IIId-1** **III-6**

Compound **Ia-1** was obtained with reference to the "preparation of compound Ia-1" in Example 1; intermediate **IIId-1** was obtained with reference to the "preparation of compound **IIId-1"** in Example 18.

In a 50 mL eggplant-shaped flask, intermediate Ia-1 (120 mg, 0.45 mmol), **IIId-1** (160 mg, 0.67 mmol), HATU (342 mg, 0.90 mmol), and DIPEA (175 mg, 1.35 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-6** in the form of a white solid (53 mg, yield: 24.2%).

MS(m/z): 488.2 $[M+H]^+$.

$^1$H NMR (400 MHz, DMSO-$d_6$) δ 8.85 (s, 1H), 8.57 (d, *J* = 6.4 Hz, 1H), 7.51 (q, *J* = 7.6 Hz, 1H), 7.33-6.95 (m, 4H), 5.57 (s, 2H), 4.47 (t, *J= 8.4* Hz, 1H), 4.22 (td, *J*= 11.2, 4.0 Hz, 1H), 3.83 (q, *J*= 10.4 Hz, 1H), 3.15-2.87 (m, 4H), 2.17 (d, *J* = 13.6 Hz, 1H), 2.03 (t, *J* = 12.4 Hz, 1H), 1.51 (p, *J* = 6.8, 1H), 0.86 (d, *J* = 8.0 Hz, 2H), 0.69 (d, *J* = 5.2 Hz, 2H).

Example 24: Preparation of **Target Compound** 1-(2-fluorobenzyl)-N-(9-((1-methyl-1H-pyrazol-4-yl)ethynyl)-4-oxo-1,2,3,4,6,7-hexahydroazepino[3,2,1-hi]indol-3-yl)-1H-1,2,4-triazole-3-carboxamide **(III-7)**

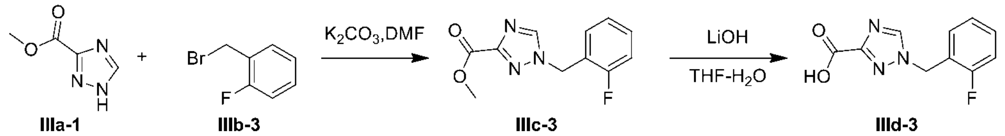


IIIa-1 IIIb-3 IIIc-3 IIId-3

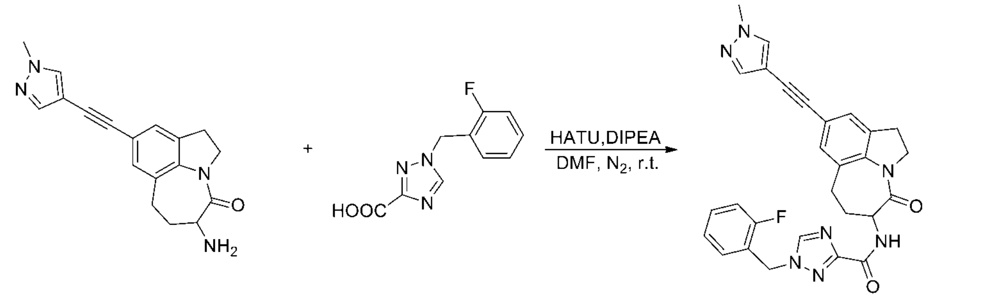


Ia-5 IIId-3 III-7

**Step** 1: **Preparation of compound IIId-3**

Compound **IIId-3** (1.56 g, two-step total yield: 47.1%) was obtained with reference to the "preparation of intermediate **IIId-1"** in Example 18, wherein only the starting material "2-(bromomethyl)-1,3-difluorobenzene" was replaced with "2-(bromomethyl)-1-fluorobenzene" while the remaining procedures were the same.

MS(m/z): 222.1 $[M+H]^+$.

$^1$H NMR (600 MHz, DMSO-$d_6$) δ 13.31 (s, 1H), 8.79 (s, 1H), 7.51-7.35 (m, 2H), 7.29-7.19 (m, 2H), 5.56 (s, 2H).

Step 2: Preparation of target compound III-7

Compound Ia-5 was obtained with reference to the "preparation of compound Ia-5" in Example 5.

In a 50 mL eggplant-shaped flask, intermediate Ia-5 (107 mg, 0.35 mmol), IIId-3 (78 mg, 0.35 mmol), HATU (266 mg, 0.7 mmol), and DIPEA (137 mg, 1.05 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-7** in the form of a white solid (10 mg, yield: 5.5%).

MS(m/z): 510.3 $[M+H]^+$.

1H NMR (600 MHz, DMSO-$d_6$) δ 8.82 (s, 1H), 8.60 (d, *J*= 6.6 Hz, 1H), 8.03 (s, 1H), 7.65 (s, 1H), 7.45-7.41 (m, 1H), 7.39-7.36 (m, 1H), 7.29-7.17 (m, 4H), 5.57 (s, 2H), 4.53-4.50 (m, 1H), 4.29-4.23 (m, 1H), 3.89-3.83 (m, 4H), 3.15-2.96 (m, 4H), 2.24-2.17 (m, 1H), 2.11-2.01 (m, 1H).

Example 25: Preparation of Target Compound 1-(4-fluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-8)**

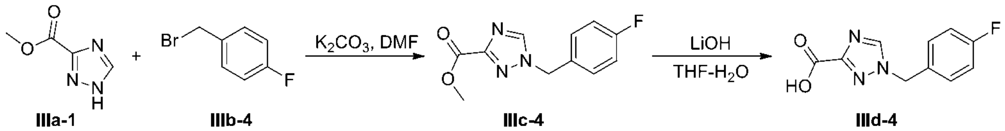


IIIa-1 IIIb-4 IIIc-4 IIId-4

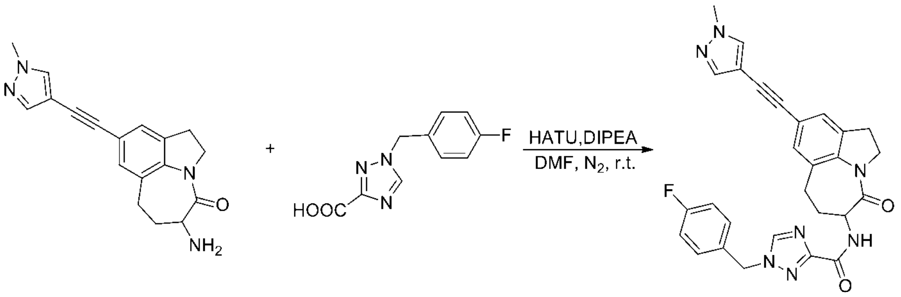


Ia-5 IIId-4 III-8

**Step 1: Preparation of compound IIId-4**

Compound **IIId-4** (1.65 g, two-step total yield: 50.0%) was obtained with reference to the "preparation of intermediate **IIId-1"** in Example 18, wherein only the starting material "2-(bromomethyl)-1,3-difluorobenzene" was replaced with "2-(bromomethyl)-4-fluorobenzene" while the remaining procedures were the same.

$^1$H NMR (600 MHz, DMSO-$d_6$) δ 13.30 (s, 1H), 8.79 (s, 1H), 7.42-7.38 (m, 2H), 7.24-7.19 (m, 2H), 5.48 (s, 2H). MS(m/z): 222.1 [M+H]$^+$.

Step 2: **Preparation of target compound III-8**

Compound **Ia-5** was obtained with reference to the "preparation of compound Ia-5" in Example 5.

In a 50 mL eggplant-shaped flask, intermediate Ia-5 (107 mg, 0.35 mmol), IIId-4 (78 mg, 0.35 mmol), HATU (266 mg, 0.7 mmol), and DIPEA (137 mg, 1.05 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-8** in the form of a white solid (12 mg, yield: 6.5%).

MS(m/z): 510.3 [M+H]$^+$.

$^1$H NMR (600 MHz, DMSO-$d_6$) δ 8.82 (s, 1H), 8.60 (d, $J$ = 6.6 Hz, 1H), 8.03 (s, 1H), 7.65 (s, 1H), 7.43-7.36 (m, 2H), 7.26-7.15 (m, 4H), 5.49 (s, 2H), 4.53-4.50 (m, 1H), 4.27-4.23 (m, 1H), 3.89-3.84 (m, 4H), 3.13-2.97 (m, 4H), 2.22-2.18 (m, 1H), 2.09-2.02 (m, 1H).

Example 26: Preparation of Target Compound 1-(2,4-difluorobenzyl)-N-(9-((1-methyl-1H-pyrazol-4-yl)ethynyl)-4-oxo-1,2,3,4,6,7-hexahydroazepino[3,2,1-hi]indol-3-yl)-1H-1,2,4-triazole-3-carboxamide (**III-9**)

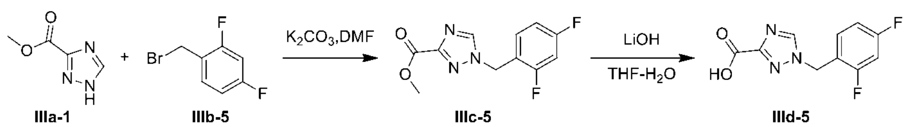


IIIa-1 IIIb-5 IIIc-5 IIId-5

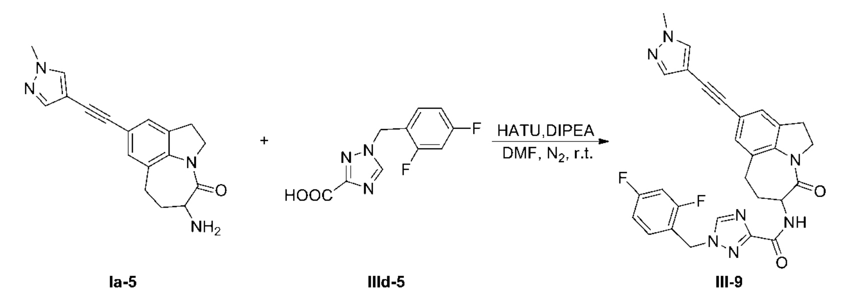


Ia-5 IIId-5 III-9

**Step** 1: **Preparation of compound IIId-5**

Compound **IIId-5** (1.29 g, two-step total yield: 54.0%) was obtained with reference to the "preparation of intermediate **IIId-1"** in Example 18, wherein only the starting material "2-(bromomethyl)-1,3-difluorobenzene" was replaced with "4-(bromomethyl)-1,3-difluorobenzene" while the remaining procedures were the same. MS(m/z): 240.1 $[M+H]^+$.

$^1$H NMR (600 MHz, DMSO-$d_6$) δ 13.31 (s, 1H), 8.78 (s, 1H), 7.51-7.46 (m, 1H), 7.34-7.29 (m, 1H), 7.16-7.12 (m, 1H), 5.53 (s, 2H).

**Step 2: Preparation of target compound III-9**

Compound **Ia-5** was obtained with reference to the "preparation of compound **Ia-5"** in Example 5.

In a 50 mL eggplant-shaped flask, intermediate **Ia-5** (107 mg, 0.35 mmol), **IIId-5** (84 mg, 0.35 mmol), HATU (266 mg, 0.7 mmol), and DIPEA (137 mg, 1.05 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-9** in the form of a white solid (15 mg, yield: 10.2%).

MS(m/z): 528.3 $[M+H]^+$.

$^1$H NMR (600 MHz, DMSO-$d_6$) δ 8.81 (s, 1H), 8.59 (d, *J* = 6.6 Hz, 1H), 8.03 (s, 1H), 7.65 (s, 1H), 7.51-7.47 (m, 1H), 7.32 (td, *J* = 10.2, 3.0 Hz, 1H), 7.23 (s, 1H), 7.20 (s, 1H), 7.14 (td, *J* = 8.4, 2.4 Hz, 1H), 5.54 (s, 2H), 4.53 - 4.50 (m, 1H), 4.29-4.20 (m, 1H), 3.89-3.83 (m, 4H), 3.14-2.96 (m, 4H), 2.22-2.18 (m, 1H), 2.08-2.02 (m, 1H).

**Example 27:** Preparation of **Target Compound** 1-(2,5-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-10)**

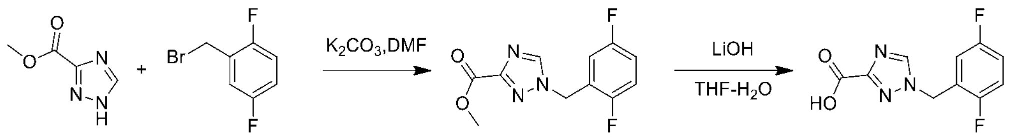


IIIa-1 IIIb-6 IIIc-6 IIId-6

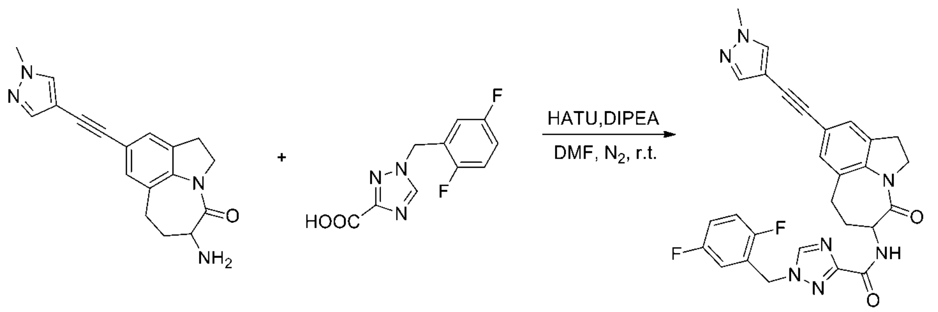


Ia-5 IIId-6 III-10

**Step 1: Preparation of compound IIId-6**

Compound **IIId-6** (510 mg, two-step total yield: 21.3%) was obtained with reference to the "preparation of intermediate **IIId-1**" in Example 18, wherein only the starting material "2-(bromomethyl)-1,3-difluorobenzene" was replaced with "2-(bromomethyl)-1,4-difluorobenzene" while the remaining procedures were the same. MS(m/z): 240.1 $[M+H]^+$.

$^1$H NMR (600 MHz, DMSO-$d_6$) δ 13.34 (s, 1H), 8.79 (s, 1H), 7.39-7.22 (m, 3H), 5.55 (s, 2H).

**Step 2: Preparation of target compound III-10**

Compound **Ia-5** was obtained with reference to the "preparation of compound **Ia-5**" in Example 5.

In a 50 mL eggplant-shaped flask, intermediate **Ia-5** (107 mg, 0.35 mmol), **IIId-6** (84 mg, 0.35 mmol), HATU (266 mg, 0.7 mmol), and DIPEA (137 mg, 1.05 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-10** in the form of a white solid (14 mg, yield: 9.7%).

MS(m/z): 528.3 $[M+H]^+$.

$^1H$ NMR (600 MHz, DMSO-$d_6$) δ 8.83 (s, 1H), 8.61 (d, *J* = 6.6 Hz, 1H), 8.03 (s, 1H), 7.65 (s, 1H), 7.33-7.28 (m, 3H), 7.23 (s, 1H), 7.20 (s, 1H), 5.56 (s, 2H), 4.53-4.50 (m, 1H), 4.28-4.22 (m, 1H), 3.89-3.84 (m, 4H), 3.15- 2.96 (m, 4H), 2.24-2.17 (m, 1H), 2.09-2.02 (m, 1H).

**Example 28:** Preparation of **Target Compound** 1-(3,5-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide (**III-11**)

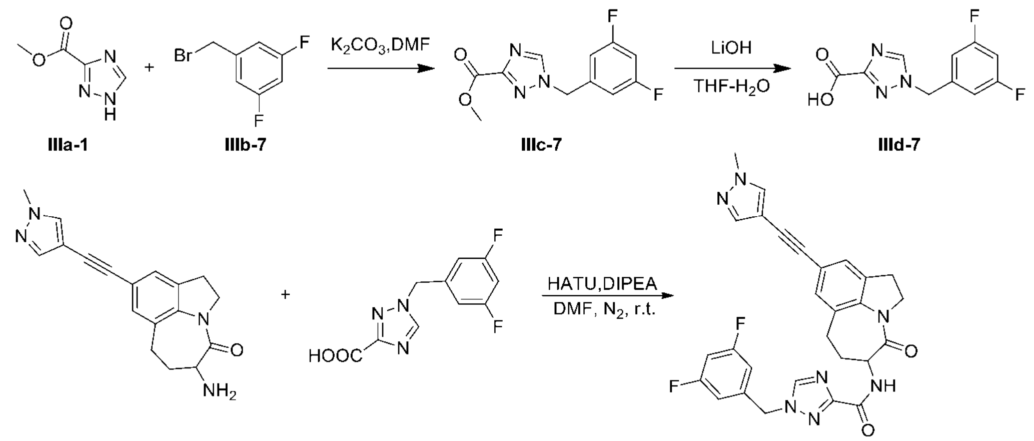


IIIa-1 IIIb-7 IIIc-7 IIId-7

Ia-5 IIId-7 III-11

### Step 1: Preparation of compound IIId-7

Compound **IIId-7** (1.06 g, two-step total yield: 44.4%) was obtained with reference to the "preparation of intermediate **IIId-1**" in Example 18, wherein only the starting material "2-(bromomethyl)-1,3-difluorobenzene" was replaced with "3-(bromomethyl)-1,5-difluorobenzene" while the remaining procedures were the same. MS(m/z): 240.1 $[M+H]^+$.

$^1H$ NMR (600 MHz, DMSO-$d_6$) δ 13.34 (s, 1H), 8.80 (s, 1H), 7.26-7.22 (m, 1H), 7.09-7.06 (m, 2H), 5.53 (s, 2H).

### Step 2: Preparation of target compound III-11

Compound **Ia-5** was obtained with reference to the "preparation of compound **Ia-5**" in Example 5.

In a 50 mL eggplant-shaped flask, intermediate **Ia-5** (107 mg, 0.35 mmol), **IIId-7** (84 mg, 0.35 mmol), HATU (266 mg, 0.7 mmol), and DIPEA (137 mg, 1.05 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-11** in the form of a white solid (17 mg, yield: 12.6%).

MS(m/z): 528.3 $[M+H]^+$.

$^1H$ NMR (600 MHz, DMSO-$d_6$) δ 8.84 (s, 1H), 8.63 (d, *J* = 6.6 Hz, 1H), 8.03 (s, 1H), 7.65 (s, 1H), 7.34-7.15 (m, 3H), 7.11-7.07 (m, 2H), 5.54 (s, 2H), 4.54-4.51 (m, 1H), 4.30-4.21 (m, 1H), 3.89-3.84 (m, 4H), 3.15-2.97 (m, 4H), 2.23-2.19 (m, 1H), 2.11-2.01 (m, 1H).

**Example 29:** Preparation of **Target Compound** 1-(3,4-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-12)**

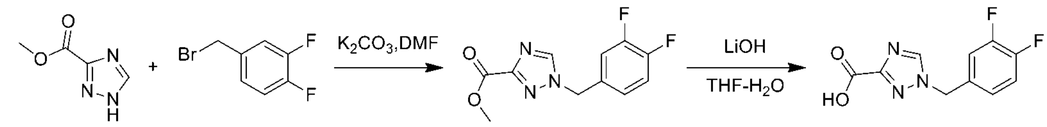


IIIa-1 IIIb-8 IIIc-8 IIId-8

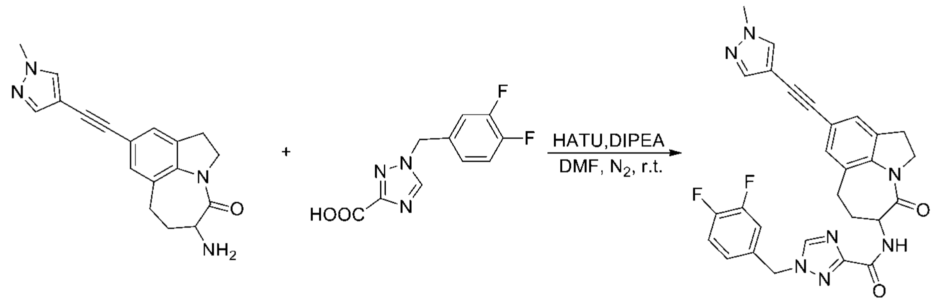


Ia-5 IIId-8 III-12

**Step 1: Preparation of compound IIId-8**

Compound **IIId-8** (1.08 g, two-step total yield: 45.2%) was obtained with reference to the "preparation of intermediate **IIId-1**" in Example 18, wherein only the starting material "2-(bromomethyl)-1,3-difluorobenzene" was replaced with "4-(bromomethyl)-1,2-difluorobenzene" while the remaining procedures were the same. MS(m/z): 240.1 $[M+H]^+$.

$^1$H NMR (600 MHz, DMSO-$d_6$) δ 13.31 (s, 1H), 8.79 (s, 1H), 7.53-7.42 (m, 2H), 7.21-7.18 (m, 1H), 5.49 (s, 2H).

**Step 2: Preparation of target compound III-12**

Compound **Ia-5** was obtained with reference to the "preparation of compound **Ia-5**" in Example 5.

In a 50 mL eggplant-shaped flask, intermediate **Ia-5** (107 mg, 0.35 mmol), **IIId-8** (84 mg, 0.35 mmol), HATU (266 mg, 0.7 mmol), and DIPEA (137 mg, 1.05 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-12** in the form of a white solid (18 mg, yield: 13.2%).

MS(m/z): 528.3 $[M+H]^+$.

$^1$H NMR (600 MHz, DMSO-$d_6$) δ 8.82 (s, 1H), 8.61 (d, *J* = 6.6 Hz, 1H), 8.03 (s, 1H), 7.65 (s, 1H), 7.54-7.40 (m, 2H), 7.31-7.12 (m, 3H), 5.50 (s, 2H), 4.54-4.51 (m, 1H), 4.28-4.23 (m, 1H), 3.88-3.84 (m, 4H), 3.15-2.97 (m, 4H), 2.22-2.18 (m, 1H), 2.10-2.01 (m, 1H).

**Example 30:** Preparation of **Target Compound** *N*-(9-((1-methyl-1*H*-pyrazol-4-yl)ethynyl)-4-oxo-1,2,3,4,6,7-hexahydroazepino[3,2,1-*hi*]indol-3-yl)-1-(2-methylbenzyl)-1*H*-1,2,4-triazole-3-carboxamide **(III-13)**

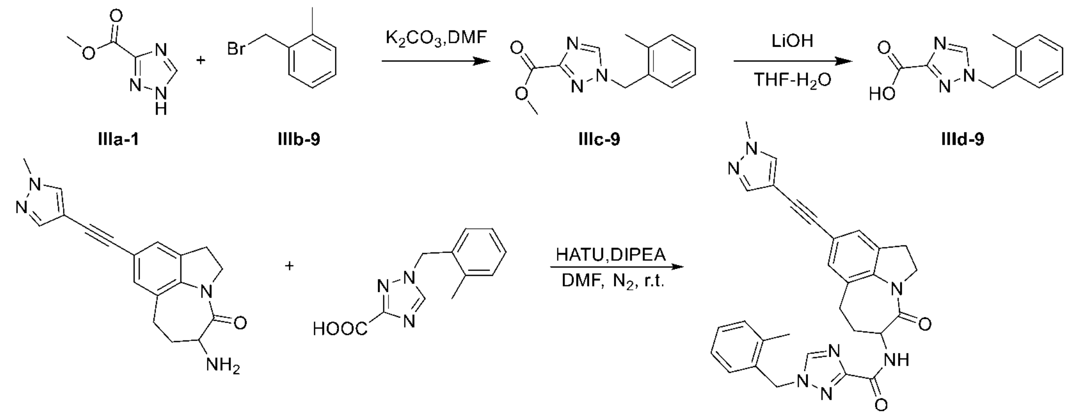


IIIa-1 IIIb-9 IIIc-9 IIId-9

Ia-5 IIId-9 III-13

**Step 1: Preparation of compound IIId-9**

Compound **IIId-9** (1.16 g, two-step total yield: 51.9%) was obtained with reference to the "preparation of intermediate **IIId-1"** in Example 18, wherein only the starting material "2-(bromomethyl)-1,3-difluorobenzene" was replaced with "2-(bromomethyl)-1-methylbenzene" while the remaining procedures were the same. MS(m/z): 218.1 $[M+H]^+$.

$^1$H NMR (600 MHz, DMSO-$d_6$) δ 13.31 (s, 1H), 8.74 (s, 1H), 7.30-7.16 (m, 3H), 7.09-7.08 (m, 1H), 5.49 (s, 2H), 2.31 (s, 3H).

**Step 2: Preparation of target compound III-13**

Compound **Ia-5** was obtained with reference to the "preparation of compound **Ia-5**" in Example 5.

In a 50 mL eggplant-shaped flask, intermediate **Ia-5** (107 mg, 0.35 mmol), **IIId-9** (76 mg, 0.35 mmol), HATU (266 mg, 0.7 mmol), and DIPEA (137 mg, 1.05 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **III-13** in the form of a white solid (19 mg, yield: 15.4%).

MS(m/z): 506.3 $[M+H]^+$.

$^1$H NMR (400 MHz, DMSO-$d_6$) δ 8.78 (s, 1H), 8.61 (d, *J* = 6.4 Hz, 1H), 8.04 (s, 1H), 7.66 (s, 1H), 7.33-7.15 (m, 5H), 7.09 (d, *J* = 7.6 Hz, 1H), 5.52 (s, 2H), 4.54-4.50 (m, 1H), 4.29-4.23 (m, 1H), 3.90-3.83 (m, 4H), 3.13-2.97 (m, 4H), 2.33 (s, 3H), 2.26-2.17 (m, 1H), 2.11-2.01 (m, 1H).

**Example 31:** Preparation of **Target Compound** *N*-(9-((1-methyl-1*H*-pyrazol-4-yl)ethynyl)-4-oxo-1,2,3,4,6,7-hexahydroazepino[3,2,1-*hi*]indol-3-yl)-1-(4-methylbenzyl)-1*H*-1,2,4-triazole-3-carboxamide **(III-14)**

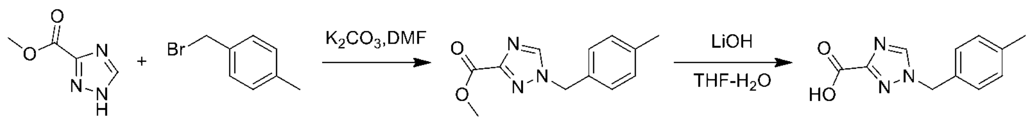


IIIa-1 IIIb-10 IIIc-10 IIId-10

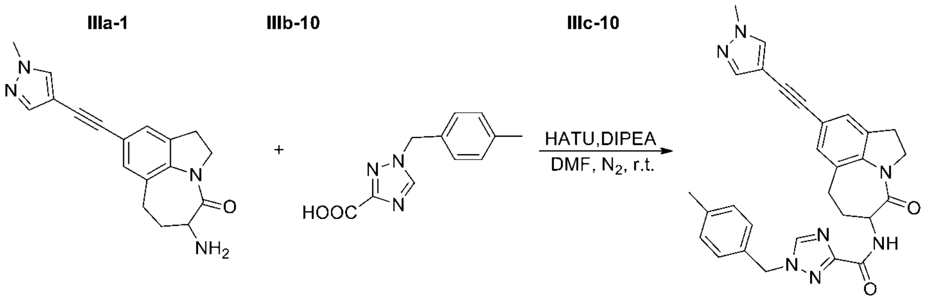


Ia-5 IIId-10 III-14

**Step 1: Preparation of compound IIId-10**

Compound **IIId-10** (930 mg, two-step total yield: 43.1%) was obtained with reference to the "preparation of intermediate **IIId-1"** in Example 18, wherein only the starting material "2-(bromomethyl)-1,3-difluorobenzene" was replaced with "4-(bromomethyl)-1-methylbenzene" while the remaining procedures were the same. MS(m/z): 218.1 $[M+H]^+$.

$^1$H NMR (400 MHz, DMSO-$d_6$) $\delta$ 13.28 (s, 1H), 8.78 (s, 1H), 7.23-7.17 (m, 4H), 5.42 (s, 2H), 2.28 (s, 3H).

**Step 2: Preparation of target compound III-14**

Compound **Ia-5** was obtained with reference to the "preparation of compound **Ia-5**" in Example 5.

In a 50 mL eggplant-shaped flask, intermediate **Ia-5** (107 mg, 0.35 mmol), **IIId-10** (76 mg, 0.35 mmol), HATU (266 mg, 0.7 mmol), and DIPEA (137 mg, 1.05 mmol) were dissolved in anhydrous DMF (5 mL), and the system was thoroughly purged with nitrogen and allowed to react overnight at room temperature. After thin layer chromatography showed that the reaction of the starting materials was substantially completed, 20 mL of water was added to the system, and the system was extracted with DCM for layer separation (20 mL $\times$ 3). The organic phases were combined, washed with a saturated sodium chloride solution, dried over anhydrous sodium sulfate, filtered, and concentrated under reduced pressure. The residue was purified by preparative liquid chromatography to give the target compound **III-14** in the form of a white solid (15 mg, yield: 14.2%).

MS(m/z): 506.3 $[M+H]^+$.

$^1$H NMR (400 MHz, DMSO-$d_6$) $\delta$ 8.81 (s, 1H), 8.59 (d, $J$ = 6.4 Hz, 1H), 8.03 (s, 1H), 7.66 (s, 1H), 7.33-7.11 (m, 6H), 5.44 (s, 2H), 4.54-4.49 (m, 1H), 4.29-4.22 (m, 1H), 3.90-3.82 (m, 4H), 3.12-2.98 (m, 4H), 2.29 (s, 3H), 2.23-2.17 (m, 1H), 2.09-2.00 (m, 1H).

**Example 32:** Preparation of **Target Compound** 1-(2,6-difluorobenzyl)-*N*-(4-oxo-9-(piperidin-4-ylethynyl)-1,2,3,4,6,7-hexahydroazepino[3,2,1-*hi*]indol-3-yl)-1*H*-1,2,4-triazole-3-carboxamide (**III-15)**

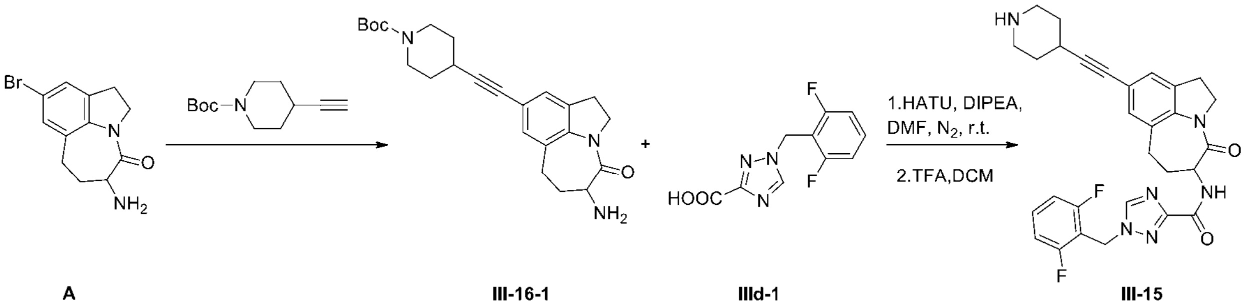


A III-16-1 IIId-1 III-15

**Step 1: Preparation of compound III-16-1**

Intermediate **A** was obtained with reference to the "method for preparing common intermediate A".

Compound **III-16-1** (228 mg, yield: 27.9%) was obtained with reference to the "method for preparing **Ia-1"** in Example 1, wherein the intermediate "ethynylcyclopropane" was replaced with "1-Boc-4-ethynylpiperidine" while the remaining procedures followed the "method for preparing **Ia-1"** in Example 1.
MS(m/z): 410.1 $[M+H]^+$.

**Step 2: Preparation of target compound III-15**

Intermediate **IIId-1** was obtained with reference to the "method for preparing intermediate **IIId-1"** in Example 18. In a 50 mL eggplant-shaped flask, intermediate **III-16-1** (147 mg, 0.36 mmol), **IIId-1** (172 mg, 0.72 mmol), HATU (273 mg, 0.72 mmol), and DIPEA (233 mg, 1.80 mmol) were dissolved in anhydrous DMF (8 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was deprotected using TFA/DCM (5 mL), concentrated, and then purified by preparative liquid chromatography to give the target compound **III-15** in the form of a white solid (10 mg, yield: 5.26%).
MS(m/z): 531.2 $[M+H]^+$.
$^1H$ NMR (400 MHz, DMSO-$d_6$) δ 8.85 (s, 1H), 8.58 (d, *J*= 6.4 Hz, 1H), 8.37 (s, 1H), 7.56-7.48 (m, 1H), 7.19 (t, *J* = 8.0 Hz, 2H), 7.16 (s, 1H), 7.12 (s, 1H), 5.58 (s, 2H), 4.50-4.45 (m, 1H), 4.27-4.20 (m, 1H), 3.88-3.80 (m, 1H), 3.13-2.93 (m, 6H), 2.85-2.81 (m, 3H), 2.20-2.16 (m, 1H), 2.08-1.97 (m, 1H), 1.92-1.89 (m, 2H), 1.68-1.56 (m, 2H).

**Example 33:** Preparation of **Target Compound** *tert*-butyl 4-((3-(1-(2,6-difluorobenzyl)-1*H*-1,2,4-triazole-3-carboxamido)-4-oxo-1,2,3,4,6,7-hexahydroazepino[3,2,1-*hi*]indol-9-yl)ethynyl)piperidine-1-carboxylate **(III-16)**

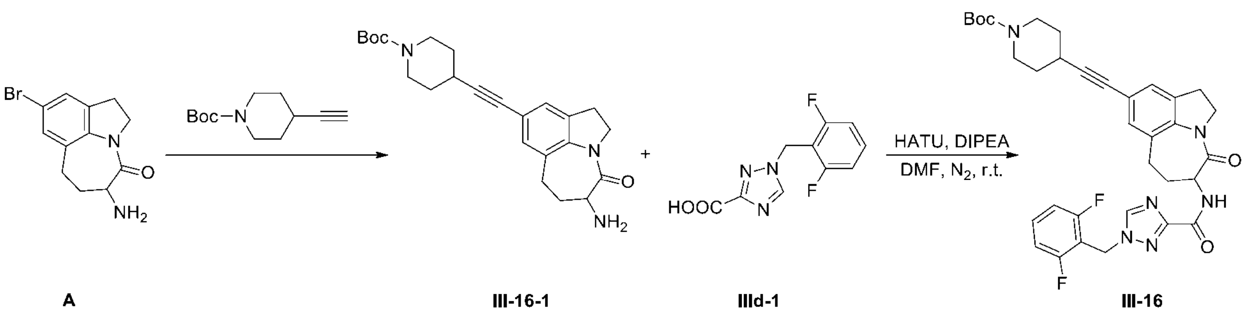


**A** **III-16-1** **IIId-1** **III-16**

**Step 1: Preparation of compound III-16-1**

Compound **III-16-1** was obtained with reference to the "preparation of compound **III-16-1"** in Example 32. MS(m/z): 410.1 $[M+H]^+$.

**Step 2: Preparation of target compound III-16**

Intermediate **IIId-1** was obtained with reference to the "method for preparing intermediate **IIId-1"** in Example 18. In a 50 mL eggplant-shaped flask, intermediate **III-16-1** (147 mg, 0.36 mmol), **IIId-1** (172 mg, 0.72 mmol), HATU (273 mg, 0.72 mmol), and DIPEA (233 mg, 1.80 mmol) were dissolved in anhydrous DMF (8 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative chromatography to give the target compound **III-16** in the form of a white solid (10 mg, yield: 9.5%).
MS(m/z): 631.3 $[M+H]^+$.
$^1H$ NMR (600 MHz, DMSO-$d_6$) δ 8.85 (s, 1H), 8.58 (d, *J*= 6.6 Hz, 1H), 7.54-7.49 (m, 1H), 7.19 (t, *J*= 7.8 Hz, 2H), 7.15 (s, 1H), 7.11 (s, 1H), 5.57 (s, 2H), 4.48-4.46 (m, 1H), 4.25-4.21 (m, 1H), 3.83 (q, *J* = 10.2 Hz, 1H), 3.65-3.61 (m, 2H), 3.19-3.10 (m, 2H), 3.08-2.93 (m, 4H), 2.86-2.80 (m, 1H), 2.19-2.14 (m, 1H), 2.06-1.98 (m, 1H), 1.82-1.78 (m, 2H), 1.51-1.48 (m, 2H), 1.40 (s, 9H).

**Example 34:** Preparation of **Target Compound** 1-(2,6-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-17)**

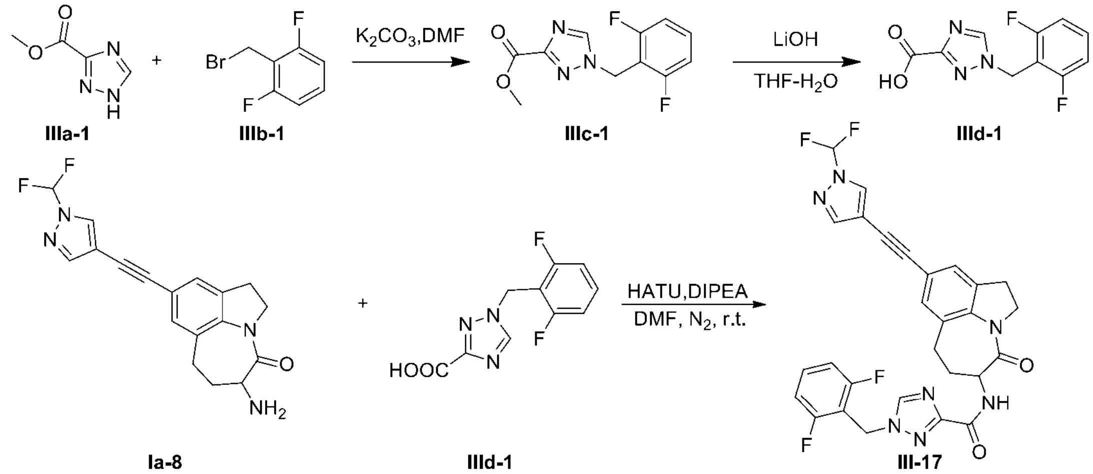


IIIa-1 IIIb-1 IIIc-1 IIId-1

Ia-8 IIId-1 III-17

**Step 1: Preparation of compound Ia-8**

Compound **Ia-8** was obtained with reference to the "preparation of intermediate **Ia-5"** in Example 5, wherein only the starting material "N-methyl-4-alkynylpyrazole" was replaced with "*N*-difluoromethyl-4-alkynylpyrazole" while the remaining procedures were the same.

MS(m/z): 343.2 $[M+H]^+$.

**Step 2: Preparation of target compound III-17**

Target compound **III-17** (59 mg, yield: 10.28%) was obtained with reference to the synthesis method in step 3 in Example 18, wherein only the intermediate **Ia-5** was replaced with **Ia-8** while the remaining procedures were the same.

MS(m/z): 564.2 $[M+H]^+$.

$^1$H NMR (400 MHz, DMSO-$d_6$) δ 8.85 (s, 1H), 8.63 (s, 1H), 8.60 (d, *J*= 6.8 Hz, 1H), 8.04 (s, 1H), 7.84 (t, *J*= 58.8 Hz, 1H), 7.52 (p, *J* = 7.6 Hz, 1H), 7.26 (d, *J* = 10.4 Hz, 2H), 7.19 (t, *J* = 8.0 Hz, 2H), 5.58 (s, 2H), 4.54-4.47 (m, 1H), 4.28-4.22 (m, 1H), 3.87 (q, *J* = 10.4 Hz, 1H), 3.13-3.00 (m, 4H), 2.21-2.03 (m, 1H), 2.11-2.01 (m, 1H).

**Example 35:** Preparation of **Target Compound** 1-(3,5-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-18)**

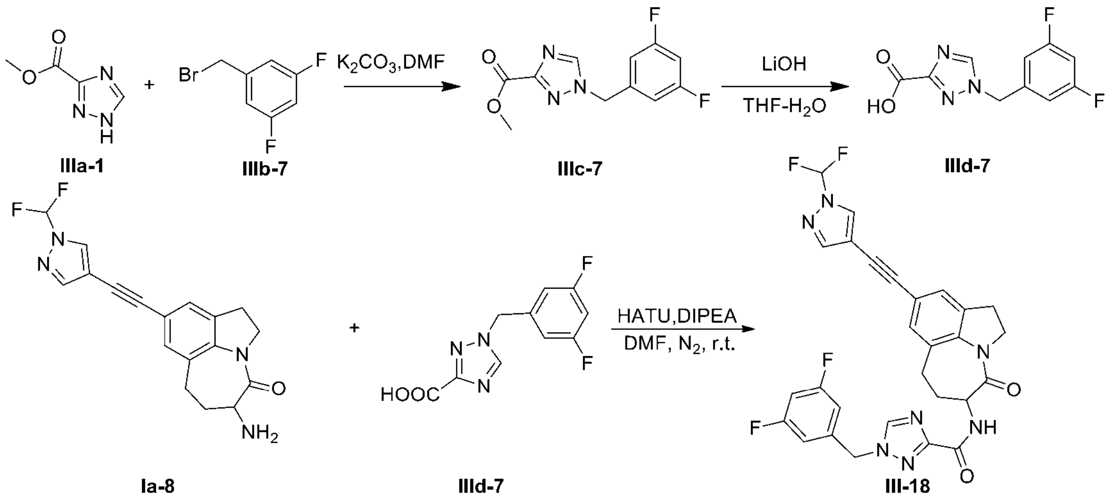


IIIa-1 IIIb-7 IIIc-7 IIId-7

Ia-8 IIId-7 III-18

**Step 1: Preparation of compound IIId-7**

Compound **IIId-7** (1.06 g, two-step total yield: 44.4%) was obtained with reference to the "preparation of intermediate **IIId-1"** in Example 18, wherein only the starting material "2-(bromomethyl)-1,3-difluorobenzene" was replaced with "3-(bromomethyl)-1,5-difluorobenzene" while the remaining procedures were the same. MS(m/z): 240.1 $[M+H]^+$.

**Step 2: Preparation of target compound III-18**

Target compound **III-18** (41 mg, yield: 13.68%) was obtained with reference to the synthesis method in step 3 in Example 18, wherein only the intermediate **Ia-5** was replaced with **Ia-8** and intermediate **IIId-1** was replaced with **IIId-7,** while the remaining procedures were the same.

MS(m/z): 564.2 $[M+H]^+$.

$^1$H NMR (400 MHz, DMSO-$d_6$) δ 8.84 (s, 1H), 8.64 (d, *J*= 8.4 Hz, 2H), 8.04 (s, 1H), 7.84 (t, *J* = 58.8 Hz, 1H), 7.36 -7.19 (m, 3H), 7.09 (d, *J* = 6.0 Hz, 2H), 5.54 (s, 2H), 4.54 (t, *J* = 8.0 Hz, 1H), 4.26 (td, *J* = 11.2, 10.4, 4.0 Hz, 1H), 3.88 (q, *J*= 10.4 Hz, 1H), 3.20-2.95 (m, 4H), 2.27-2.16 (m, 1H), 2.15-2.00 (m, 1H).

**Example 36:** Preparation of **Target Compound** 1-(2,5-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide; **(III-19)**

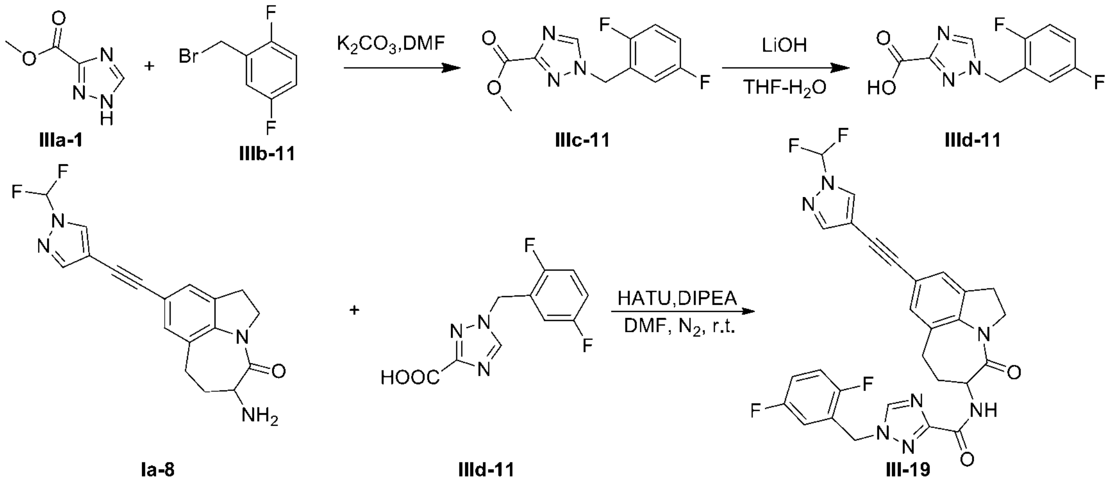


IIIa-1 IIIb-11 IIIc-11 IIId-11

Ia-8 IIId-11 III-19

**Step 1: Preparation of compound IIId-11**

Compound **IIId-11** was obtained with reference to the "preparation of intermediate **IIId-1"** in Example 18, wherein only the starting material "2-(bromomethyl)-1,3-difluorobenzene" was replaced with "2,5-difluorobenzyl bromide" while the remaining procedures were the same.
MS(m/z): 240.1 $[M+H]^+$.

**Step 2: Preparation of target compound III-19**

Target compound **III-19** (36 mg, yield: 9.68%) was obtained with reference to the synthesis method in step 3 in Example 18, wherein only the intermediate **Ia-5** was replaced with **Ia-8** and intermediate **IIId-1** was replaced with **IIId-11,** while the remaining procedures were the same.
MS(m/z): 564.2 $[M+H]^+$.
$^1H$ NMR (400 MHz, DMSO-$d_6$) δ 8.83 (s, 1H), 8.63 (s, 1H), 8.62 (d, *J* = 6.8, 1H), 8.04 (s, 1H), 7.84 (t, *J* = 58.8 Hz, 1H), 7.36-7.25 (m, 5H), 5.56 (s, 2H), 4.56-4.51 (m, 1H), 4.29-4.23 (m, 1H), 3.87 (q, *J* = 10.4 Hz, 1H), 3.13-2.99 (m, 4H), 2.24 -2.18 (m, 1H), 2.11-2.01 (m, 1H).

**Example 37:** Preparation of **Target Compound** 1-(2,4-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-20)**

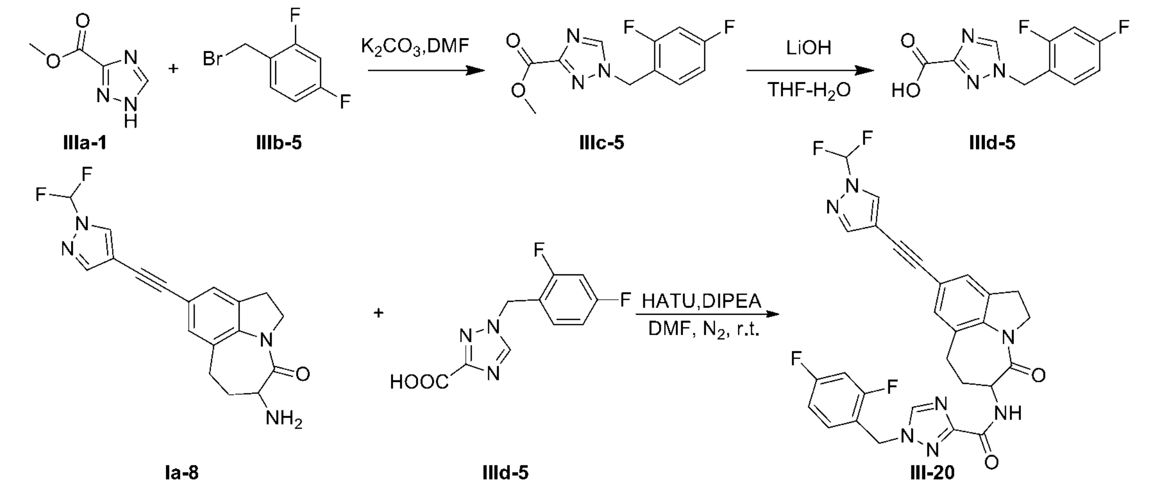


**Step 1: Preparation of compound IIId-5**

Compound **IIId-5** (1.29 g, two-step total yield: 54.0%) was obtained with reference to the "preparation of intermediate **IIId-1"** in Example 18, wherein only the starting material "2-(bromomethyl)-1,3-difluorobenzene" was replaced with "4-(bromomethyl)-1,3-difluorobenzene" while the remaining procedures were the same. MS(m/z): 240.1 $[M+H]^+$.

**Step 2: Preparation of target compound III-20**

Target compound **III-20** (38 mg, yield: 10.57%) was obtained with reference to the synthesis method in step 3 in Example 18, wherein only the intermediate **Ia-5** was replaced with **Ia-8** and intermediate **IIId-1** was replaced with **IIId-5,** while the remaining procedures were the same.
MS(m/z): 564.2 $[M+H]^+$.
$^1H$ NMR (400 MHz, DMSO-$d_6$) δ 8.82 (s, 1H), 8.63 (s, 1H), 8.61 (d, *J*=6.4 Hz, 1H), 8.04 (s, 1H), 7.84 (t, *J*=58.8 Hz, 1H), 7.53-7.47 (m, 1H), 7.35-7.30 (m, 1H), 7.28-7.25 (m, 2H), 7.17-7.12 (m, 1H), 5.55 (s, 2H), 4.55-4.50 (m, 1H), 4.29-4.22 (m, 1H), 3.87 (q, *J* = 10.8 Hz, 1H), 3.13-3.01 (m, 4H), 2.26-2.16 (m, 1H), 2.11-2.01 (m, 1H).

**Example 38:** Preparation of **Target Compound** *N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1-(2-fluorobenzyl)-1*H*-1,2,4-triazole-3-carboxamide **(III-21)**

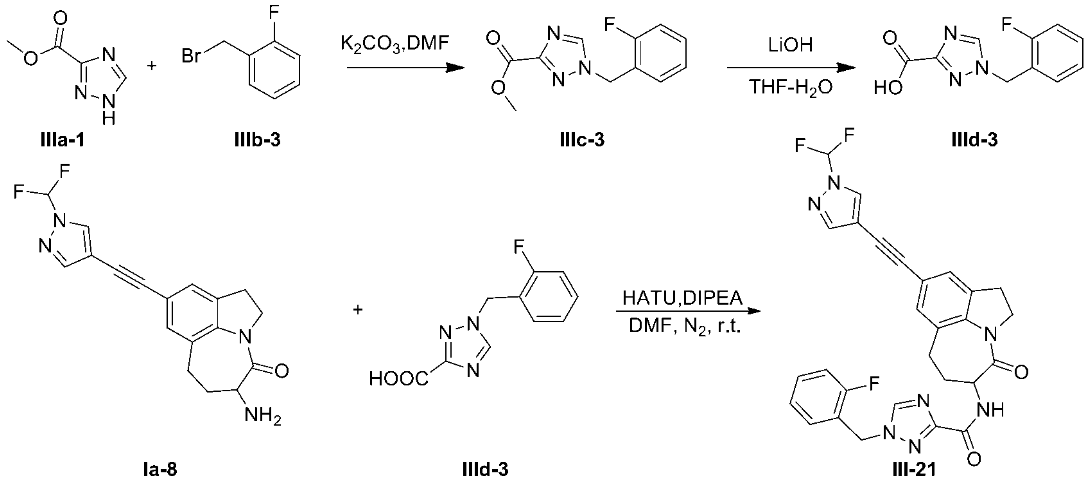


**IIIa-1** **IIIb-3** **IIIc-3** **IIId-3**

**Ia-8** **IIId-3** **III-21**

**Step** 1: **Preparation of compound IIId-3**

Compound **IIId-3** (1.56 g, two-step total yield: 47.1%) was obtained with reference to the "preparation of intermediate **IIId-1**" in Example 18, wherein only the starting material "2-(bromomethyl)-1,3-difluorobenzene" was replaced with "2-(bromomethyl)-1-fluorobenzene" while the remaining procedures were the same.

MS(m/z): 222.1 $[M+H]^+$.

$^1$H NMR (600 MHz, DMSO-$d_6$) δ 13.31 (s, 1H), 8.79 (s, 1H), 7.51-7.35 (m, 2H), 7.29-7.19 (m, 2H), 5.56 (s, 2H).

**Step 2: Preparation of target compound III-21**

Target compound **III-21** (38 mg, yield: 10.57%) was obtained with reference to the synthesis method in step 3 in Example 18, wherein only the intermediate **Ia-5** was replaced with **Ia-8** and intermediate **IIId-1** was replaced with **IIId-3,** while the remaining procedures were the same.

MS(m/z): 546.2 $[M+H]^+$.

$^1$H NMR (400 MHz, DMSO-$d_6$) δ 8.83 (s, 1H), 8.63 (s, 1H), 8.62 (d, *J*= 6.8 Hz, 1H), 8.04 (s, 1H), 7.84 (t, *J*= 58.8 Hz, 1H), 7.46-7.36 (m, 2H), 7.28-7.22 (m, 4H), 5.57 (s, 2H), 4.55-4.50 (m, 1H), 4.29-4.22 (m, 1H), 3.87 (q, *J* = 10.4 Hz, 1H), 3.14-2.99 (m, 4H), 2.23-2.17 (m, 1H), 2.11-2.00 (m, 1H).

**Example 39:** Preparation of **Target Compound** *N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1-(2-methylbenzyl)-1*H*-1,2,4-triazole-3-carboxamide **(III-22)**

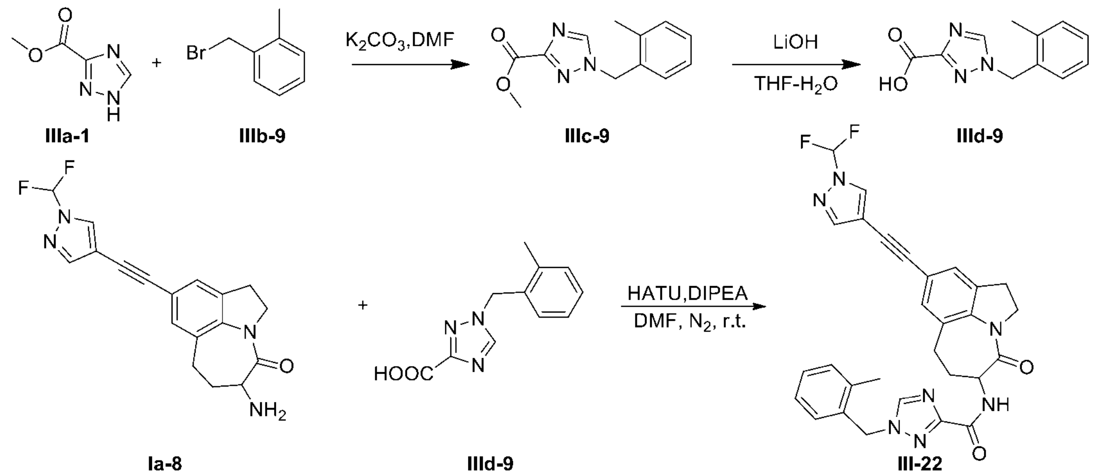


IIIa-1 IIIb-9 IIIc-9 IIId-9

Ia-8 IIId-9 III-22

**Step 1: Preparation of compound IIId-9**

Compound **IIId-9** (1.16 g, two-step total yield: 51.9%) was obtained with reference to the "preparation of intermediate **IIId-1"** in Example 18, wherein only the starting material "2-(bromomethyl)-1,3-difluorobenzene" was replaced with "2-(bromomethyl)-1-methylbenzene" while the remaining procedures were the same. MS(m/z): 218.1 $[M+H]^+$.

$^1$H NMR (600 MHz, DMSO-$d_6$) δ 13.31 (s, 1H), 8.74 (s, 1H), 7.30-7.16 (m, 3H), 7.09-7.08 (m, 1H), 5.49 (s, 2H), 2.31 (s, 3H).

**Step 2: Preparation of target compound III-22**

Target compound **III-22** (23 mg, yield: 8.75%) was obtained with reference to the synthesis method in step 3 in Example 18, wherein only the intermediate **Ia-5** was replaced with **Ia-8** and intermediate **IIId-1** was replaced with **IIId-9,** while the remaining procedures were the same.

MS(m/z): 542.3 $[M+H]^+$.

$^1$H NMR (400 MHz, DMSO-$d_6$) δ 8.79 (s, 1H), 8.64 (s, 1H), 8.62 (d, *J*=6.4 Hz, 1H), 8.05 (s, 1H), 7.84 (t, *J*=58.8 Hz, 1H), 7.30-7.19 (m, 5H), 7.09 (d, *J* = 7.6 Hz, 1H), 5.52 (s, 2H), 4.56-4.51 (m, 1H), 4.30-4.23 (m, 1H), 3.88 (q, *J* = 10.4 Hz, 1H), 3.16-2.99 (m, 4H), 2.34 (s, 3H), 2.25-2.17 (m, 1H), 2.12-2.01 (m, 1H).

**Example 40:** Preparation of **Target Compound** 1-(3,4-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-23)**

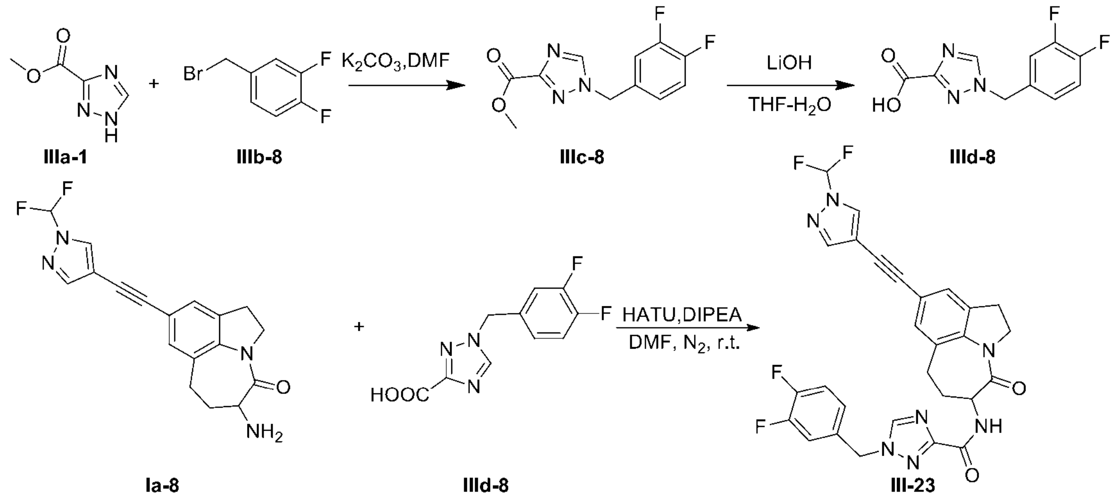


IIIa-1 IIIb-8 IIIc-8 IIId-8

Ia-8 IIId-8 III-23

Step **1: Preparation of compound IIId-8**

Compound **IIId-8** (1.08 g, two-step total yield: 45.2%) was obtained with reference to the "preparation of intermediate **IIId-1"** in Example 18, wherein only the starting material "2-(bromomethyl)-1,3-difluorobenzene" was replaced with "4-(bromomethyl)-1,2-difluorobenzene" while the remaining procedures were the same. MS(m/z): 240.1 $[M+H]^+$.

$^1$H NMR (600 MHz, DMSO-$d_6$) δ 13.31 (s, 1H), 8.79 (s, 1H), 7.53-7.42 (m, 2H), 7.21-7.18 (m, 1H), 5.49 (s, 2H).

Step 2: **Preparation of target compound III-23**

Target compound **III-23** (47 mg, yield: 9.25%) was obtained with reference to the synthesis method in step 3 in Example 18, wherein only the intermediate **Ia-5** was replaced with **Ia-8** and intermediate **IIId-1** was replaced with **IIId-8,** while the remaining procedures were the same.

MS(m/z): 564.2 $[M+H]^+$.

$^1$H NMR (400 MHz, DMSO-$d_6$) δ 8.83 (s, 1H), 8.66-8.60 (m, 2H), 8.04 (s, 1H), 7.84 (t, *J* = 58.8 Hz, 1H), 7.52-7.42 (m, 2H), 7.30-7.24 (m, 2H), 7.23-7.19 (m, 1H), 5.50 (s, 2H), 4.56-4.51 (m, 1H), 4.29-4.23 (m, 1H), 3.74 (q, *J* = 10.0 Hz, 1H), 3.13-3.01 (m, 4H), 2.23-2.19 (m, 1H), 2.12-2.02 (m, 1H).

**Example 41:** Preparation of **Target Compound** *N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1-(4-fluorobenzyl)-1*H*-1,2,4-triazole-3-carboxamide **(III-24)**

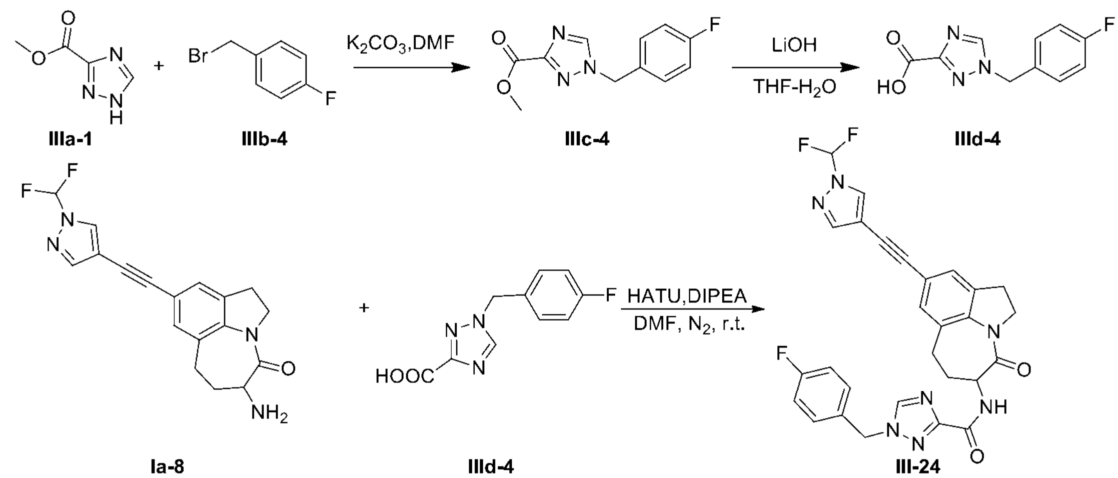


IIIa-1 IIIb-4 IIIc-4 IIId-4

Ia-8 IIId-4 III-24

**Step 1: Preparation of compound IIId-4**

Compound **IIId-4** (1.65 g, two-step total yield: 50.0%) was obtained with reference to the "preparation of intermediate **IIId-1**" in Example 18, wherein only the starting material "2-(bromomethyl)-1,3-difluorobenzene" was replaced with "2-(bromomethyl)-4-fluorobenzene" while the remaining procedures were the same.

$^1$H NMR (600 MHz, DMSO-$d_6$) δ 13.30 (s, 1H), 8.79 (s, 1H), 7.42-7.38 (m, 2H), 7.24-7.19 (m, 2H), 5.48 (s, 2H). MS(m/z): 222.1 $[M+H]^+$.

**Step 2: Preparation of target compound III-24**

Target compound **III-24** (40 mg, yield: 10.45%) was obtained with reference to the synthesis method in step 3 in Example 18, wherein only the intermediate **Ia-5** was replaced with **Ia-8** and intermediate **IIId-1** was replaced with **IIId-4,** while the remaining procedures were the same.

MS(m/z): 546.2 $[M+H]^+$.

$^1$H NMR (400 MHz, DMSO-$d_6$) δ 8.83 (s, 1H), 8.62 (d, *J* = 8.4 Hz, 2H), 8.04 (s, 1H), 7.84 (t, *J* = 58.8 Hz, 1H), 7.45-7.36 (m, 2H), 7.30-7.18 (m, 4H), 5.50 (s, 2H), 4.57-4.50 (m, 1H), 4.30-4.21 (m, 1H), 3.93-3.82 (m, 1H), 3.19-2.95 (m, 4H), 2.25-2.16 (m, 1H), 2.12-2.00 (m, 1H).

**Example 42:** Preparation of **Target Compound** *N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1-(4-methylbenzyl)-1*H*-1,2,4-triazole-3-carboxamide **(III-25)**

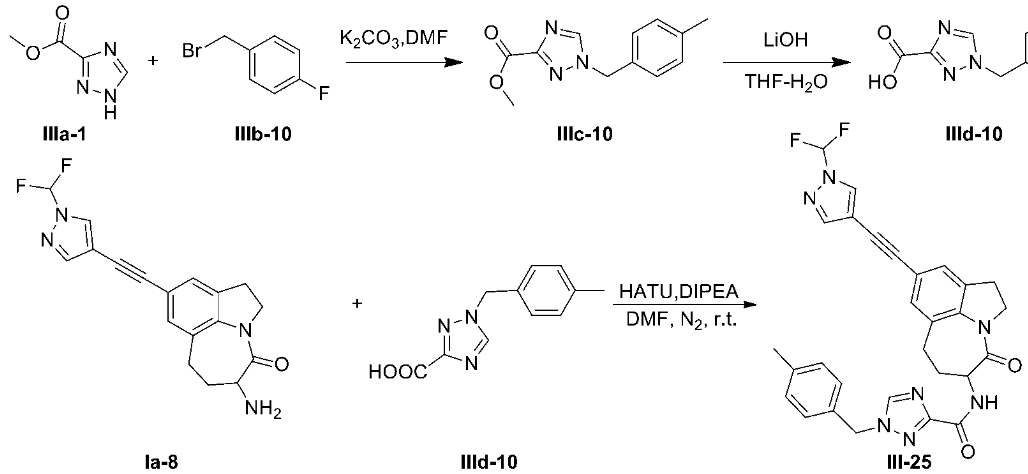


IIIa-1 IIIb-10 IIIc-10 IIId-10

Ia-8 IIId-10 III-25

**Step 1: Preparation of compound IIId-10**

Compound **IIId-10** (930 mg, two-step total yield: 43.1%) was obtained with reference to the "preparation of intermediate **IIId-1"** in Example 18, wherein only the starting material "2-(bromomethyl)-1,3-difluorobenzene" was replaced with "4-(bromomethyl)-1-methylbenzene" while the remaining procedures were the same. MS(m/z): 218.1 $[M+H]^+$.

$^1$H NMR (400 MHz, DMSO-$d_6$) δ 13.28 (s, 1H), 8.78 (s, 1H), 7.23-7.17 (m, 4H), 5.42 (s, 2H), 2.28 (s, 3H).

**Step 2: Preparation of target compound III-25**

Target compound **III-25** (38 mg, yield: 9.82%) was obtained with reference to the synthesis method in step 3 in Example 18, wherein only the intermediate **Ia-5** was replaced with **Ia-8** and intermediate **IIId-1** was replaced with **IIId-10,** while the remaining procedures were the same.

MS(m/z): 543.2 $[M+H]^+$.

$^1$H NMR (400 MHz, DMSO-$d_6$) δ 8.81 (s, 1H), 8.63 (s, 1H), 8.61 (d, *J*=6.4 Hz, 1H), 8.04 (s, 1H), 7.84 (t, *J*=58.8 Hz, 1H), 7.30-7.16 (m, 6H), 5.44 (s, 2H), 4.57-4.50 (m, 1H), 4.30-4.21 (m, 1H), 3.87 (q, *J* = 10.4 Hz, 1H), 3.18-2.96 (m, 4H), 2.29 (s, 3H), 2.25-2.16 (m, 1H), 2.12-2.00 (m, 1H).

**Example 43:** Preparation of **Target Compound** 1-(2,6-difluorobenzyl)-*N*-(9-ethynyl-4-oxo-1,2,3,4,6,7-hexahydroazepino[3,2,1-*hi*]indol-3-yl)-1*H*-1,2,4-triazole-3-carboxamide **(III-26)**

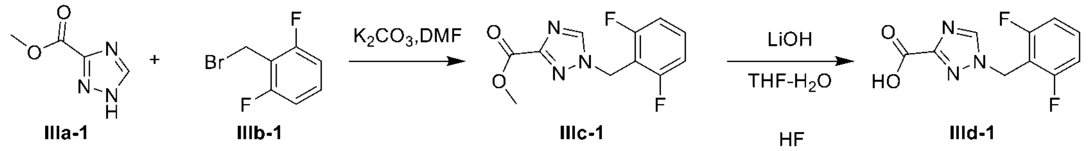


IIIa-1 IIIb-1 IIIc-1 IIId-1

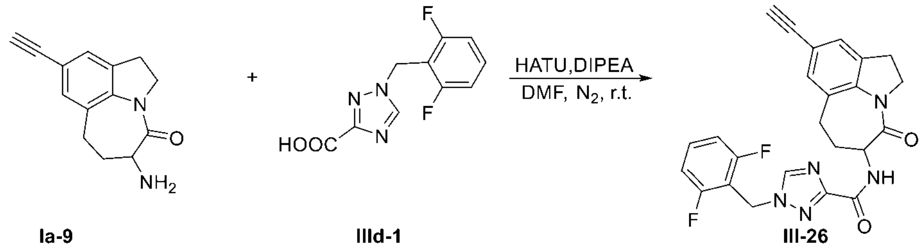


Ia-9 IIId-1 III-26

**Step 1: Preparation of compound Ia-9**

Intermediate **A** was obtained with reference to the "method for preparing common intermediate A".

Compound **Ia-9** (85 mg, yield: 32.4%) was obtained with reference to the "method for preparing **Ia-1**" in Example 1, wherein the intermediate "ethynylcyclopropane" was replaced with "TBDMS acetylene" while the remaining procedures followed the "method for preparing **Ia-1**" in Example 1.

MS(m/z): 227.1 $[M+H]^+$.

**Step 2: Preparation of target compound III-26**

Target compound **III-26** (25 mg, yield: 5.14%) was obtained with reference to the synthesis method in step 3 in Example 18, wherein only the intermediate **Ia-5** was replaced with **Ia-9** while the remaining procedures were the same.

MS(m/z): 448.2 $[M+H]^+$.

$^1$H NMR (400 MHz, DMSO-$d_6$) δ 8.85 (s, 1H), 8.59 (d, *J* = 6.4 Hz, 1H), 7.52 (p, *J* = 7.2 Hz, 1H), 7.28-7.12 (m, 4H), 5.57 (s, 2H), 4.52-4.47 (m, 1H), 4.27-4.20 (m, 1H), 4.09 (s, 1H), 3.85 (q, *J*= 10.4 Hz, 1H), 3.09-2.97 (m, 4H), 2.22-2.13 (m, 1H), 2.09-1.97 (m, 1H).

**Compound III-26a:**

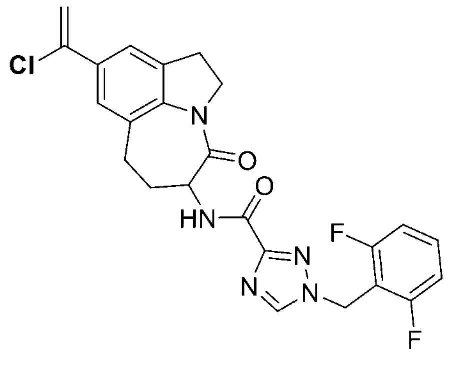

MS(m/z): 484.1 $[M+H]^+$.

$^1$H NMR (400 MHz, DMSO-$d_6$) δ 8.85 (s, 1H), 8.59 (d, *J* = 6.8 Hz, 1H), 7.55-7.48 (m, 1H), 7.45 (d, *J* = 2.0 Hz, 1H), 7.38 (d, *J* = 2.0 Hz, 1H), 7.18 (t, *J* = 8.1 Hz, 2H), 6.00 (d, *J* = 2.4 Hz, 1H), 5.57 (s, 2H), 5.53 (d, *J* = 2.4 Hz, 1H), 4.53-4.49 (m, 1H), 4.29-4.22 (m, 1H), 3.92-3.84 (m, 1H), 3.15-3.00 (m, 4H), 2.22-2.16 (m, 1H), 2.10-2.00 (m, 1H).

**Compound III-26b:**

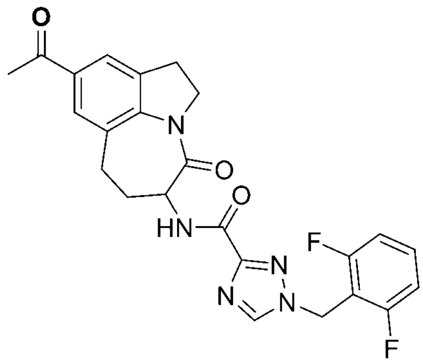

MS(m/z): 466.1 $[M+H]^+$.

$^1H$ NMR (400 MHz, DMSO-$d_6$) δ 8.85 (s, 1H), 8.62 (d, *J*= 6.8 Hz, 1H), 7.71 (s, 2H), 7.52 (p, *J* = 7.2 Hz, 1H), 7.19 (t, *J*= 8.0 Hz, 2H), 5.58 (s, 2H), 4.62-4.51 (m, 1H), 4.29-4.23 (m, 1H), 3.92 (q, *J*= 10.4 Hz, 1H), 3.22-3.03 (m, 4H), 2.53 (s, 3H), 2.26-2.17 (m, 1H), 2.13-2.05 (m, 1H).

**Example 44:** Preparation of **Target Compound** 1-(2,6-difluorobenzyl)-*N*-(9-((6,7-dihydro-4*H*-pyrazolo[5,1-c] [1,4]oxazin-3-yl)ethynyl)-4-oxo-1,2,3,4,6,7-hexahydroazepino[3,2,1-*hi*]indol-3-yl)-1*H*-1,2,4-triazole-3-carboxamide (**III-27)**

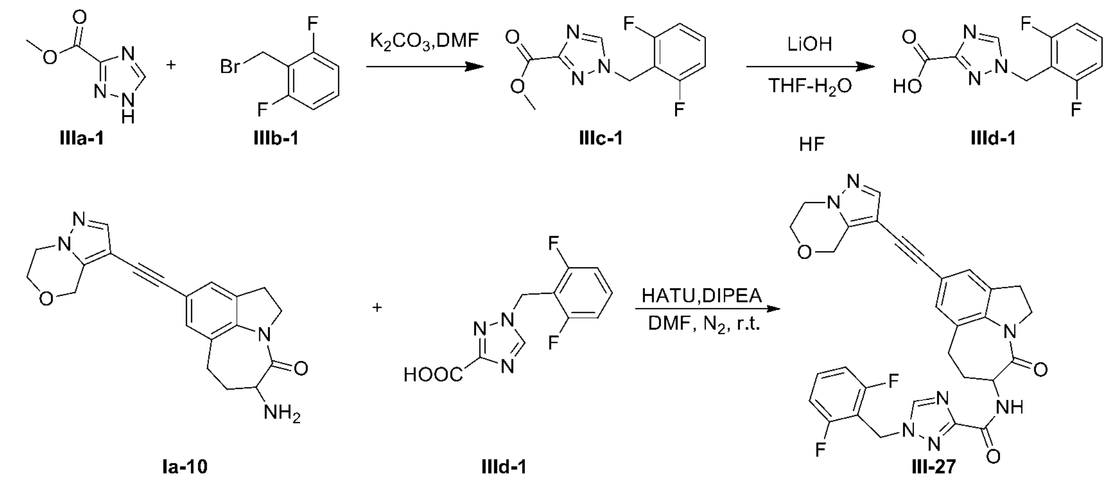


**IIIa-1** **IIIb-1** **IIIc-1** **IIId-1**

**Ia-10** **IIId-1** **III-27**

**Step 1: Preparation of compound Ia-10**

Intermediate **A** was obtained with reference to the "method for preparing common intermediate A".

Compound **Ia-10** (85 mg, yield: 22.4%) was obtained with reference to the "method for preparing **Ia-1**" in Example 1, wherein the intermediate "ethynylcyclopropane" was replaced with "3-ethynyl-6,7-dihydro-4*H*-pyrazolo[5,1-*c*][1,4] oxazine" while the remaining procedures followed the "method for preparing **Ia-1**" in Example 1. MS(m/z): 349.2 $[M+H]^+$.

**Step 2: Preparation of target compound III-27**

Target compound **III-27** (3 mg, yield: 3.14%) was obtained with reference to the synthesis method in step 3 in Example 18, wherein only the intermediate **Ia-5** was replaced with **Ia-10** while the remaining procedures were the same.

MS(m/z): 570.1 $[M+H]^+$.

$^1H$ NMR (400 MHz, DMSO-$d_6$) δ 8.86 (s, 1H), 8.59 (d, *J*= 6.4 Hz, 1H), 7.70 (s, 1H), 7.56-7.49 (m, 1H), 7.27-7.15 (m, 4H), 5.58 (s, 2H), 4.89 (s, 2H), 4.53-4.48 (m, 1H), 4.28-4.22 (m, 1H), 4.15-4.12 (m, 2H), 4.09-4.07 (m, 2H), 3.86 (q, *J*= 10.4 Hz, 1H), 3.11-2.99 (m, 4H), 2.23-2.15 (m, 1H), 2.10-2.00 (m, 1H).

**Example 45:** Preparation of **Target Compound** 1-(2,6-difluorobenzyl)-*N*-(9-((5-formyl-1-vinyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-28)**

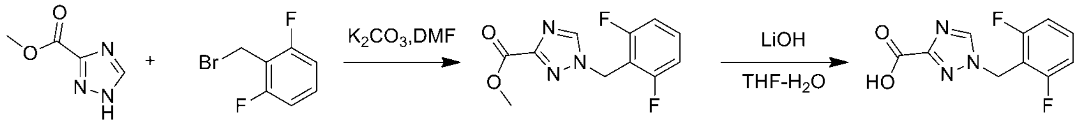


**IIIa-1** **IIIb-1** **IIIc-1** HF **IIId-1**

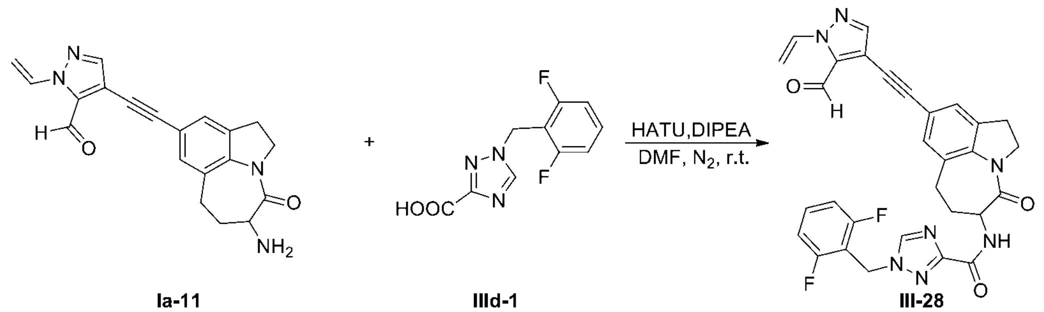


**Ia-11** **IIId-1** **III-28**

**Step 1: Preparation of compound Ia-11**

Intermediate A was obtained with reference to the "method for preparing common intermediate A".

Compound **Ia-11** (185 mg, yield: 33.7%) was obtained with reference to the "method for preparing **Ia-1**" in Example 1, wherein the intermediate "ethynylcyclopropane" was replaced with "4-ethynyl-1-vinyl-1H-pyrazole-5-carbaldehyde" while the remaining procedures followed the "method for preparing **Ia-1"** in Example 1. MS(m/z): 347.2 $[M+H]^+$.

**Step 2: Preparation of target compound III-28**

Target compound **III-28** (20 mg, yield: 13.34%) was obtained with reference to the synthesis method in step 3 in Example 18, wherein only the intermediate **Ia-5** was replaced with **Ia-11** while the remaining procedures were the same.

MS(m/z): 568.2 $[M+H]^+$.

$^1H$ NMR (400 MHz, DMSO-$d_6$) $\delta$ 8.86 (s, 1H), 8.60 (d, *J*= 6.8 Hz, 1H), 7.86 (s, 1H), 7.55-7.50 (m, 1H), 7.40-7.33 (m, 1H), 7.31 (d, *J* = 1.6 Hz, 1H), 7.26 (d, *J*= 1.6 Hz, 1H), 7.23-7.16 (m, 2H), 5.69 (d, *J*= 15.6 Hz, 1H), 5.63 (s, 1H), 5.58 (s, 2H), 4.98 (d, *J* = 8.8 Hz, 1H), 4.54-4.49 (m, 1H), 4.29-4.23 (m, 1H), 3.91-3.84 (m, 1H), 3.14-3.00 (m, 4H), 2.25-2.16 (m, 1H), 2.11-2.01 (m, 1H).

**Example 46:** Preparation of **Target Compound** 1-(2,6-difluorobenzyl)-*N*-(9-((1-(oxetan-3-yl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-29)**

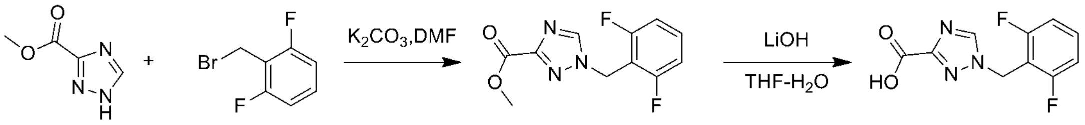


IIIa-1 IIIb-1 IIIc-1 HF IIId-1

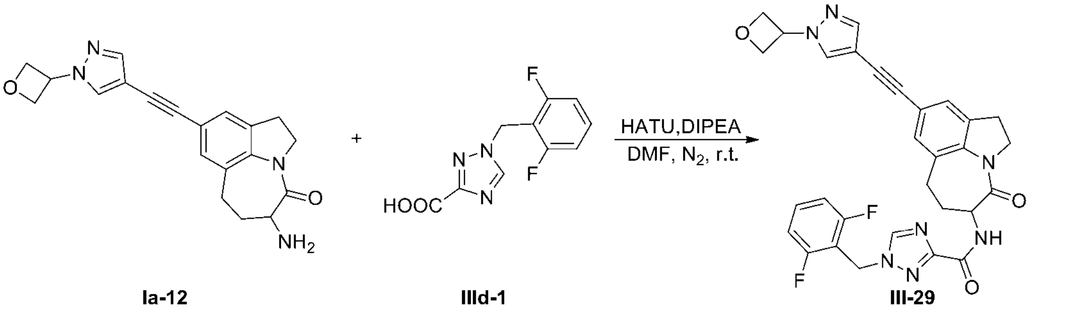


Ia-12 IIId-1 III-29

**Step 1: Preparation of compound Ia-12**

Intermediate **A** was obtained with reference to the "method for preparing common intermediate A".
Compound **Ia-12** (122 mg, yield: 25.7%) was obtained with reference to the "method for preparing **Ia-1**" in Example 1, wherein the intermediate "ethynylcyclopropane" was replaced with "4-ethynyl-1-(oxetan-3-yl)-1*H*-pyrazole" while the remaining procedures followed the "method for preparing **Ia-1**" in Example 1.
MS(m/z): 349.2 $[M+H]^+$.

**Step 2: Preparation of target compound III-29**

Target compound **III-29** (9 mg, yield: 6.34%) was obtained with reference to the synthesis method in step 3 in Example 18, wherein only the intermediate **Ia-5** was replaced with **Ia-12** while the remaining procedures were the same.
MS(m/z): 570.2 $[M+H]^+$.
$^1$H NMR (400 MHz, DMSO-$d_6$) δ 8.85 (s, 1H), 8.59 (d, *J* = 6.4 Hz, 1H), 8.25 (s, 1H), 7.82 (s, 1H), 7.56-7.48 (m, 1H), 7.23-7.14 (m, 4H), 5.62-5.50 (m, 3H), 4.94-4.86 (m, 4H), 4.52-4.48 (m, 1H), 4.28-4.21 (m, 1H), 3.86 (q, *J*= 10.4 Hz, 1H), 3.14-2.97 (m, 4H), 2.23-2.15 (m, 1H), 2.10-1.99 (m, 1H).

**Example 47:** Preparation of **Target Compound** *N*-(9-((1-cyclobutyl-1*H*-pyrazol-4-yl)ethynyl)-4-oxo-1,2,3,4,6,7-hexahydroazepino[3,2,1-*hi*]indol-3-yl)-1-(2,6-difluorobenzyl)-1*H*-1,2,4-triazole-3-carboxamide **(III-30)**

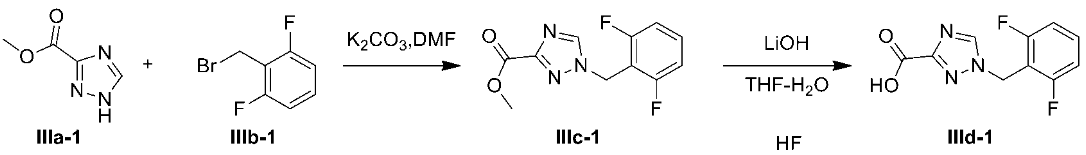


IIIa-1 IIIb-1 IIIc-1 HF IIId-1

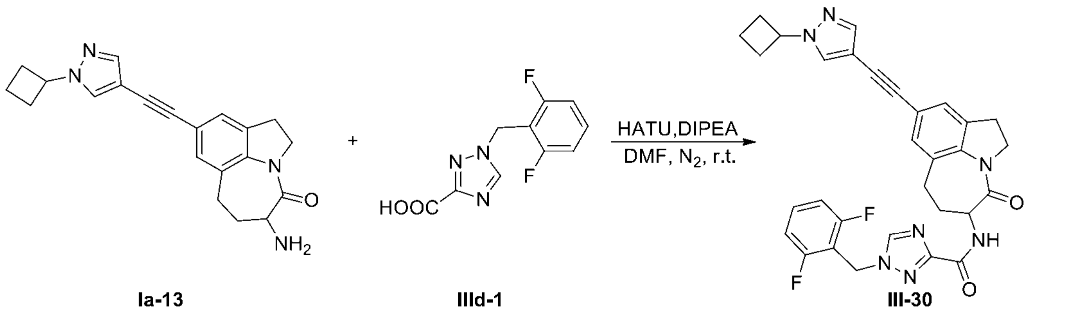


Ia-13 IIId-1 III-30

**Step** 1: **Preparation of compound Ia-13**

Intermediate **A** was obtained with reference to the "method for preparing common intermediate A".

Compound **Ia-13** (122 mg, yield: 27.5%) was obtained with reference to the "method for preparing **Ia-1"** in Example 1, wherein the intermediate "ethynylcyclopropane" was replaced with "4-ethynyl-1-(cyclobutan-3-yl)-1*H*-pyrazole" while the remaining procedures followed the "method for preparing **Ia-1**" in Example 1.
MS(m/z): 347.1 [M+H]+.

**Step 2: Preparation of target compound III-30**

Target compound **III-30** (9 mg, yield: 6.41%) was obtained with reference to the synthesis method in step 3 in Example 18, wherein only the intermediate **Ia-5** was replaced with **Ia-13** while the remaining procedures were the same.
MS(m/z): 568.2 [M+H]+.
$^1$H NMR (400 MHz, DMSO-$d_6$) δ 8.85 (s, 1H), 8.59 (d, *J* = 6.4 Hz, 1H), 8.18 (s, 1H), 7.69 (s, 1H), 7.56-7.48 (m, 1H), 7.24-7.15 (m, 4H), 5.58 (s, 2H), 4.83 (p, *J*= 8.4 Hz, 1H), 4.52-4.48 (m, 1H), 4.28-4.21 (m, 1H), 3.91-3.82 (m, 1H), 3.15 -2.98 (m, 4H), 2.48-2.33 (m, 4H), 2.23-2.14 (m, 1H), 2.09-2.02 (m, 1H), 1.83-1.74 (m, 2H).

**Example 48:** Preparation of **Target Compound** *N*-(9-((1-methyl-1*H*-pyrazol-4-yl)ethynyl)-4-oxo-1,2,3,4,6,7-hexahydroazepino[3,2,1-*hi*]indol-3-yl)-1-(pyridin-2-ylmethyl)-1*H*-1,2,4-triazole-3-carboxamide **(III-31)**

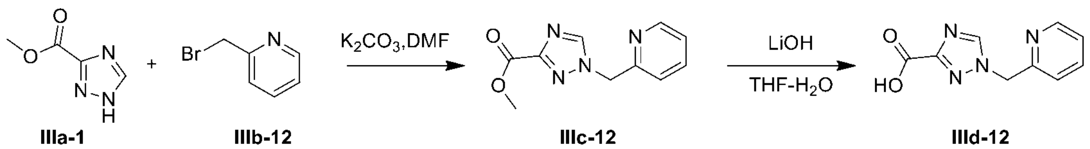


**IIIa-1** **IIIb-12** **IIIc-12** **IIId-12**

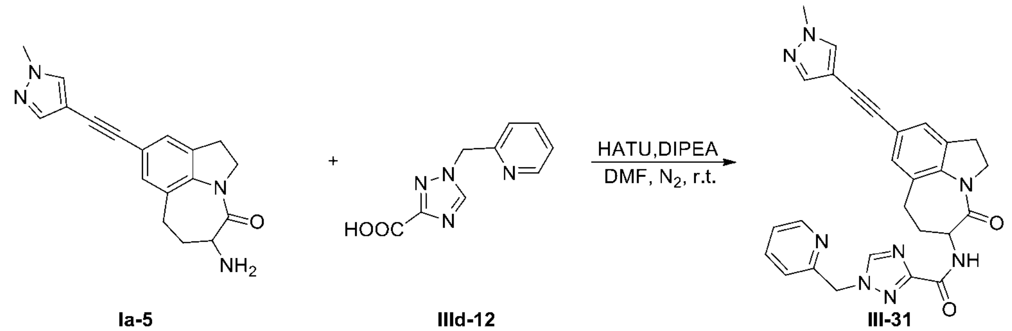


**Ia-5** **IIId-12** **III-31**

**Step 1: Preparation of compound IIId-12**

Compound **IIId-12** was obtained with reference to the "preparation of intermediate **IIId-1"** in Example 18, wherein only the starting material "2,6-difluorobenzyl bromide" was replaced with "2-(bromomethyl)pyridine" while the remaining procedures were the same.
MS(m/z): 205.1 [M+H]+.

**Step 2: Preparation of target compound III-31**

Target compound **III-31** (73 mg, yield: 19.52%) was obtained with reference to the synthesis method in step 3 in Example 18, wherein only the intermediate **IIId-1** was replaced with **IIId-12** while the remaining procedures were the same.
MS(m/z): 493.1 [M+H]+.
$^1$H NMR (400 MHz, DMSO-$d_6$) δ 8.85 (s, 1H), 8.63 (d, *J*= 6.8 Hz, 1H), 8.57-8.51 (m, 1H), 8.04 (s, 1H), 7.84 (td, *J*= 7.6, 1.6 Hz, 1H), 7.66 (s, 1H), 7.38-7.34 (m, 2H), 7.22 (d, *J* = 10.8 Hz, 2H), 5.63 (s, 2H), 4.57-4.50 (m, 1H), 4.29-4.23 (m, 1H), 3.91-3.83 (m, 4H), 3.13-3.00 (m, 4H), 2.23-2.19 (m, 1H), 2.12-2.00 (m, 1H).

**Example 49:** Preparation of **Target Compound** 1-benzyl-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide **(III-32)**

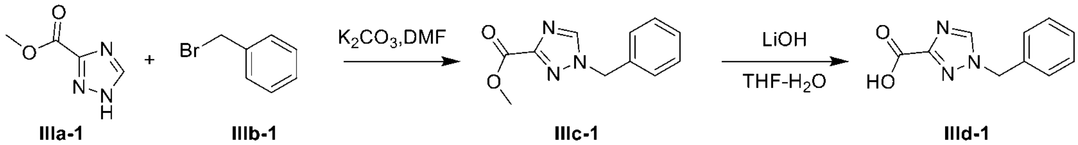


IIIa-1 IIIb-1 IIIc-1 IIId-1

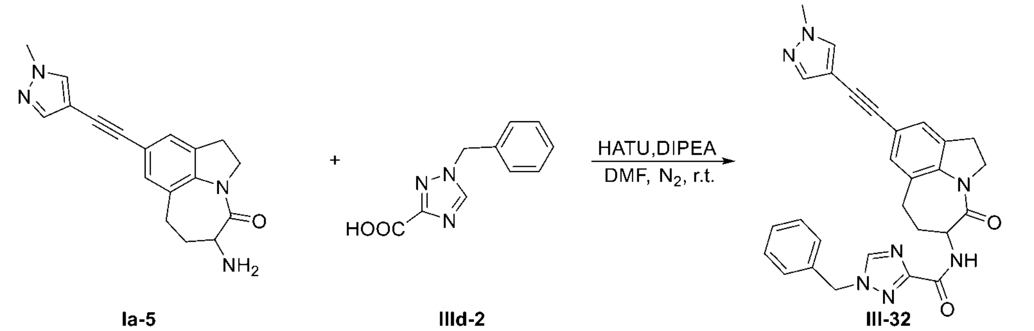


Ia-5 IIId-2 III-32

**Step** 1: **Preparation of compound IIId-2**

Compound **IIId-2** (811 mg, two-step total yield: 40.0%) was obtained with reference to the "method for preparing compound **IIId-1"** in Example 18, wherein only the starting material "2-(bromomethyl)-1,3-difluorobenzene" was replaced with "benzyl bromide" while the remaining procedures were the same.
MS(m/z): 204.1 $[M+H]^+$.
$^1$H NMR (600 MHz, DMSO-$d_6$) δ 13.32 (s, 1H), 8.79 (s, 1H), 7.46-7.20 (m, 5H), 5.48 (s, 2H).

**Step 2: Preparation of target compound III-32**

Target compound **III-31** (80 mg, yield: 21.38%) was obtained with reference to the synthesis method in step 3 in Example 18, wherein only the intermediate **IIId-1** was replaced with **IIId-2** while the remaining procedures were the same.
MS(m/z): 492.1 $[M+H]^+$.
$^1$H NMR (600 MHz, DMSO-$d_6$) δ 8.84 (s, 1H), 8.61 (d, $J$ = 6.6 Hz, 1H), 8.03 (s, 1H), 7.65 (s, 1H), 7.42-7.30 (m, 5H), 7.22 (d, $J$ = 15.6 Hz, 2H), 5.50 (s, 2H), 4.53-4.50 (m, 1H), 4.27-4.10 (m, 1H), 3.87-3.83 (m, 4H), 3.13-2.97 (m, 4H), 2.25-2.18 (m, 1H), 2.09 -2.02 (m, 1H).
**Example 50:** Preparation of **Target Compound** 1-benzyl-*N*-(4-oxo-8-(pyridin-4-ylethynyl)-1,2,3,4,6,7-hexahydroazepino[3,2,1-*hi*]indol-3-yl)-1*H*-1,2,4-triazole-3-carboxamide **(IV-1)**
**The synthetic route of target compound IV-1 is as follows:**

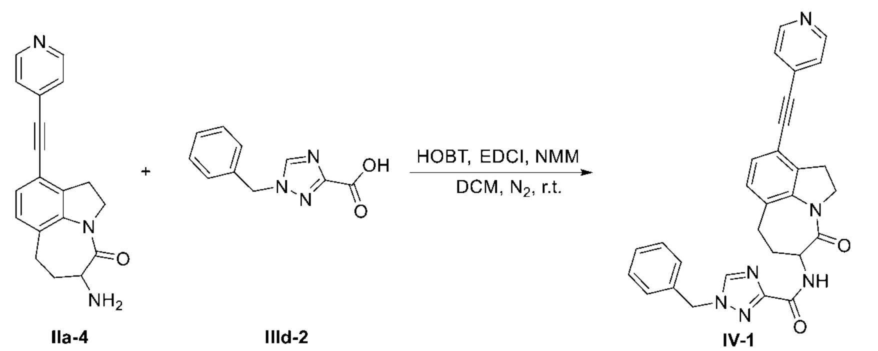


IIa-4 IIId-2 IV-1

Compound **IIa-4** was obtained with reference to the "preparation of compound **IIa-4"** in Example 17; compound **IIId-2** was obtained with reference to the "preparation of compound **IIId-2"** in Example 20.
In a 50 mL eggplant-shaped flask, intermediate **IIa-4** (40 mg, 0.13 mmol), HOBT (20 mg, 0.15 mmol), EDCI (28 mg, 0.15 mmol), and NMM (40 mg, 0.40 mmol) were dissolved in anhydrous DCM (2 mL), and the system was thoroughly purged with nitrogen. After stirring at room temperature for 10 min, **IIId-2** (41 mg, 0.20 mmol) dissolved in DCM (2 mL) was added under a nitrogen atmosphere, and the system was allowed to react overnight at room temperature. After thin layer chromatography showed that the reaction of the starting materials was substantially completed, 20 mL of water was added to the system, and the system was extracted with DCM for layer separation (20 mL × 3). The organic phases were combined, washed with a saturated sodium chloride solution, dried over anhydrous sodium sulfate, filtered, and

concentrated under reduced pressure. The residue was purified by preparative liquid chromatography to give the target compound **IV-1** in the form of a white solid (10 mg, yield: 15.3%).

MS(m/z): 489.2 $[M+H]^+$.

$^1$H NMR (400 MHz, Chloroform-*d*) δ 8.90 (s, 1H), 8.56-8.38 (m, 2H), 7.80-6.79 (m, 9H), 5.34 (s, 1H), 4.60-4.47 (m, 1H), 4.13- 3.91 (m, 2H), 3.32-2.97 (m, 2H), 2.40-2.02 (m, 2H), 1.98-1.88 (m, 2H), 1.82-1.76 (m, 2H).

**Example 51:** Preparation of **Target Compound** *N*-(9-((1-methyl-1*H*-pyrazol-4-yl)ethynyl)-4-oxo-1,2,3,4,6,7-hexahydroazepino[3,2,1-*hi*]indol-3-yl)-6-phenoxypicolinamide **(V-1)**

**The synthetic route of target compound V-1 is as follows:**

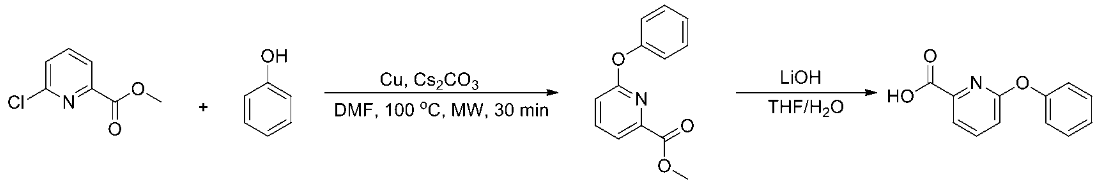


**Va-2** **Vb** **Vc-2** **Vd-2**

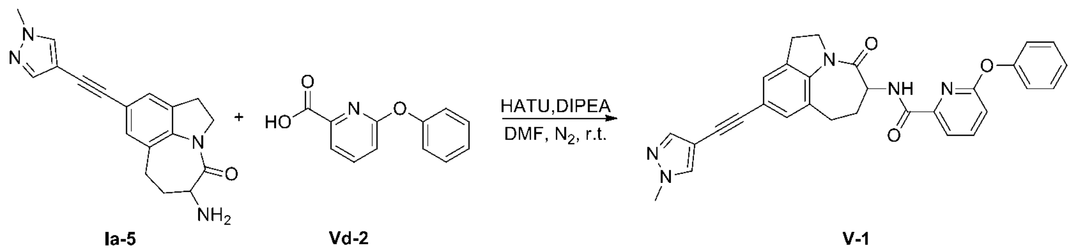


**Ia-5** **Vd-2** **V-1**

**Step 1: Preparation of compound Vd-2**

Compound Vd-2 (250 mg, two-step yield: 38.7%) was obtained with reference to the "preparation of intermediate **Vd-1"** in Example 36, wherein only the starting material "methyl 5-chloropicolinate" was replaced with "methyl 6-chloropicolinate" while the remaining procedures were the same.

MS(m/z): 489.2 $[M+H]^+$.

**Step 2: Preparation of target compound V-1**

Compound **Ia-5** was obtained with reference to the "preparation of compound **Ia-5"** in Example 5.

In a 50 mL eggplant-shaped flask, intermediate **Ia-5** (110 mg, 0.36 mmol), **Vd-2** (155 mg, 0.72 mmol), HATU (273 mg, 0.72 mmol), and DIPEA (233 mg, 1.80 mmol) were dissolved in anhydrous DMF (8 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **V-1** in the form of a white solid (18 mg, yield: 10.0%).

MS(m/z): 504.2 $[M+H]^+$.

$^1$H NMR (400 MHz, DMSO-$d_6$) δ 8.68 (d, *J*= 6.0 Hz, 1H), 8.12-7.99 (m, 2H), 7.81 (d, *J*= 7.2 Hz, 1H), 7.67 (s, 1H), 7.54-7.44 (m, 2H), 7.36-7.12 (m, 6H), 4.50-4.42 (m, 1H), 4.32-4.22 (m, 1H), 3.87-3.80 (m, 4H), 3.20-3.12 (m, 1H), 3.10-2.94 (m, 3H), 2.27-2.18 (m, 1H), 2.04-1.91 (m, 1H).

**Example 52:** Preparation of **Target Compound** *N*-(9-((1-methyl-1*H*-pyrazol-4-yl)ethynyl)-4-oxo-1,2,3,4,6,7-hexahydroazepino[3,2,1-*hi*]indol-3-yl)-5-phenoxypicolinamide **(V-2)**

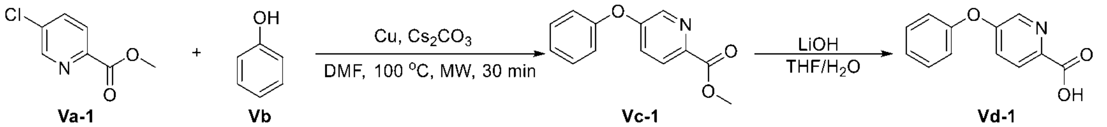


Va-1 Vb Vc-1 Vd-1

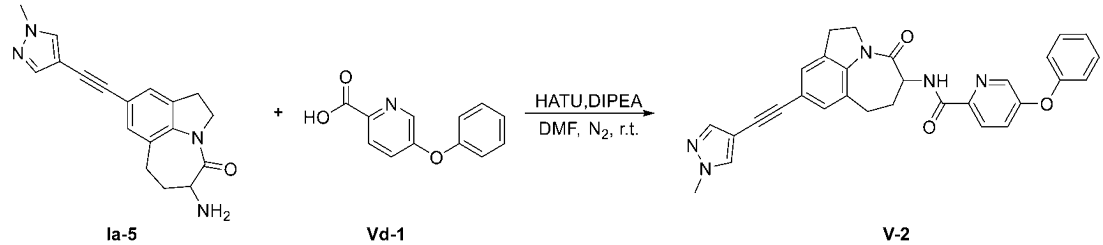


Ia-5 Vd-1 V-2

**Step 1: Synthesis of compound Vc-1**

In a microwave reaction flask, methyl compound **Va-1** (515 mg, 3 mmol), compound **Vb** (424 mg, 4.5 mmol), copper powder (19.2 mg, 0.3 mmol), and cesium carbonate (2.93 g, 9 mmol) were dissolved in 20 mL of anhydrous DMF solvent. The system was allowed to react at 100 ° C for 30 min under microwave conditions. The system was diluted with DCM, washed with a 10% sodium hydroxide solution, washed with water and saturated brine, dried over anhydrous sodium sulfate, and concentrated. The residue was purified by flash column to give **Vc-1** in the form of a white solid (140 mg, yield: 20.4%).

MS(m/z): 230.1 $[M+H]^+$.

**Step 2: Synthesis of compound Vd-1**

In a 100 mL eggplant-shaped reaction flask, intermediate **Vc-1** (140 mg, 0.61 mmol) was dissolved in 4 mL of anhydrous THF solution. Then, lithium hydroxide (30 mg, 1.22 mmol) was dissolved in 1 mL of water, and the solution was slowly added dropwise to the reaction system in an ice bath. After the dropwise addition was completed, the ice bath was removed, and the system was warmed to room temperature and allowed to react overnight. After thin layer chromatography showed that the reaction of the starting materials was substantially completed, THF was removed under reduced pressure, 2 mL of water was added, and then concentrated hydrochloric acid was added dropwise to adjust the pH to 1-2. A white solid was gradually precipitated, followed by slurring and filtration under reduced pressure. The filter residue was washed with water to give **Vd-1** in the form of a light white solid (113 mg, yield: 86.0%), which was directly used in the next step without purification.

MS (m/z): 216.1 $[M+H]^+$.

**Step 3: Preparation of target compound V-2**

Compound **Ia-5** was obtained with reference to the "preparation of compound **Ia-5"** in Example 5.

In a 50 mL eggplant-shaped flask, intermediate **Ia-5** (110 mg, 0.36 mmol), **Vd-1** (155 mg, 0.72 mmol), HATU (273 mg, 0.72 mmol), and DIPEA (233 mg, 1.80 mmol) were dissolved in anhydrous DMF (8 mL), and the system was thoroughly purged with nitrogen and allowed to react overnight at room temperature. After thin layer chromatography showed that the reaction of the starting materials was substantially completed, 20 mL of water was added to the system, and the system was extracted with ethyl acetate for layer separation (20 mL × 3). The organic phases were combined, washed with a saturated sodium chloride solution, dried over anhydrous sodium sulfate, filtered, and concentrated under reduced pressure. The residue was purified by preparative liquid chromatography to give the target compound **V-2** in the form of a white solid (10 mg, yield: 9.5%).

MS(m/z): 504.2 $[M+H]^+$.

$^1H$ NMR (400 MHz, DMSO-$d_6$) δ 9.04 (d, *J*= 6.4 Hz, 1H), 8.49 (d, *J*= 2.8 Hz, 1H), 8.14-7.96 (m, 2H), 7.67 (s, 1H), 7.56-7.41 (m, 3H), 7.35-7.12 (m, 5H), 4.59-4.52 (m, 1H), 4.32-4.26 (m, 1H), 3.92-3.84 (m, 4H), 3.21-2.96 (m, 4H), 2.31-2.23 (m, 1H), 2.14-2.02 (m, 1H).

**Example 53:** Preparation of **Target Compound** 1-(2,6-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-pyrazole-3-carboxamide **(XIII-1)**

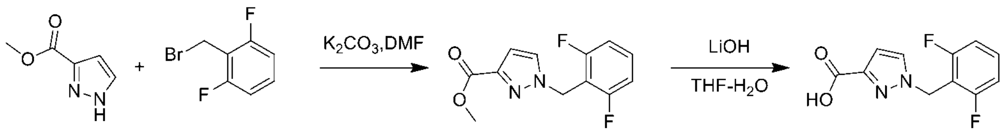


**IIIa-13** **IIIb-13** **IIIc-13** **IIId-13**

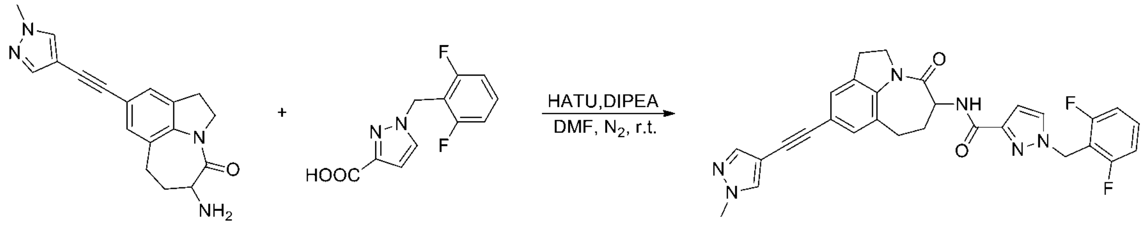


**Ia-5** **IIId-13** **XIII-1**

**Step 1: Preparation of compound IIId-13**

Compound **IIId-13** (530 mg, two-step total yield: 35.1%) was obtained with reference to the "preparation of intermediate **IIId-1"** in Example 18, wherein only the starting material "1,2,4-triazole-3-methyl ester " was replaced with "pyrazole-3-methyl ester" while the remaining procedures were the same.
MS(m/z): 239.1 $[M+H]^+$.

**Step 2: Preparation of target compound XIII-1**

Target compound **XIII-1** (45 mg, yield: 7.58%) was obtained with reference to the synthesis method in step 3 in Example 18, wherein only the intermediate **IIId-1** was replaced with **IIId-13** while the remaining procedures were the same.
MS(m/z): 527.2 $[M+H]^+$.
$^1$H NMR (400 MHz, DMSO-*d*$_6$) δ 8.16 (d, *J*= 6.4 Hz, 1H), 8.03 (s, 1H), 7.90 (d, *J*= 2.4 Hz, 1H), 7.66 (s, 1H), 7.50 (tt, *J* = 8.4, 6.8 Hz, 1H), 7.19 (q, *J* = 8.8, 8.0 Hz, 4H), 6.66 (d, *J* = 2.4 Hz, 1H), 5.49 (s, 2H), 4.52-4.43 (m, 1H), 4.30- 4.20 (m, 1H), 3.85 (s, 4H), 3.15-2.95 (m, 4H), 2.18 (dd, *J* = 13.6, 2.0 Hz, 1H), 2.07-1.96 (m, 1H).
**Example 54:** Preparation of **Control Compound** (*S*)-5-benzyl-*N*-(5-methyl-4-oxo-2,3,4,5-tetrahydrobenzo[*b*][1,4]oxazepin-3-yl)-4*H*-1,2,4-triazole-3-carboxamide **(GSK2982772):**

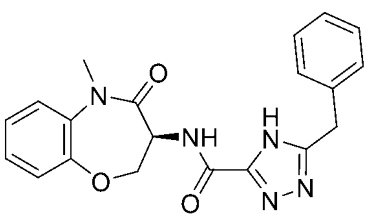

**For the specific synthetic route, see Patent No. CN201580044931.**

MS(m/z): 378.1 $[M+H]^+$.
$^1$H NMR (400 MHz, Chloroform-d) δ 8.11 (d, *J* = 7.2 Hz, 1H), 7.44-7.04 (m, 10H), 5.04 (dt, *J*= 11.2, 7.2 Hz, 1H), 4.66 (dd, *J*= 9.6, 7.2 Hz, 1H), 4.26 (t, *J*= 10.8 Hz, 1H), 4.12 (d, *J*= 3.0 Hz, 2H), 3.41 (s, 3H).

**Example 55:** Preparation of **Control Compound** 1-(2,6-difluorobenzyl)-*N*-(4-oxo-1,2,3,4,6,7-hexahydroazepino[3,2,1-*hi*]indol-3-yl)-1*H*-1,2,4-triazole-3-carboxamide (compound of Example 13 in CN201910518248X):

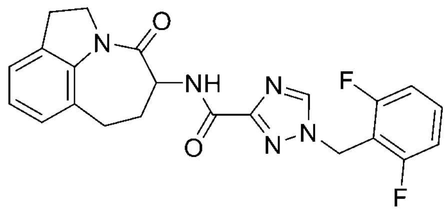

**For the specific synthetic route, see Patent No. CN201910518248X.**

MS(m/z): 424.2 $[M+H]^+$.

$^1H$ NMR (400 MHz, DMSO-$d_6$) δ 8.85 (s, 1H), 8.56 (d, *J* = 6.4 Hz, 1H), 7.52 (p, *J* = 7.2 Hz, 1H), 7.19 (t, *J*= 8.0 Hz, 2H), 7.13 (d, *J*= 7.2 Hz, 1H), 7.05 (d, *J* = 7.6 Hz, 1H), 6.98 (t, *J* = 7.2 Hz, 1H), 5.57 (s, 2H), 4.46 (t, *J*= 8.4 Hz, 1H), 4.26 (dt, *J*= 10.8, 6.8 Hz, 1H), 3.82 (q, *J*= 10.4 Hz, 1H), 3.15-2.95 (m, 4H), 2.25-2.16 (m, 1H), 2.10-1.98 (m, 1H).

**Example 56: Inhibition of TNF-α-Induced Necroptosis of U937 Cells by Compounds**

1. Experimental reagents: RPMI-1640 culture medium, fetal bovine serum, penicillin-streptomycin, TNF-α (MCE, HY-P7058), BV6 (MCE, HY-16701), Z-VAD-FMK (MCE, HY-16658B), and CCK-8 (Dalian Meilun Biotech Co., Ltd., MA0218).
2. Experimental instruments: medical low-temperature freezer (Thermo Fisher scientific, 902-ULTS), electrically heated thermostatic water bath (Shanghai Bluepard Instruments Co., Ltd., CD-420), low-speed centrifuge (ANHUI USTC Zonkia Scientific Instruments Co., Ltd, SC-3616), carbon dioxide incubator (Shanghai Bluepard Instruments Co., Ltd., BPN-150CH(UV)), inverted microscope (Olympus, CKX53), multi-functional microplate reader (Thermo Tecan, SPARK), class-II biosafety cabinet (AIRTECH, BSC-1304IIA2), and desktop cell counter (Thermo Fisher Scientific, Countess II).
3. Cell culturing: U937 cells (human histiocytic lymphoma cells) were cultured in an RPMI-1640 culture solution containing 10% fetal bovine serum and 1% penicillin-streptomycin solution in a 5% $CO_2$ incubator at 37 °C and a saturated humidity. The cell culture flask was vertically placed in the incubator. A T25 culture flask was used as an example, the culture solution in the flask was 6-8 mL, and culture medium changes and cell passaging were performed 3-4 times a week.
4. Experimental method: CCK-8 assay was used to assess the inhibitory effect of compounds on TNF-α-induced necroptosis of U937 cells to screen for compounds exhibiting inhibitory activity against receptor-interacting protein kinase 1.

4.1 Cell plating:

(1) The water bath was turned on and its temperature was adjusted to 37 °C. The culture medium was taken out from the freezer and transferred to the water bath for pre-heating. An appropriate amount of culture medium was then taken for use.
(2) The cell suspension was transferred to a 15 mL sterile centrifuge tube and centrifuged at 850 rpm for 4 min to collect U937 cells.
(3) The supernatant was carefully discarded, and an appropriate amount of culture medium was added. The mixture was gently pipetted using a pipette to homogenize the cells and thus prepare a single-cell suspension, and the cells were counted.
(4) The cells were diluted to $4 \times 10^5$/mL and seeded into a 96-well plate at 90 μL/well, such that the cell density in the 96-well plate was $3.6 \times 10^4$ cells/well. The cells were incubated in a 5% $CO_2$ incubator at 37 °C and a saturated humidity for 6 h, followed by the addition of compounds.

4.2 Dilution and addition of compounds:

(1) TNF-α was dissolved in PBS to obtain a 100 μg/mL stock solution and aliquoted; Z-VAD-FMK was dissolved in DMSO to obtain a 10 mM stock solution and aliquoted; BV6 was dissolved in DMSO to obtain a 10 mM stock solution and aliquoted; GSK2982772 was dissolved in DMSO to obtain a 10 mM stock solution and aliquoted. One aliquot of each stock solution was stored at -20 °C for immediate use, while the remaining aliquots were stored at -80 °C and used within their shelf life.
(2) RPMI-1640 culture medium containing TNF-α, BV6, and Z-VAD-FMK was prepared (5 μL/per well), such that

the final concentrations of TNF-α, BV6, and Z-VAD-FMK were 20 ng/mL, 2 μM, and 20 μM, respectively.

(3) Each target compound was three-fold diluted with a culture medium to 8 concentration gradients, with each concentration set in duplicate (5 μL/well). The final concentrations of each target compound were 1000.00/333.33/111.11/37.04/12.35/4.12/1.37/0.46 nM.

(4) The 96-well plate containing 90 μL of cell suspension was taken out from an incubator, and a positive control group, test compound groups, and a DMSO (5‰) control group were set. 5 μL of RPMI-1640 culture solution containing TNF-α, BV6, and Z-VAD-FMK and 5 μL of culture solution containing the test compound were added to the positive control group and the test compound groups. After compound addition, the mixtures were cultured for 18 h, followed by the CCK-8 assay.

(5) The 96-well plate was taken out, and the CCK-8 reagent was carefully added at 10 μL/well. The mixtures were incubated at 37 °C for 2-4 h, and the absorbance value was measured at a wavelength of 450 nm.

5. Evaluation of inhibitory activity of compounds on TNF-α-induced necroptosis of U937

Cell viability% = (OD value of treatment group - OD value of blank background)/(OD value of DMSO group - OD value of blank background) × 100

$EC_{50}$ values of the compounds were calculated using Graphpad prism 6 software, and the test results of the example compounds are shown in Table 1.

**Example 57: Inhibition of TNF-α-Induced Necroptosis of HT-29 Cells by Compounds**

1. Experimental reagents: McCOY's 5A culture medium, fetal bovine serum, penicillin-streptomycin, trypsin, PBS, TNF-α (MCE, HY-P7058), BV6 (MCE, HY-16701), Z-VAD-FMK (MCE, HY-16658B), SRB kit (Shanghai BestBio Biotechnology Co., Ltd., BB-4208-500T).

2. Experimental instruments: medical low-temperature freezer (Thermo Fisher scientific, 902-ULTS), electrically heated thermostatic water bath (Shanghai Bluepard Instruments Co., Ltd., CD-420), low-speed centrifuge (ANHUI USTC Zonkia Scientific Instruments Co., Ltd, SC-3616), carbon dioxide incubator (Shanghai Bluepard Instruments Co., Ltd., BPN-150CH(UV)), inverted microscope (Olympus, CKX53), multi-functional microplate reader (Thermo Tecan, SPARK), class-II biosafety cabinet (AIRTECH, BSC-1304IIA2), and desktop cell counter (Thermo Fisher Scientific, Countess II).

3. Cell culturing: HT-29 cells (human colon cancer cells) were cultured in a McCOY's 5A culture solution containing 10% fetal bovine serum and 1% penicillin-streptomycin solution in a 5% $CO_2$ at 37 °C and a saturated humidity, and culture medium changes and cell passaging were performed 3-4 times a week.

4. Experimental method: The SRB method was used to assess the inhibitory effect of compounds on TNF-α-induced necroptosis of HT-29 cells to screen for compounds exhibiting inhibitory activity against receptor-interacting protein kinase 1.

4.1 Cell plating:

(1) The water bath was turned on and its temperature was adjusted to 37 °C. The culture medium, trypsin, etc., were taken out from the freezer and transferred to the water bath for pre-heating. An appropriate amount of these substances was then taken for use.

(2) The old culture medium in the flask was aspirated using a sterile pipette and discarded, and the cells were washed 3 times with PBS. 1 mL of trypsin (25 $cm^2$ cell culture flask) was added into the flask, and the flask was gently shaken to allow the trypsin to cover all the cell surfaces. Digestion was performed in an incubator at 37 °C. After 2 min of digestion, the flask was placed under a microscope for observation. After it was found that the cytoplasm retracted, the cell gap increased, and the cells became round, the flask was immediately allowed to stand upright, and 4 mL of culture solution containing serum was added to terminate the digestion.

(3) The cell suspension was transferred to a 15 mL sterile centrifuge tube and centrifuged at 1000 rpm for 4 min to collect HT-29 cells.

(4) The supernatant was carefully discarded, and an appropriate amount of culture medium was added. The mixture was gently pipetted using a pipette to homogenize the cells and thus prepare a single-cell suspension, and the cells were counted.

(5) The cells were diluted to $3 \times 10^5$/mL and seeded into a 96-well plate at 100 μL/well, such that there were $3 \times 10^4$ cells per well in the 96-well plate. The cells were incubated in a 5% $CO_2$ incubator at 37 °C and a saturated

humidity for 24 h, followed by the addition of compounds.

4.2 Dilution and addition of compounds:

(1) TNF-α was dissolved in PBS to obtain a 100 μg/mL stock solution and aliquoted; Z-VAD-FMK was dissolved in DMSO to obtain a 10 mM stock solution and aliquoted; BV6 was dissolved in DMSO to obtain a 10 mM stock solution and aliquoted; GSK2982772 was dissolved in DMSO to obtain a 10 mM stock solution and aliquoted. One aliquot of each stock solution was stored at -20 °C for immediate use, while the remaining aliquots were stored at -80 °C and used within their shelf life.
(2) McCOY's 5A culture solution containing TNF-α, BV6, and Z-VAD-FMK was prepared. For the positive control group and the test compound groups, the McCOY's 5A culture solution was added at 90 μL/well, and 10 μL of the culture medium containing the test compound was also added, thus making the final concentrations of TNF-α, BV6, and Z-VAD-FMK be 20 ng/mL, 2 μM, and 20 μM, respectively. For the DMSO (5‰) control group, 90 μL of the McCOY's 5A complete culture solution was added, and then 10 μL of DMSO was added, thus making the final concentration 5‰.
(3) Each target compound was three-fold diluted with a culture medium to 8 concentration gradients, with each concentration set in duplicate (10 μL/well). The final concentrations of each target compound were 1000.00/333.33/111.11/37.04/12.35/4.12/1.37/0.46 nM.
(4) The 96-well plate cultured for 24 h was taken out from the incubator, and the culture medium in the 96-well plate was carefully aspirated and discarded. A positive control group, test compound groups, and a DMSO (5‰) control group were set, and 10 μL of the corresponding compound was added to each group. After compound addition, the mixtures were cultured for 18 h, followed by the SRB assay.
(5) The 96-well plate was taken out, and the absorbance was measured according to the use method for the SRB kit.

5. Evaluation of inhibitory activity of compounds on TNF-α-induced necroptosis of HT-29

Cell viability% = (OD value of treatment group - OD value of blank background)/(OD value of DMSO group - OD value of blank background) × 100

$EC_{50}$ values of the compounds were calculated using Graphpad prism 6 software, and the test results of the example compounds are shown in Table 1.

Table 1. Inhibitory activity of compounds against HT-29 and U937 cells

| Compounds ID | CELL | |
|---|---|---|
| | $EC_{50}$(nM) on HT-29 | $EC_{50}$(nM) on U937 |
| GSK2982772 | ++++ | +++ |
| I-1 | ++ | + |
| I-2 | +++ | ++ |
| I-3 | +++ | ++ |
| I-4 | ++ | ++ |
| I-5 | ++ | +++ |
| I-6 | +++ | +++ |
| I-7 | + | ++ |
| I-8 | + | + |
| I-9 | + | + |
| I-10 | + | ++ |
| I-11 | ++ | ++ |
| I-12 | +++ | +++ |
| I-13 | + | + |

(continued)

| Compounds ID | CELL | |
|---|---|---|
| | $EC_{50}$(nM) on HT-29 | $EC_{50}$(nM) on U937 |
| II-1 | +++ | +++ |
| II-2 | +++ | ++ |
| II-3 | ++ | + |
| II-4 | ++ | +++ |
| III-1 | +++++ | +++++ |
| III-2 | +++ | +++++ |
| III-3 | +++ | +++ |
| III-4 | +++++ | +++++ |
| III-5 | +++++ | +++++ |
| III-6 | ++++ | +++ |
| III-7 | +++ | +++++ |
| III-8 | +++ | ++++ |
| III-9 | +++++ | +++++ |
| III-10 | +++++ | +++++ |
| III-11 | +++++ | +++++ |
| III-12 | +++ | ++++ |
| III-13 | ++++ | +++++ |
| III-14 | ++++ | +++++ |
| III-15 | +++++ | +++++ |
| III-16 | +++ | +++++ |
| III-17 | +++++ | +++++ |
| III-18 | ++++ | +++ |
| III-19 | +++++ | ++++ |
| III-20 | ++++ | ++++ |
| III-21 | +++++ | +++++ |
| III-22 | ++++ | +++ |
| III-23 | +++ | +++ |
| III-24 | +++ | ++++ |
| III-25 | ++++ | +++ |
| III-26 | +++++ | +++++ |
| III-27 | +++++ | +++++ |
| III-28 | +++++ | +++++ |
| III-29 | +++++ | +++++ |
| III-30 | +++++ | +++++ |
| III-31 | +++++ | ++++ |
| III-32 | +++++ | +++++ |
| V-1 | + | + |
| V-2 | + | + |

(continued)

| Compounds ID | CELL | |
|---|---|---|
| | $EC_{50}$(nM) on HT-29 | $EC_{50}$(nM) on U937 |
| XIII-1 | +++ | +++ |
| Note: ++++ ≤ 5 nM; 5 nM < ++++ ≤ 10 nM; 10 nM < +++ ≤ 100 nM; 100 nM < ++ ≤ 200 nM; + > 200 nM. | | |

**Example 58: Inhibition of hERG Ion Channel by Compounds**

1. Cell culturing

The hERG ion channel was stably expressed in HEK293 cells.

Materials: 0.25% trypsin; DPBS (calcium- and magnesium-free); DMEM/F-12 (10% FBS + P/S); cells in logarithmic growth phase;

Operation process:

(1) 0.25% trypsin, DPBS (calcium- and magnesium-free), and DMEM/F-12 (10% FBS + P/S) are placed in a 37 °C thermostatic water bath and pre-heated for use.
(2) The cells placed in a 37 °C/$CO_2$ thermostatic incubator were taken out, and the old culture solution in the culture dish was aspirated using a pipette tip and discarded.
(3) 1 mL of DPBS wash solution was pipetted using a pipette to wash the cells twice (note: the pipette tip was placed against the wall of the culture dish, and the solution was added slowly), then the culture dish was gently rotated on the table. The wash solution was aspirated and discarded to completely remove the fetal bovine serum.
(4) 180 μL of trypsin was added for digestion, and the culture dish was gently rotated on the table to ensure the trypsin covered the bottom of the culture dish. The cell culture dish was placed in a $CO_2$ incubator at 37 °C for digestion.
(5) The digested cells were taken out, and 1 mL of DMEM/F12 (10% FBS+ P/S) culture solution was pipetted using a pipette and added along the bottom of the culture dish with an appropriate force. The culture solution was slightly pipetted, and a 200 μL pipette was used to gently triturate the cells.
(6) A certain amount of cells were transferred into a new 35 mm culture dish, and the culture dish was shaken, such that the cells were evenly distributed.
(7) The culture dish lid was marked with the cell type, passage number, date, etc., and the culture dish was incubated in a 5% $CO_2$ incubator at 37 °C.

2. Preparation of test sample

The test sample was dissolved in dimethyl sulfoxide (DMSO) to prepare a 9 mM stock solution, which was stored in a -20 °C freezer. On the testing day, the stock solution was further dissolved in the extracellular fluid to achieve the required concentrations. The concentration for electrophysiological test was 10 μM.

Formula of intracellular and extracellular fluids for electrophysiological test

The extracellular fluid (mM) was: sodium chloride, 137; potassium chloride, 4; calcium chloride, 1.8; magnesium chloride, 1; HEPES, 10; glucose, 10. The pH was adjusted to 7.34 with sodium hydroxide. The extracellular fluid was stored at 2-8 °C.

The intracellular fluid (mM) was: potassium aspartate, 130; magnesium chloride, 5; ethylene glycol tetraacetic acid (EGTA), 5; *N*-2-hydroxyethylpiperazine-N'-2-ethanesulfonic acid (HEPES), 10. The pH was adjusted to 7.4 with potassium hydroxide. The intracellular fluid was aliquoted into 1 mL centrifuge tubes and stored in a -80 °C freezer.

3. Experimental instruments

| Instrument | Specification/model | Manufacturer |
|---|---|---|
| Patch clamp amplifier | PatchClamp PC-505B | Warner instrument |
| Digital-to-analog converter | Digidata 1440A | Axon instruments |
| Micromanipulator | MP-225 | SUTTER instrument |
| Inverted microscope | TL4 | Olympus |
| Glass micropipette puller | PC-10 | NARISHIGE |
| Micropipette glass capillary | B12024F | Wuhan Weitan Scientific Instruments Co., Ltd. |

4. Procedures of electrophysiological experiment

All the tests were performed at room temperature. The cells were transferred to a perfusion chamber and perfusion was carried out using the extracellular fluid. The intracellular fluid was thawed on the day of the experiment. The electrodes were produced by pulling with PC-10 (Narishige, Japan). Whole-cell patch-clamp recording was carried out. Noise was filtered at one fifth of the sampling frequency.

For administration of the test samples, perfusion was carried out using a perfusion system utilizing the gravity of one's own. During the initial recording, the peak current amplitude was observed for at least 1 min until it was stable. During this period, the CV% of all peak current amplitudes should be less than 10% to exclude up and down fluctuations of the initial current. The mean of the peak current amplitudes recorded for the last three times during the initial recording served as the current peak of the negative control. After the initial current was stable, the test samples were administered starting from a low concentration until three recorded peak currents were again stable or the peak currents were "constant" after and before continuous administration for 5 min. We defined the following two cases as "stable" or "constant": 1) if the absolute mean of the peak current for three consecutive sweeps exceeded 200 pA and the CV value was less than 10%, 2) or the mean of the peak current for three consecutive sweeps was between 200 pA and 50 pA and the CV value was less than 30%. The next test of higher concentration was then given.

The mean of the peak current of the last three sweeps of each concentration was taken as the peak current for that concentration for data analysis. If the stable state was not reached within 5 min, the peak current mean of the last three sweeps at that time was taken as the peak current for that concentration for data analysis. At the same time, the cells were discarded and no longer used for testing higher concentrations. For each concentration, the test was repeated using at least one additional independent cell.

5. Voltage pulse program

The cells were clamped at -80 mV, then depolarized to 40 mV with a 4 s lasting square wave, and hyperpolarized to -40 mV with a 2 s lasting square wave to obtain the hERG tail current. This procedure was repeated every 20 s.

6. Data processing and fitting

Data were analyzed using Excel 2003 (Microsoft Corporation) and OriginPro 8.

For each cell, the mean of its last three current peaks was taken as the current peak of the negative control during the monitoring period (before administration of the test drug). When each concentration was tested, the mean of the last three current peaks was similarly used as the current peak under the action of that concentration for data analysis and statistics. The blocking rate of each test concentration on hERG current was calculated using the following formula:

(1 - peak current recorded after compound perfusion/peak current recorded before compound perfusion) × 100% Percent inhibition of hERG current was averaged for all cells recorded at the same test concentration.

The half maximal inhibitory concentration $IC_{50}$ value was fitted using the Hill1 equation (OriginPro 8):

$$Y = START + (END - START) \times x^n / (k^n + x^n)$$

where Y = blocking rate of current after perfusion with the test substance; x = concentration of the test substance; k = half maximal inhibitory concentration $IC_{50}$; START = percentage of blocking recorded after perfusion at the initial concentration of the test substance; END = percentage of blocking recorded at the plateau phase of the test substance; n = number of different cooperative sites for the test substance on the ion channel.

Table 2. Blocking rate of compound for hERG ion channel

| Test of compound effect on hERG channel current | |
|---|---|
| Compound (10 μM) | Percentage blocking rate of hERG channel |
| III-17 | 38.89% |
| III-25 | 39.58% |

**Example 59: Pharmacokinetic Experiment of Compounds in SD Rats**

1. Experimental animals

SD rats, male, 6-8 weeks old, purchased from Zhejiang Vital River Laboratory Animal Technology Co., Ltd., production license No.: SCXK (Zhejiang) 2019-0001. The animals were maintained in an SPF-grade animal room of Hangzhou Bio-Sincerity Pharma-Tech Co., Ltd., with the experimental animal use license No. of SYXK (Zhejiang) 2021-0014. The animals were used after being acclimatized for at least one week. All experiments were performed in strict compliance with the regulations on experimental animals.

2. Experimental reagents and equipment

2.1 Experimental reagents: dimethyl sulfoxide (DMSO) was purchased from SIGMA-ALDRICH, ammonium acetate and polyoxyl-35 castor oil EL (Kolliphor EL) were purchased from Aladdin, normal saline (Saline) was purchased from Zhejiang Kancheer Pharmaceutical Co., Ltd., and methanol, acetonitrile, and formic acid were purchased from Merck.

2.2 Experimental materials: Centrifuge tubes of various specifications were purchased from Biosharp; pipettes of various specifications were purchased from Eppendorf.

2.3 Experimental equipment: Exion AD-SCIEX TRIPE QUADTM 5500 was purchased from AB SCIEX.

3. Experimental protocol

3.1 Method for preparing compound administration solutions:

3.1.1 Intravenous injection solution: GSK2982772, Example 13 in CN201910518248X, and compounds III-1, III-10, III-12, III-19, III-21, and III-26 were each formulated into a 0.2 mg/mL administration solution in a vehicle of 5% DMSO + 10% kolliphor EL + 85% saline.

3.1.2 Intragastric administration solution: GSK2982772, Example 13 in CN201910518248X, and compounds III-1, III-10, III-12, III-19, III-21, and III-26 were each formulated into a 0.5 mg/mL administration solution in a vehicle of 5% DMSO + 10% kolliphor EL + 85% saline.

3.2 Administration to SD rats

1) SD rats were fasted for at least 12 h with free access to water. The dose for intragastric administration was 5.0 mg/kg, and the administration volume was 10 mL/kg; the dose for intravenous injection was 0.5 mg/kg, and the administration volume was 2.5 mL/kg. Four hours after administration, food was provided.

4. Experimental process

After administration to SD rats, about 300 μL of blood was collected from the orbit of each animal and anticoagulated with EDTA-$Na_2$. The blood collection time points of the intravenous injection group were: 2 min, 10 min, 30 min, 1 h, 2 h, 4 h, 6 h, 8 h, and 24 h after administration of the test substance; the blood collection time points of the intragastric administration group were: 5 min, 15 min, 30 min, 1 h, 2 h, 4 h, 6 h, 8 h, and 24 h after administration of the test substance. The collected blood was placed in an ice bath and centrifuged within 1 h to separate plasma (centrifugation conditions: 2000× g, 10 min, 4 °C). 50 μL of plasma sample was taken, subjected to protein precipitation, and then assayed by UHPLC-MS. The assay data were analyzed and processed using WinNonLin8.1 pharmacokinetic software to obtain pharmacokinetic parameters.

5. Experimental results

The pharmacokinetic parameters of the compounds of the present disclosure are shown in Table 2.

Conclusion: The compounds III-1, III-10, and III-12 of the present disclosure exhibited good oral exposure, high bioavailability, and good half-life after intragastric administration, and their exposure and bioavailability were significantly improved compared with Example 13 in CN201910518248X; after intravenous injection, the compounds III-1, III-10, and III-12 exhibited good half-life, good clearance, and relatively large steady-state apparent volume of distribution, suggesting that the compounds had greater tissue distribution in SD rats than GSK2982772 and Example 13 in CN201910518248X. In summary, compounds III-1, III-10, and III-12 have good pharmacokinetic characteristics.
NA: Not tested.

Table 2. Pharmacokinetic parameters of test compound

| Compound No. | Route/dose of administration | Pharmacokinetic parameters | | | | | | |
|---|---|---|---|---|---|---|---|---|
| | | Half life (h) | Peak concentration (ng/mL) | Area under the plasma concentration-time curve (h*ng/mL) | Clearance (mL/min/kg) | Steady-state apparent volume of distribution (L/kg) | Apparent clearance (mL/min/kg) | $F_{abs}$ (%) |
| | | $T_{1/2}$ | $C_{max}$ | $AUC_{last}$ | $Cl_{obs}$ | Vss | $Cl_{F\ obs}$ | Fabs |
| GSK2982772 | i.v, 0.5 mg/kg | 3.28 | 4324 | 5564.79 | 1.50 | 0.1 | NA | NA |
| | p.o, 5 mg/kg | 3.06 ±0.41 | 5821.33 ±1213.90 | 21736.56 ±6529.34 | NA | NA | 4.03 ± 1.05 | 39.13 ± 11.8 |
| Example 13 in CN20191051 8248X | i.v, 0.5 mg/kg | 0.44 ±0.26 | 982.93±105.90 | 270.78 ±37.96 | 30.96 ± 4.47 | 0.60 ± 0.13 | NA | NA |
| | p.o, 5 mg/kg | 0.34 ±0.14 | 466.93±214.94 | 340.18 ±173.82 | NA | NA | 8.37 ± 4.01 | 12.55 ± 6.41 |
| Compound III-1 | i.v, 0.5 mg/kg | 2.38 ±1.76 | 1428±181.08 | 1983.47 ±203.72 | 5.54 ± 2.45 | 0.67 ± 0.10 | NA | NA |
| | p.o, 5 mg/kg | 2.94±0.16 | 4098±519.77 | 22596.98 ±2369.18 | NA | NA | 3.70 ± 0.39 | 111.92 ± 11.87 |
| Compound III-10 | i.v, 0.5 mg/kg | 2.37± 0.64 | 1944.33 ± 319.33 | 5338.17 ± 1331.02 | 1.60 ± 0.39 | 0.32 ± 0.04 | NA | NA |
| | p.o, 5 mg/kg | 2.57 ± 0.69 | 4663.33 ± 131.94 | 27664.75 ±1556.46 | NA | NA | 3.01 ± 0.18 | 51.37 ± 3.03 |
| Compound III-12 | i.v, 0.5 mg/kg | 5.78 ± 2.23 | 1296.67 ± 23.21 | 8834.69 ± 1288.46 | 0.91 ± 0.18 | 0.38 ± 0.08 | NA | NA |
| | p.o, 5 mg/kg | 3.19 ± 0.52 | 7327.33 ± 780.61 | 60376.00 ± 9303.94 | NA | NA | 1.39 ± 0.20 | 64.54 ± 10.10 |
| Compound III-19 | i.v, 0.5 mg/kg | 1.37 ± 0.20 | 661.5 ± 109.99 | 702.72 ± 190.57 | 12.24 ± 3.04 | 1.31 ± 0.18 | NA | NA |
| | p.o, 5 mg/kg | 2.96 ± 0.69 | 1477.33 ± 544.85 | 8125.01 ± 3571.42 | NA | NA | 10.31 ± 3.12 | 121.33 ± 40.32 |
| Compound III-21 | i.v, 0.5 mg/kg | 1.19 ± 0.1 | 618.83 ± 65.8 | 651.25 ± 66.39 | 12.78 ± 1.27 | 1.1 ± 0.08 | NA | NA |
| | p.o, 5 mg/kg | 2.66 ± 0.69 | 1276 ± 508.56 | 6252.05 ± 2793.25 | NA | NA | 14.92 ± 6.92 | 97.15 ± 40.17 |

(continued)

| Compound No. | Route/dose of administration | Pharmacokinetic parameters | | | | | | |
|---|---|---|---|---|---|---|---|---|
| | | Half life (h) | Peak concentration (ng/mL) | Area under the plasma concentration-time curve (h*ng/mL) | Clearance (mL/min/kg) | Steady-state apparent volume of distribution (L/kg) | Apparent clearance (mL/min/kg) | $F_{abs}$ (%) |
| | | $T_{1/2}$ | $C_{max}$ | $AUC_{last}$ | $Cl_{obs}$ | Vss | $Cl_{F\ obs}$ | Fabs |
| Compound III-26 | i.v, 0.5 mg/kg | 0.44 ± 0.03 | 896.17 ± 158.43 | 550.2 ± 34.46 | 15.16 ± 0.98 | 0.46 ± 0.01 | NA | NA |
| | p.o, 5 mg/kg | 3.98 ± 2.49 | 1372.33 ± 389.43 | 2808 ± 176.53 | NA | NA | 29.62 ± 1.95 | 51.2 ± 3.29 |

## Claims

1. A benzazepinone compound, wherein the compound is a compound represented by formula I, an isomer, or a pharmaceutically acceptable salt thereof:

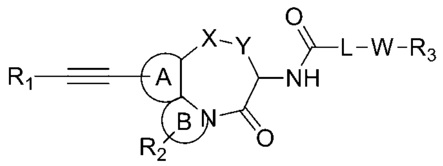

(I)

wherein: ring A is selected from a 6- to 10-membered aromatic ring and a 5- to 10-membered heteroaromatic ring, and ring A is substituted with at least one substituent

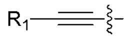

;

ring B is selected from 3- to 6-membered cycloalkyl and 3- to 6-membered heterocyclyl, and ring B is substituted with at least one substituent $R_2$;

X and Y are each independently selected from $CR_4$, $OR_4$, $SR_4$, and $NR_4$, and X and Y are not both $NR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_2$ is selected from hydrogen, halogen, $C_{1-6}$ alkyl, and $C_{1-6}$ haloalkyl;

L is selected from a 5- to 6-membered heteroaromatic ring substituted with at least one substituent selected from one or more of the following groups: hydrogen, halogen, $C_{1-6}$ alkyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;

W is selected from one of a linking bond, $-(CH_2)_n-$, -O-, $-(CH_2)_nO(CH_2)_m-$, -CO-, and $-(CH_2)_nCO(CH_2)_m-$;

$R_3$ is selected from a 6- to 10-membered aromatic ring and a 5- to 10-membered heteroaromatic ring substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;

$R_4$ is selected from hydrogen, halogen, $C_{1-6}$ alkyl, $C_{1-6}$ alkoxy, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;

n and m are each independently selected from 0, 1, 2, 3, and 4, and n and m are not both 0;

the 5- to 10-membered heteroaromatic ring, the 5- to 8-membered heteroaryl, and the 3- to 6-membered heterocyclyl contain at least one heteroatom selected from N, O, and S.

2. The benzazepinone compound according to claim 1, wherein the compound is a compound represented by formula 11, an isomer, or a pharmaceutically acceptable salt thereof:

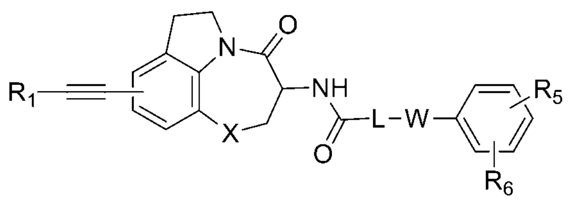

(II)

wherein: X is selected from $CR_4$, $OR_4$, and $SR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl

substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

L is selected from triazole, pyridine, pyrazole, and pyrazolotetrahydropyridone substituted with at least one substituent selected from one or more of the following groups: hydrogen, halogen, $C_{1-6}$ alkyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;

W is selected from one of $-(CH_2)_n-$, -O-, and -CO-;

$R_4$, $R_5$, and $R_6$ are each independently selected from hydrogen, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, and $C_{1-6}$ alkoxysulfinyl;

n is selected from 1, 2, 3, and 4;

the 5- to 10-membered heteroaromatic ring, the 5- to 8-membered heteroaryl, and the 3- to 6-membered heterocyclyl contain at least one heteroatom selected from N, O, and S.

**3.** The benzazepinone compound according to claim 2, wherein the compound is a compound represented by formula II-1, an isomer, or a pharmaceutically acceptable salt thereof:

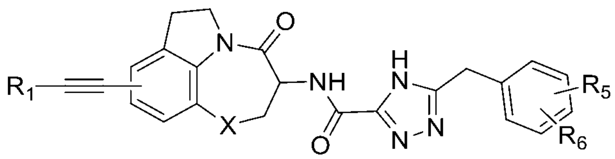

(II-1)

wherein: X is selected from $CR_4$, $OR_4$, and $SR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_4$, $R_5$, and $R_6$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

**4.** The benzazepinone compound according to claim 2, wherein the compound is a compound represented by formula II-2, an isomer, or a pharmaceutically acceptable salt thereof:

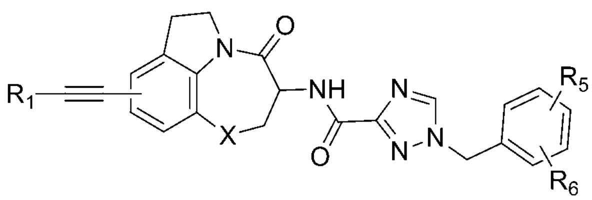

(II-2)

wherein: X is selected from $CR_4$, $OR_4$, and $SR_4$;

$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_4$, $R_5$, and $R_6$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

**5.** The benzazepinone compound according to claim 2, wherein the compound is a compound represented by formula II-3, an isomer, or a pharmaceutically acceptable salt thereof:

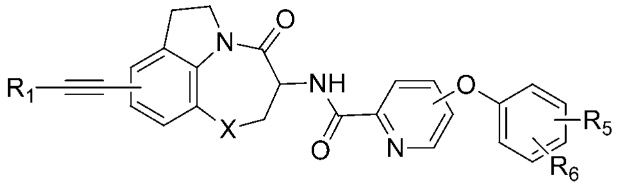
(II-3)

wherein: X is selected from $CR_4$, $OR_4$, and $SR_4$;
$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;
$R_4$, $R_5$, and $R_6$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

**6.** The benzazepinone compound according to claim 2, wherein the compound is a compound represented by formula II-4, an isomer, or a pharmaceutically acceptable salt thereof:

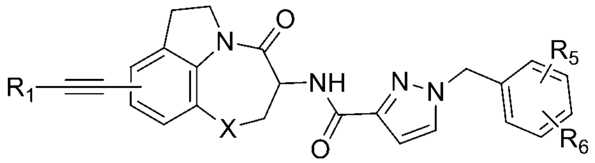
(II-4)

wherein: X is selected from $CR_4$, $OR_4$, and $SR_4$;
$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;
$R_4$, $R_5$, and $R_6$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

**7.** The benzazepinone compound according to claim 2, wherein the compound is a compound represented by formula II-5, an isomer, or a pharmaceutically acceptable salt thereof:

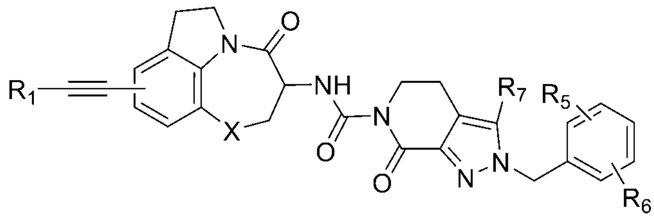
(II-5)

wherein: X is selected from $CR_4$, $OR_4$, and $SR_4$;
$R_1$ is selected from $C_{1-6}$ alkyl, $C_{1-6}$ alkoxy, $C_{3-6}$ cycloalkyl, $C_{3-6}$ heterocyclyl, $C_{6-10}$ aryl, and $C_{5-10}$ heteroaryl substituted with at least one substituent selected from one or more of the following groups: hydrogen, halogen, $C_{1-6}$ alkyl, $C_{2-6}$ alkenyl, $C_{2-6}$ alkynyl, $C_{1-6}$ alkoxy, amino, hydroxy, cyano, amido, sulfonyl, sulfinyl, $C_{1-6}$ haloalkyl, $C_{1-6}$ alkylhydroxy, $C_{1-6}$ alkylamino, $C_{1-6}$ alkylamido, $C_{1-6}$ alkylsulfonyl, $C_{1-6}$ alkylsulfinyl, $C_{1-6}$ haloalkoxy, $C_{1-6}$ alkoxyhydroxy, $C_{1-6}$ alkoxyamino, $C_{1-6}$ alkoxyamido, $C_{1-6}$ alkoxysulfonyl, $C_{1-6}$ alkoxysulfinyl, 3- to 6-membered cycloalkyl, 3- to 6-membered heterocyclyl, 5- to 8-membered aryl, and 5- to 8-membered heteroaryl;

$R_4$, $R_5$, $R_6$, and $R_7$ are each selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ alkylhydroxy, $C_{1-6}$ haloalkoxy, and $C_{1-6}$ alkoxyhydroxy.

**8.** A benzazepinone compound, wherein the compound is a compound having the following structure, an isomer, or a pharmaceutically acceptable salt thereof:

5-benzyl-*N*-(9-(cyclopropylethynyl)-4-oxo-1,2,3,4,6,7-hexahydroazepino[3,2,1-*hi*]indol-3-yl)-4*H*-1,2,4-triazole-3-carboxamide;
5-benzyl-*N*-(4-oxo-9-(pyridin-2-ylethynyl)-1,2,3,4,6,7-hexahydroazepino[3,2,1-*hi*]indol-3-yl)-4*H*-1,2,4-triazole-3-carboxamide;
5-benzyl-*N*-(4-oxo-9-(pyridin-3-ylethynyl)-1,2,3,4,6,7-hexahydroazepino[3,2,1-*hi*]indol-3-yl)-4*H*-1,2,4-triazole-3-carboxamide;
5-benzyl-*N*-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;
5-benzyl-*N*-(9-((1-methyl-1*H*-pyrazol-4-yl)ethynyl)-4-oxo-1,2,3,4,6,7-hexahydroazepino[3,2,1-*hi*]indol-3-yl)-4*H*-1,2,4-triazole-3-carboxamide;
5-benzyl-*N*-(4-oxo-9-((tetrahydro-2*H*-pyran-4-yl)ethynyl)-1,2,3,4,6,7-hexahydroazepino[3,2,1-*hi*]indol-3-yl)-4*H*-1,2,4-triazole-3-carboxamide;
5-benzyl-*N*-(9-(3-hydroxy-3-methylbut-1-yn-1-yl)-4-oxo-1,2,3,4,6,7-hexahydroazepino[3,2,1-*hi*]indol-3-yl)-4*H*-1,2,4-triazole-3-carboxamide;
5-(4-fluorobenzyl)-*N*-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;
5-(4-chlorobenzyl)-*N*-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;
5-(2-chlorobenzyl)-*N*-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;
5-(2-fluorobenzyl)-*N*-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;
5-(2,6-difluorobenzyl)-*N*-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;
5-(2,6-dichlorobenzyl)-*N*-(4-oxo-9-(pyridin-4-ylethynyl)-1,2,3,4,6,7-hexahydroazepino[3,2,1-*hi*]indol-3-yl)-4*H*-1,2,4-triazole-3-carboxamide;
5-benzyl-*N*-(4-oxo-8-(pyridin-2-ylethynyl)-1,2,3,4,6,7-hexahydroazepino[3,2,1-*hi*]indol-3-yl)-4*H*-1,2,4-triazole-3-carboxamide;
5-benzyl-*N*-(4-oxo-8-(pyridin-3-ylethynyl)-1,2,3,4,6,7-hexahydroazepino[3,2,1-*hi*]indol-3-yl)-4*H*-1,2,4-triazole-3-carboxamide;
5-benzyl-*N*-(8-(cyclopropylethynyl)-4-oxo-1,2,3,4,6,7-hexahydroazepino[3,2,1-*hi*]indol-3-yl)-4*H*-1,2,4-triazole-3-carboxamide;
5-benzyl-*N*-(4-oxo-8-(pyridin-4-ylethynyl)-1,2,3,4,6,7-hexahydroazepino[3,2,1-*hi*]indol-3-yl)-4*H*-1,2,4-triazole-3-carboxamide;
1-(2,6-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(2,6-difluorobenzyl)-*N*-(4-oxo-9-(pyridin-4-ylethynyl)-1,2,3,4,6,7-hexahydroazepino[3,2,1-*hi*]indol-3-yl)-1*H*-1,2,4-triazole-3-carboxamide;
1-benzyl-*N*-(4-oxo-9-(pyridin-4-ylethynyl)-1,2,3,4,6,7-hexahydroazepino[3,2,1-*hi*]indol-3-yl)-1*H*-1,2,4-triazole-3-carboxamide;
1-(2,6-difluorobenzyl)-*N*-(9-(3-hydroxy-3-methylbut-1-yn-1-yl)-4-oxo-1,2,3,4,6,7-hexahydroazepino[3,2,1-*hi*]indol-3-yl)-1*H*-1,2,4-triazole-3-carboxamide;
1-(2,6-difluorobenzyl)-*N*-(9-(3-methoxyprop-1-yn-1-yl)-4-oxo-1,2,3,4,6,7-hexahydroazepino[3,2,1-*hi*]indol-3-yl)-1*H*-1,2,4-triazole-3-carboxamide;
*N*-(9-(cyclopropylethynyl)-4-oxo-1,2,3,4,6,7-hexahydroazepino[3,2,1-*hi*]indol-3-yl)-1-(2,6-difluorobenzyl)-1*H*-1,2,4-triazole-3-carboxamide;
1-(2-fluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(4-fluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(2,4-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;

1-(2,5-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(3,5-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(3,4-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
*N*-(9-((1-methyl-1*H*-pyrazol-4-yl)ethynyl)-4-oxo-1,2,3,4,6,7-hexahydroazepino[3,2,1-*hi*]indol-3-yl)-1-(2-methylbenzyl)-1*H*-1,2,4-triazole-3-carboxamide;
*N*-(9-((1-methyl-1*H*-pyrazol-4-yl)ethynyl)-4-oxo-1,2,3,4,6,7-hexahydroazepino[3,2,1-*hi*]indol-3-yl)-1-(4-methylbenzyl)-1*H*-1,2,4-triazole-3-carboxamide;
1-(2,6-difluorobenzyl)-*N*-(4-oxo-9-(piperidin-4-ylethynyl)-1,2,3,4,6,7-hexahydroazepino[3,2,1-*hi*]indol-3-yl)-1*H*-1,2,4-triazole-3-carboxamide;
*tert*-butyl 4-((3-(1-(2,6-difluorobenzyl)-1*H*-1,2,4-triazole-3-carboxamido)-4-oxo-1,2,3,4,6,7-hexahydroazepino[3,2,1-*hi*]indol-9-yl)ethynyl)piperidine-1-carboxylate;
1-(2,6-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(3,5-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(2,5-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(2,4-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1-(2-fluorobenzyl)-1*H*-1,2,4-triazole-3-carboxamide;
*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1-(2-methylbenzyl)-1*H*-1,2,4-triazole-3-carboxamide;
1-(3,4-difluorobenzyl)-*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1-(4-fluorobenzyl)-1*H*-1,2,4-triazole-3-carboxamide;
*N*-(9-((1-(difluoromethyl)-1*H*-pyrazol-4-yl)ethynyl)-4-oxo-1,2,3,4,6,7-hexahydroazepino[3,2,1-*hi*]indol-3-yl)-1-(4-methylbenzyl)-1*H*-1,2,4-triazole-3-carboxamide;
1-(2,6-difluorobenzyl)-*N*-(9-ethynyl-4-oxo-1,2,3,4,6,7-hexahydroazepino[3,2,1-*hi*]indol-3-yl)-1*H*-1,2,4-triazole-3-carboxamide;
1-(2,6-difluorobenzyl)-*N*-(9-((6,7-dihydro-4*H*-pyrazolo[5,1-*c*][1,4]oxazin-3-yl)ethynyl)-4-oxo-1,2,3,4,6,7-hexahydroazepino[3,2,1-*hi*]indol-3-yl)-1*H*-1,2,4-triazole-3-carboxamide;
1-(2,6-difluorobenzyl)-*N*-(9-((5-formyl-1-vinyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-(2,6-difluorobenzyl)-*N*-(9-((1-(oxetan-3-yl)-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
*N*-(9-((1-cyclobutyl-1*H*-pyrazol-4-yl)ethynyl)-4-oxo-1,2,3,4,6,7-hexahydroazepino[3,2,1-*hi*]indol-3-yl)-1-(2,6-difluorobenzyl)-1*H*-1,2,4-triazole-3-carboxamide;
*N*-(9-((1-methyl-1*H*-pyrazol-4-yl)ethynyl)-4-oxo-1,2,3,4,6,7-hexahydroazepino[3,2,1-*hi*]indol-3-yl)-1-(pyridin-2-ylmethyl)-1*H*-1,2,4-triazole-3-carboxamide;
1-benzyl-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-1,2,4-triazole-3-carboxamide;
1-benzyl-*N*-(4-oxo-8-(pyridin-4-ylethynyl)-1,2,3,4,6,7-hexahydroazepino[3,2,1-*hi*]indol-3-yl)-1*H*-1,2,4-triazole-3-carboxamide;
*N*-(9-((1-methyl-1*H*-pyrazol-4-yl)ethynyl)-4-oxo-1,2,3,4,6,7-hexahydroazepino[3,2,1-*hi*]indol-3-yl)-6-phenoxypicolinamide;
*N*-(9-((1-methyl-1*H*-pyrazol-4-yl)ethynyl)-4-oxo-1,2,3,4,6,7-hexahydroazepino[3,2,1-*hi*]indol-3-yl)-5-phenoxypicolinamide;
5-[(2,6-difluorophenyl)methyl]-*N*-{9-[(1-methylpyrazol-4-yl)ethynyl]-4-oxo-3,4,6,7-tetrahydro-2*H*-[1,4]oxazepino[2,3,4-*hi*]indol-3-yl}-4*H*-1,2,4-triazole-3-carboxamide;
5-[(2,6-difluorophenyl)methyl]-*N*-{9-[(1-methylpyrazol-4-yl)ethynyl]-4-oxo-3,4,6,7-tetrahydro-2*H*-[1,4]thiazepino[2,3,4-*hi*]indol-3-yl}-4*H*-1,2,4-triazole-3-carboxamide;
*N*-{7,7-difluoro-9-[(1-methylpyrazol-4-yl)ethynyl]-4-oxo-1,2,4,5,6,7-hexahydroazepino[3,2,1-*hi*]indol-5-yl}-5-[(2,6-difluorophenyl)methyl]-4*H*-1,2,4-triazole-3-carboxamide;

5-benzyl-*N*-[8-(cyclopropylethynyl)-4-oxo-3,4,6,7-tetrahydro-2*H*-[1,4]oxazepino-[2,3,4-*hi*]indol-3-yl]-4*H*-1,2,4-triazole-3-carboxamide;
1-[(2,6-difluorophenyl)methyl]-*N*-{9-[(1-methylpyrazol-4-yl)ethynyl]-4-oxo-3,4,6,7-tetrahydro-2*H*-[1,4]oxazepino[2,3,4-*hi*]indol-3-yl}-1,2,4-triazole-3-carboxamide;
1-[(2,6-difluorophenyl)methyl]-*N*-{9-[(1-methylpyrazol-4-yl)ethynyl]-4-oxo-3,4,6,7-tetrahydro-2*H*-[1,4]thiazepino[2,3,4-*hi*]indol-3-yl}-1,2,4-triazole-3-carboxamide;
*N*-{7,7-difluoro-9-[(1-methylpyrazol-4-yl)ethynyl]-4-oxo-1,2,4,5,6,7-hexahydroazepino[3,2,1-*hi*]indol-5-yl}-1-[(2,6-difluorophenyl)methyl]-1,2,4-triazole-3-carboxamide;
1-(2,6-difluorobenzyl)-*N*-(9-((1-methyl-1*H*-pyrazol-4-yl)ethynyl)-4-oxo-1,2,3,4,6,7-hexahydroazepino[3,2,1-*hi*]indol-3-yl)-1*H*-pyrazole-3-carboxamide;
1-[(4-fluorophenyl)methyl]-*N*-{9-[(1-methylpyrazol-4-yl)ethynyl]-4-oxo-1,2,4,5,6,7-hexahydroazepino[3,2,1-*hi*]indol-5-yl}pyrazole-3-carboxamide;
5-(2-benzyl-3-chloro-7-oxo-4,5,6,7-tetrahydropyrazolo[3,4-*c*]pyridin-6-yl)-9-[(1-methylpyrazol-4-yl)ethynyl]-1,2,4,5,6,7-hexahydroazepino[3,2,1-*hi*]indole-4-one;
4-[(3-fluorophenyl)methyl]-*N*-{9-[(1-methylpyrazol-4-yl)ethynyl]-4-oxo-1,2,4,5,6,7-hexahydroazepino[3,2,1-*hi*]indol-5-yl}pyrazole-1-carboxamide;
4-[(3-fluorophenyl)methyl]-*N*-{9-[(1-methylpyrazol-4-yl)ethynyl]-4-oxo-3,4,6,7-tetrahydro-2*H*-[1,4]oxazepino-[2,3,4-h*i*]indol-3-yl]-4*H*-1,2,4-triazole-3-carboxamide.

**9.** A pharmaceutical composition, comprising the compound according to any one of claims 1 to 8 as an active ingredient and at least one pharmaceutically acceptable carrier.

**10.** Use of the compound according to any one of claims 1 to 8 or the pharmaceutical composition according to claim 9 in the manufacture of a medicament for preventing or treating a disease associated with a receptor-interacting protein kinase.

**11.** The use according to claim 10, wherein the disease is a disease associated with activity change of receptor-interacting protein kinase 1.

**12.** The use according to claim 11, wherein the disease is selected from tumors, including but not limited to intestinal cancer, lung cancer, liver cancer, pancreatic cancer, breast cancer, lymphatic cancer, melanoma, etc.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014125444 A
- WO 2016185423 A
- CN 112079839
- CN 201910518248X


### Non-patent literature cited in the description


- *GenesDev.*, 2013, vol. 27, 1640-1649
- *Cell Host&Microbe*, 2014, vol. 15, 23-35
- *Cell*, 2013, vol. 153, 1-14